(19) Europäisches Patentamt
European Patent Office
Office européen des brevets

(11) EP 2 934 677 B1

# (12) EUROPEAN PATENT SPECIFICATION

(45) Date of publication and mention of the grant of the patent:
**19.07.2017 Bulletin 2017/29**

(21) Application number: **13812426.8**

(22) Date of filing: **12.12.2013**

(51) Int Cl.:
***A61Q 5/02*** (2006.01) ***A61K 8/81*** (2006.01)
***A61Q 19/10*** (2006.01)

(86) International application number:
**PCT/US2013/074549**

(87) International publication number:
**WO 2014/099573 (26.06.2014 Gazette 2014/26)**

## (54) IRRITATION MITIGATING POLYMERS AND USES THEREFOR

IRRITATIONSSCHWÄCHENDE POLYMERE UND VERWENDUNGEN DAVON

POLYMÈRES DE SOULAGEMENT DE L'IRRITATION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2012 US 201261739829 P**

(43) Date of publication of application:
**28.10.2015 Bulletin 2015/44**

(73) Proprietor: **Lubrizol Advanced Materials, Inc.**
**Cleveland, OH 44141-3247 (US)**

(72) Inventors:
- **FIGURA, Brian D.**
  **Cleveland, Ohio 44141-3247 (US)**
- **CHARI, Krishnan**
  **Cleveland, Ohio 44141-3247 (US)**
- **HSU, Shui-Jen Raymond**
  **Cleveland, Ohio 44141-3247 (US)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) References cited:
**WO-A1-2005/023970 WO-A1-2008/060997**
**WO-A1-2011/042379 WO-A1-2013/040167**
**WO-A1-2013/040174 WO-A1-2013/040178**
**WO-A1-2013/054760 US-A1- 2004 096 474**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

In one aspect, the present invention relates to linear and crosslinked nonionic amphiphilic polymers and their use as ocular and/or dermal irritation mitigants in surfactant containing compositions. The crosslinked nonionic amphiphilic polymers of the invention are advantageous because they also provide tailored yield stress properties to cleansing formulations across a wide pH range. Irritation of the skin and eyes in surfactant containing personal care cleansing compositions, pet care cleansing compositions, household care cleaning compositions, and reduced irritation industrial and institutional care cleaning compositions can be mitigated by including therein the linear and/or crosslinked nonionic, amphipilic emulsion polymers of the invention.

### BACKGROUND

Detersive agents such as anionic, cationic, amphoteric and nonionic surfactants are widely used in aqueous based cleansing formulations. In personal care cleansing products (e.g., shampoos, body washes, facial cleansers, liquid hand soaps, hand wipes, etc.), pet care products (e.g., shampoos), household care cleaning products (e.g., hard surface cleaners, laundry detergents, dish soaps, automatic dish washer detergents, shower and bathtub cleansers, bathroom cleansers, car wash detergents, etc.) and industrial and institutional care cleaners (high strength cleaners, detergents, etc.) the surfactant package is the most important component in the detersive formulation. These compositions generally comprise a mixture of one or more surfactants as the active detersive ingredient. The surfactant: 1) improves the wettability of the soiled substrate; 2) loosens soils/oils/sebum from the substrate; and 3) emulsifies, solubilizes and/or suspends the loosened soils/oils/sebum particles in the aqueous wash medium.

Although in principle any surfactant class (e.g., cationic, anionic, nonionic, amphoteric) is suitable as the detersive agent in cleansing or cleaning applications, in practice most personal care cleansers and household cleaning products are formulated with anionic surfactants or with a combination of an anionic surfactant as the primary detersive agent with one or more secondary surfactants selected from the other surfactant classes. Anionic surfactants are used as one of detersive agents in personal care cleansers and detergent cleaning products because of their superior detersive properties. Exemplary anionic surfactants traditionally utilized in these formulations include alkyl sulfates, alkyl benzene sulfonates, and olefin sulfonates. While the anionic surfactants and in particular the anionic sulfates and sulfonates are very efficient detersive agents, they tend to be irritating to the skin and eyes at concentrations typically utilized for efficient detergency. It is widely known that anionic surfactants are adsorbed and even penetrate into the top layers of the skin resulting in irritation to the skin. This irritation is characteristically expressed by reddening of the skin, chapping, scaling, rash development, itching and, in extreme cases, cracking of the skin, or a burning sensation in the eyes.

It has become more and more important to consumers that aqueous cleansing compositions are efficient cleansers as well as mild. These combined properties are especially useful if the cleansing compositions are to be in direct contact with the hair and skin. Consequently, efforts have been made by formulators to deliver personal care cleansing products, household detergents and cleaners and institutional and industrial cleaners that have these properties.

Attempts to impart mildness to cleansers, particularly those formulated for personal care use, involved careful selection of the surfactants employed in the product. It is known that the irritation caused by anionic sulfates can be reduced by introducing ethoxylation into the surfactant molecule. However, a reduction in irritation is accompanied by a corresponding reduction in detergency. For example, sodium lauryl sulfate, a highly detersive surfactant, causes significant eye irritation. In contrast, sodium laureth-12 sulfate (the corresponding ethoxylate containing 12 moles of ethoxylation) is almost completely non-irritating, but is a poor detersive agent (see Schoenberg, "Baby Shampoo," Household & Personal Products Industry 60 (September 1979)). The poor detersive properties of ethoxylated alkyl sulfates are reported in many other publications.

Additional attempts to attenuate the adverse irritant effects of anionic surfactants have been made by replacing some of the anionic surfactant with very mild secondary surfactants such as amphoteric and/or nonionic surfactants as disclosed in U.S. Patent No. 4,726,915. However, reducing the amount of anionic surfactant in a cleansing or cleaning composition adversely affects the detersive properties of the composition. The major problem in providing such products resides in the fact that both properties (efficient detergency and mildness) tend to be mutually incompatible. While highly detersive surfactants are generally very harsh, mild surfactants tend to provide insufficient detersive properties.

Another approach for attenuating the adverse irritant effects of anionic detersive surfactants while maintaining high detersive properties in personal care cleansing compositions is disclosed in International Patent Application Pub. No. WO 2005/023970. It is disclosed that certain hydrophobically modified polymeric materials capable of binding surfactant can be combined with anionic surfactants to produce personal care compositions that exhibit relatively low ocular and/or dermal irritation while maintaining high detersive properties. Disclosed hydrophobically modified materials include

hydrophobically modified crosslinked acrylic copolymers that are synthesized from at least one ethylenically unsaturated carboxylic acid monomer and at least one ethylenically unsaturated hydrophobically modified monomer. Exemplary hydrophobically modified acrylic polymers are set forth in U.S. Patent No. 6,433,061 to Noveon, Inc. (now Lubrizol Advanced Materials, Inc.). The disclosure additionally exemplifies polymers available under the trade names Carbopol® Aqua SF-1 and Carbopol® ETD 2020 both provided by Lubrizol Advanced Materials, Inc. as suitable polymers for use as a surfactant binder.

In Pub. No. WO 2005/023970 the applicants therein disclose a relationship between the critical micelle concentration (CMC) of an anionic surfactant in solution and the tendency of the surfactant to induce irritation. The CMC is illustrated by curve 11 in Figure 1 of the WO 2005/023970 disclosure. As the surfactant is sequentially dosed into a container (of standardized dimension) of water the surfactant initially occupies the surface (liquid/air interface) of the water/surfactant solution. With each sequential dose of surfactant there is a concomitant reduction in the surface tension of the solution until essentially all of the interfacial surface area is filled. Continued dosing of surfactant results in the formation of micelles within the solution. The surfactant concentration at which the further addition of surfactant does not elicit any appreciable affect in solution surface tension is defined as the CMC (point 12 of curve 11). Additional surfactant added after the CMC has been attained was found to induce irritation. In contrast, as illustrated in curve 15 of Figure 1, as anionic surfactant is added to an aqueous solution comprising a hydrophobically modified crosslinked acrylic polymeric material, the CMC is shifted to a significantly higher surfactant concentration. Accordingly, the inclusion of hydrophobically modified crosslinked acrylic copolymers allows the use of higher concentrations of anionic surfactant in cleansing and cleaning compositions without the attendant ocular and dermal irritation effects.

It is to be noted that the polymers disclosed in U.S. Patent No. 6,433,061 as well as the polymers identified under the Carbopol® Aqua SF-1 and ETD 2020 trade names are rheology modifiers which thicken or enhance the rheology of the composition in which they are included. In the trade literature, Carbopol® Aqua SF-1 polymer is described by Lubrizol Advanced Materials, Inc. Technical Data Sheet TDS-294 (July, 2003) as: "...a lightly crosslinked acrylic polymer dispersion designed to impart suspending, stabilizing, and thickening properties to a variety of surfactant-based personal cleansing products". The foregoing acrylic based polymers are non-linear (crosslinked), branched polymer chains which interconnect to form three dimensional network structures and have long been used in personal care applications for their rheological and structure building properties. Upon neutralization, these water soluble or dispersible polymers possess the unique ability to greatly increase the viscosity of the liquid in which they are dissolved or dispersed.

The disclosed hydrophobically modified crosslinked acrylic copolymers are viscosity building agents that increase the viscosity of compositions in which they are dissolved or dispersed upon suitable neutralization of the carboxylic acid moieties on the polymer backbone with an alkaline material. As increasing amounts of viscosity builder are added to a cleansing or cleaning formulation to mitigate the adverse irritation effects of the anionic surfactant there is a corresponding increase in the viscosity of the composition. It is well known in the personal care, household care and industrial and institutional care formulation art that a liquid cleanser or cleaner should have an ideal viscosity. Indeed, viscosity allows for the controlled handling and dispensing of the product during use as compared to a thinner product. In personal care cleansing applications, a thick, rich shampoo or body cleanser is appealing to consumers from a sensory perspective. In household care applications, viscosity permits a better efficacy of the product when applied to non-horizontal surfaces such as toilet bowls, sinks, shower stalls, bath tubs, and the like. In addition, cleansing and cleaning products are expected to be easy to use. In other words, the shear thinning profile of the liquid composition should exhibit high viscosity at low shear conditions and lower viscosity at high shear conditions to aid in the application and removal of the product from the substrate to be cleaned.

The commercially available Carbopol® SF-1 polymer and similar polymers comprise a chemically crosslinked backbone having a pH-responsive functionality that is either base or acid sensitive. The polymers may be mixed with other ingredients in a formulation and then neutralized by the addition of a neutralization agent such as an acid or a base. Acid sensitive thickeners are activated upon contact with an acidic agent, while base-sensitive thickeners are activated upon contact with an alkaline agent. Upon neutralization, the polymers swell significantly to form a randomly close-packed (RCP) jammed network of swollen cross-linked micro-gel particles imparting a desired rheological profile, i.e., yield stress, elastic modulus, and viscosity, as well as optical clarity to the formulation.

There are drawbacks associated with increasing the viscosity of a product beyond its ideal viscosity. Highly viscous products are typically difficult to apply and rinse away, especially if the shear thinning profile of the viscosity building agent is poor. High viscosities can also adversely affect packaging, dispensing, dissolution, and the foaming and sensory properties of the product.

While a certain rheology modifier may thicken or enhance the viscosity of a composition in which it is included, it does not necessarily have desirable yield stress properties. A desirable yield stress property is critical to achieving certain physical and aesthetic characteristics in a liquid medium, such as the indefinite suspension of particles, insoluble liquid droplets, or the stabilization of gas bubbles within a liquid medium. Particles dispersed in a liquid medium will remain suspended if the yield stress (yield value) of the medium is sufficient to overcome the effect of gravity or buoyancy on those particles. Insoluble liquid droplets can be prevented from rising and coalescing and gas bubbles can be suspended and uniformly distributed in a liquid medium using yield value as a formulating tool. A yield stress fluid is used generally to adjust or modify the rheological properties of aqueous compositions. Such properties include, without limitation, viscosity improvement, flow rate improvement, stability to viscosity change over time, and the ability to suspend particles for indefinite periods of time.

To alleviate the substantial viscosity profile of the above described crosslinked hydrophobically modified acrylic polymers, U.S. Patent No. 8,293,845 describes the use of low molecular weight hydrophobically modified linear (non-crosslinked) acrylic polymers to increase the CMC of a surfactant containing composition to mitigate irritation. The applicants teach that the ideal CMC is achieved by neutralizing the linear polymer with an alkaline material to a degree of neutralization ranging from about 15 to about 30 %, based on the acid number of the polymer. As with the crosslinked hydrophobically modified acrylic polymer irritation mitigants, these linear acrylic polymer counterparts are pH dependent in that the degree of neutralization of the polymer must be maintained within a narrow range in order to reach the optimal CMC value. In addition, the disclosed linear polymers are not crosslinked and do not create a yield stress.

WO-A-2011/042379 proposes superhydrophilic amphiphilic copolymers with low irritation of the eyes and skin. The polymers are useful for producing a composition that has both low irritation and high amounts of flash foam.

WO-A-2008/060997 discloses non-crosslinked, linear acrylic compolymers of low molecular weight and their use as ocular and/or dermal irritation mitigants in surfactant containing compositions.

WO-A-2005/023970 discloses a method of reducing the irritation associated with a personal care composition comprising at least one anionic surfactant, the method comprising combining a hydrophobically-modified material capable of binding a surfactant, wherein the hydrophobically-modified material is selected from the group consisting of hydrophobically-modified acrylic polymers, hydrophobically-modified cellulosics, hydrophobically-modified starches.

US-A-2004/0096474 discloses nonionic amphiphilic polymers in hair care compositions, such as hairstyling compositions.

Accordingly, there is a need for an irriation mitigation polymer that is not pH dependent and that can be tailored to create a desired yield stress in the detersive compositions in which it is incorporated.

## SUMMARY OF THE INVENTION

The present invention relates to the following embodiments:

1. A crosslinked nonionic amphiphilic emulsion polymer prepared from a free radically polymerizable monomer composition comprising at least 30 wt.% of at least one $C_1$-$C_4$ hydroxyalkyl (meth)acrylate, 15 to 70 wt. of at least one $C_1$-$C_{12}$ alkyl (meth)acrylate, 5 to 40 wt.% of at least one vinyl ester of a $C_1$-$C_{10}$ aliphatic carboxylic acid (based on the weight of the total monomers), and 0.01 to 0.3 wt.% of at least one crosslinker (based on the dry weight of the polymer) for use in mitigating ocular and/or skin irritation of detersive compositions comprising at least one anionic surfactant.

2. A crosslinked nonionic amphiphilic emulsion polymer prepared from a free radically polymerizable monomer composition comprising at least 30 wt.% of at least one $C_1$-$C_4$ hydroxyalkyl (meth)acrylate, 15 to 70 wt. of at least one $C_1$-$C_{12}$ alkyl (meth)acrylate, 1 to 10 wt.% of at least one monomer selected from an associative monomer, a semi-hydrophobic monomer, or mixtures thereof (based on the weight of the total monomers), and 0.01 to 0.3 wt.% of at least one crosslinker (based on the dry weight of the polymer) for use in mitigating ocular and/or skin irritation of detersive compositions comprising at least one anionic surfactant, wherein said associative monomer is represented by formulas VII and/or VIIA:

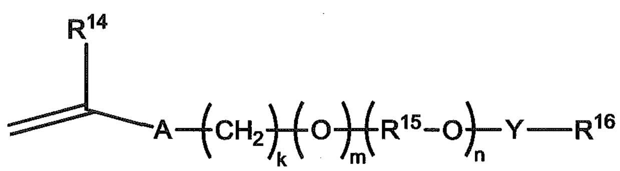

VII

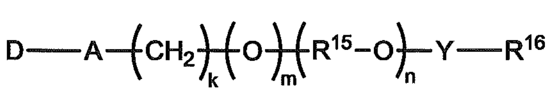

VIIA

wherein $R^{14}$ is hydrogen or methyl; A is $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; Ar is a divalent arylene, e.g. phenylene; E is H or methyl; z is 0 or 1; k is an integer ranging from 0 to abut 30, and m is 0 or 1, with the proviso that when k is 0,

m is 0, and when k is in the range of 1 to 30, m is 1; D represents a vinyl or an allyl moiety; $(R^{15}\text{-}O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 10 to 120 in another aspect, and from 15 to 60 in a further aspect; Y is -$R^{15}$O-, -$R^{15}$NH-, -C(O)-, -C(O)NH-, -$R^{15}$NHC(O)NH-, or-C(O)NHC(O)-; $R^{16}$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl; wherein the $R^{16}$ alkyl group, aryl group, phenyl group optionally comprises one or more substituents selected from the group consisting of a hydroxyl group, an alkoxyl group, benzyl group, styryl group, and a halogen group; and said semi-hydrophobic monomer is selected from at least one monomer represented by formulas VIII and IX:

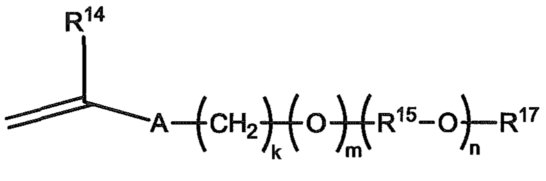
VIII

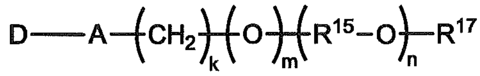
IX

wherein $R^{14}$ is hydrogen or methyl; A is -$CH_2$C(O)O-, -C(O)O-, -O-, -$CH_2$O-,-NHC(O)NH-, -C(O)NH-, -Ar-$(CE_2)_z$-NHC(O)O-, -Ar-$(CE_2)_z$-NHC(O)NH-, or -$CH_2CH_2$NHC(O)-; Ar is a divalent arylene, e.g. phenylene; E is H or methyl; z is 0 or 1; k is an integer ranging from 0 to 30, and m is 0 or 1, with the proviso that when k is 0, m is 0, and when k is in the range of 1 to 30, m is 1; $(R^{15}\text{-}O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 5 to 120 in another aspect, and from 10 to 60 in a further aspect; $R^{17}$ is selected from hydrogen and a linear or branched $C_1$-$C_4$ alkyl group; and D represents a vinyl or an allyl moiety.

3. The polymer for the use according to embodiment 1 wherein said $C_1$-$C_4$ hydroxyalkyl (meth)acrylate is hydroxyethyl methacrylate, said $C_1$-$C_{12}$ alkyl acrylate is selected from methyl methacrylate, ethyl acrylate, butyl acrylate, or mixtures thereof, said vinyl ester of a $C_1$-$C_{10}$ carboxylic acid is selected from vinyl formate, vinyl acetate, vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, vinyl stearate, or mixtures thereof; or mixtures thereof.

4. The polymer for the use according to embodiment 1 wherein said emulsion polymer is polymerized from a monomer mixture comprising hydroxyethyl methacrylate, and a monomer selected from methyl methacrylate, ethyl acrylate, butyl acrylate, vinyl acetate, vinyl neodecanoate, vinyl decanoate, or mixtures thereof.

5. The polymer for the use according to embodiment 4 wherein said emulsion polymer is polymerized from a monomer mixture comprising hydroxyethyl methacrylate, ethyl acrylate, butyl acrylate and vinyl acetate.

6. The polymer for the use according to embodiment 2, wherein said emulsion polymer is polymerized from a monomer mixture comprising hydroxyethyl methacrylate, ethyl acrylate, and butyl acrylate and a monomer selected from an associative and/or a semi-hydrophobic monomer.

7. The polymer for the use according to embodiment 2 wherein said associative monomer is represented by formula VIIB:

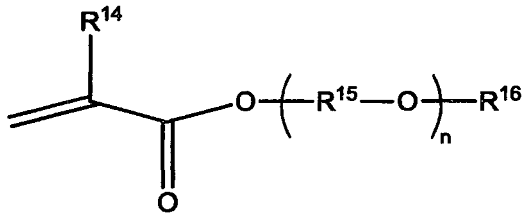

VIIB

wherein $R^{14}$ is hydrogen or methyl; $R^{15}$ is a divalent alkylene moiety independently selected from $C_2H_4$, $C_3H_6$, and $C_4H_8$, and n represents an integer ranging from 10 to 60, ($R^{15}$-O) can be arranged in a random or a block configuration; $R^{16}$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substitutedwphenyl, an araalkyl substituted phenyl, and an aryl-substituted $C_2$-$C_{30}$ alkyl, wherein the $R^{16}$ alkyl group, aryl group, phenyl group optionally comprises one or more substituents selected from the group consisting of a hydroxyl group, an alkoxyl group, benzyl group styryl group, and a halogen group.

8. The polymer for the use according to embodiment 2 wherein said semi-hydrophobic monomer comprises (i) an ethylenically unsaturated end group portion; (ii) a polyoxyalkylene mid-section portion, and (iii) an end group portion selected from hydrogen or a alkyl group containing 1 to 4 carbon atoms.

9. The polymer for the use according to embodiment 2 wherein said semi-hydrophobic monomer is selected from at least one monomer represented by formulas VIIIA and VIIIB:

$CH_2$=C($R^{14}$)C(O)O-$(C_2H_4O)_a(C_3H_6O)_b$-H VIIIA

$CH_2$=C($R^{14}$)C(O)O-$(C_2H_4O)$a$(C_3H_6O)_b$-$CH_3$ VIIIB

wherein $R^{14}$ is hydrogen or methyl, and "a" is an integer ranging from 0 or 2 to 120 in one aspect, from 5 to 45 in another aspect, and from 10 to 0.25 in a further aspect, and "b" is an integer ranging from 0 or 2 to 120 in one aspect, from 5 to 45 in another aspect, and from 10 to 0.25 in a further aspect, subject to the proviso that "a" and "b" cannot be 0 at the same time.

10. The polymer for the use according to embodiment 9 wherein b is 0.

11. The polymer for the use according to embodiment 7 wherein said associative monomer is selected from lauryl polyethoxylated (meth)acrylate, cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated (meth)acrylate, cerotyl polyethoxylated (meth)acrylate, montanyl polyethoxylated (meth)acrylate, melissyl polyethoxylated (meth)acrylate, where the polyethoxylated portion of the monomer contains 2 to 50 ethylene oxide units, and said semi-hydrophobic monomer is selected from methoxy polyethyleneglycol (meth)acrylate or polyethyleneglycol (meth)acrylate, where the polyethoxylated portion of the monomer contains 2 to 50 ethylene oxide units.

12. The polymer for the use according to any of embodiments 1 to 6 wherein said crosslinker is selected from a monomer having an average of 3 crosslinkable unsaturated functional groups.

13. The polymer for the use according to embodiment 12 wherein said crosslinker is pentaerythritol triallyl ether.

14. The polymer for the use according to embodiment 13 wherein said pentaerythritol triallyl ether is present in an amount ranging from 0.01 to 0.3 (based on the dry weight of the polymer).

15. The polymer for the use according to any of embodiments 2, 6 to 11 wherein said emulsion polymer is polymerized from a monomer mixture comprising from 40 to 45 wt.% of hydroxyethyl acrylate, 30 to 50 wt. % of ethyl acrylate, 10 to 20 wt.% of butyl acrylate and from 1 to 5 wt.% of at least one associative and/or semi-hydrophobic monomer (based on the weight of the total monomers), and at least one crosslinker.

16. A crosslinked nonionic amphiphilic emulsion polymer prepared from a free radically polymerizable monomer mixture comprising:

i) 40 to 50 wt.% of at least one hydroxy($C_1$-$C_5$)alkyl (meth)acrylate monomer (based on the total monomer wt.);

ii) 15 to 70 wt.% of at least two different monomers selected from a ($C_1$-$C_5$)alkyl (meth)acrylate monomer (based on the total monomer wt.);
iii) 0.5 to 5 wt.% of an associative and/or a semi-hydrophobic monomer; and
iv) 0.01 to 0.3 wt.% of at least one crosslinker (based on the dry weight of the polymer) for use in mitigating ocular and/or skin irritation of a detersive compositions comprising

a) water;
b) 1 to 5 wt.% of said crosslinked nonionic amphiphilic emulsion polymer; and
c) 6 to 20 wt.% of a surfactant mixture containing an anionic surfactant and an amphoteric surfactant.

17. The polymer for the use according to embodiment 16 wherein said monomer i) is hydroxyethyl methacrylate.

18. The polymer for the use according to any of embodiments 16 or 17 wherein said monomers ii) are ethyl acrylate and n-butyl acrylate.

19. The polymer for the use according to any of embodiments 16 to 18 wherein ethyl acrylate is present in an amount ranging from 15 to 50 wt.% of the monomer mixture.

20. The polymer for the use according to any of embodiments 16 to 19 wherein butyl acrylate is present in an amount ranging from 10 to 20 wt.% of the monomer mixture.

21. The polymer for the use according to any of embodiments 16 to 20 wherein said associative monomer is selected from behenyl polyethoxylated methacrylate.

22. The polymer for the use according to any of embodiments 16 to 21 wherein said associative monomer contains 2 to 30 moles of ethoxylation.

23. The polymer for the use according to any of embodiments 16 to 22 wherein said semi hydrophobic monomer is selected from methoxy polyethyleneglycol methacrylate.

24. The polymer for the use according to any of embodiments 16 to 23 wherein said anionic surfactant contains an average of 1 to 3 moles of ethoxylation in one aspect, or an average of 1 to 2 moles of ethoxylation in another aspect.

25. The polymer for the use according to any of embodiments 16 to 24 wherein the ratio of said anionic surfactant to said amphoteric surfactant ranges from 10:1 to 2:1 (wt./wt.).

26. The polymer for the use according to any of embodiments 16 to 25 wherein said anionic surfactant is selected from the sodium or ammonium salts of dodecyl sulfate, lauryl sulfate, laureth sulfate, or mixtures thereof.

27. The polymer for the use according to any of embodiments 16 to 26 wherein said amphoteric surfactant is cocamidopropyl betaine.

28. The polymer for the use according to any of embodiments 16 to 27 wherein said detersive composition is selected from shampoos, baby shampoos, body washes, shower gels, liquid hand soaps, liquid dishwashing detergents, pet cleansing product, moist cleansing wipes, or facial cleansers.

The present invention provides mild cleansing and cleaning compositions which mitigate irritation induced by harsh detersive surfactants contained therein. It has been discovered that a milder cleansing composition possessing excellent detersive properties can be obtained by incorporating at least one crosslinked nonionic amphiphilic emulsion polymer into the formulation to mitigate the irritant effects of harsh detersive surfactants contained therein.
It has been discovered that the crosslinked, nonionic, amphiphilic emulsion polymers of the invention exhibit a unique and unexpected combination of properties including the ability to mitigate irritation in and to provide yield stress properties to surfactant containing cleansing and cleaning compositions that is independent of pH.
In one aspect, the invention provides mild cleansing and cleaning compositions comprising at least one crosslinked, nonionic amphiphilic emulsion polymer and at least one detersive surfactant selected from anionic surfactants, amphoteric surfactants, nonionic surfactants and combinations of two or more thereof.
Skin irritation associated with a cleansing composition comprising at least one surfactant, can be reduced by combining a crosslinked, nonionic amphiphilic emulsion polymer with at least one detersive surfactant selected from

anionic surfactants, amphoteric surfactants, nonionic surfactants and combinations of two or more thereof.

Skin irritation induced by a surfactant containing composition can be reduced by contacting a mammalian body with a detersive composition comprising at least one crosslinked, nonionic amphiphilic emulsion polymer and at least one detersive surfactant selected from anionic surfactants, amphoteric surfactants, nonionic surfactants and combinations of two or more thereof.

In another aspect, the at least one crosslinked, nonionic amphiphilic emulsion polymer capable of mitigating irritation to the eyes and skin in surfactant containing compositions comprising at least one surfactant selected from anionic surfactants, amphoteric surfactants, nonionic surfactants and combinations of two or more thereof is not pH dependent and can be tailored to provide desired yield stress properties to a given detersive surfactant containing cleansing and cleaning formulation.

Skin irritation associated with a thickened cleansing composition comprising at least one surfactant, can be reduced by combining a crosslinked, nonionic amphiphilic emulsion polymer with at least one detersive surfactant selected from anionic surfactants, amphoteric surfactants, nonionic surfactants and combinations of two or more thereof, wherein the concentration of the crosslinked, nonionic amphiphilic emulsion polymer is no more than 5 wt.%, and the at least one surfactant is no more than 30 wt.% (all weight percents are based on the total weight of the composition), wherein the yield stress of the composition is at least 0.1 Pa with a shear thinning index of less than 0.5 at shear rates between 0.1 and 1 reciprocal seconds, and wherein the yield stress, elastic modulus and optical clarity of the composition are substantially independent of pH in the range of 2 to 14.

Skin irritation associated with a thickened cleansing composition comprising at least one surfactant, can be reduced by combining a crosslinked, nonionic amphiphilic emulsion polymer with at least one detersive surfactant selected from anionic surfactants, amphoteric surfactants, nonionic surfactants and combinations of two or more thereof, wherein the concentration of the amphiphilic polymer is no more than 5 wt.%, and the at least one surfactant is no more than 30 wt.% (all weight percents are based on the total weight of the composition), wherein the yield stress of the composition is at least 0.1 Pa with a shear thinning index of less than 0.5 at shear rates between 0.1 and 1 reciprocal seconds, wherein the yield stress, elastic modulus of the composition are substantially independent of pH in the range of 2 to 14, and wherein the composition is able to suspend beads of a size between 0.5 and 1.5 mm where the difference in specific gravity of the beads relative to water is in the range of 0.2 to 0.5 for a period of at least 4 weeks at room temperature.

A crosslinked nonionic amphiphilic emulsion polymer prepared from a free radically polymerizable monomer composition comprising at least 30 wt.% of at least one $C_1$-$C_4$ hydroxyalkyl (meth)acrylate, 15 to 70 wt. of at least one $C_1$-$C_{12}$ alkyl (meth)acrylate, 5 to 40 wt.% of at least one vinyl ester of a $C_1$-$C_{10}$ aliphatic carboxylic acid (based on the weight of the total monomers), and 0.01 to 0.3 wt.% of at least one crosslinker (based on the dry weight of the polymer) for use in mitigating ocular and/or skin irritation of detersive compositions comprising at least one anionic surfactant.

The nonionic, amphiphilic polymer compositions as well as the thickened aqueous fluid comprising the crosslinked, nonionic, amphiphilic, emulsion polymer compositions and the at least one surfactant of the present invention may suitably comprise, consist of, or consist essentially of the components, elements, and process delineations described herein. The invention illustratively disclosed herein suitably may be practiced in the absence of any element or process step which is not specifically disclosed herein.

Unless otherwise stated, all percentages, parts, and ratios expressed herein are based upon the total weight of the components contained in the compositions of the present invention.

As used herein, the term "amphiphilic polymer" means that the polymeric material has distinct hydrophilic and hydrophobic portions. "Hydrophilic" typically means a portion that interacts intramolecularly with water and other polar molecules. "Hydrophobic" typically means a portion that interacts preferentially with oils, fats or other non-polar molecules rather than aqueous media.

As used herein, the term "hydrophilic monomer" means a monomer that is substantially water soluble. "Substantially water soluble" refers to a material that is soluble in distilled (or equivalent) water, at 25°C, at a concentration of 3.5% by weight in one aspect, and soluble at 10% by weight in another aspect (calculated on a water plus monomer weight basis).

As used herein, the term "hydrophobic monomer" means a monomer that is substantially water insoluble. "Substantially water insoluble" refers to a material that is not soluble in distilled (or equivalent) water, at 25°C, at a concentration of 3% by weight in one aspect, and not soluble at 2.5% by weight in another aspect (calculated on a water plus monomer weight basis).

By "nonionic" is meant that a monomer, monomer composition or a polymer polymerized from a monomer composition is devoid of ionic or ionizable moieties ("nonionizable").

An ionizable moiety is any group that can be made ionic by neutralization with an acid or a base.

An ionic or an ionized moiety is any moiety that has been neutralized by an acid or a base.

By "substantially nonionic" is meant that the monomer, monomer composition or polymer polymerized from a monomer composition contains less than 5 wt.% in one aspect, less than 3 wt.% in another aspect, less than 1 wt.% in a further aspect, less than 0.5 wt.% in a still further aspect, less than 0.1 wt.% in an additional aspect, and less than

0.05 wt.% in a further aspect, of an ionizable and/or an ionized moiety.

For the purpose of the specification, the prefix "(meth)acryl" includes "acryl" as well as "methacryl". For example, the term "(meth)acrylamide" includes both acrylamide and methacrylamide.

## BRIEF DESCRIPTION OF THE DRAWINGS

Fig. 1 is a plot of the average particle size of a crosslinked, nonionic, amphiphilic polymer in the yield stress fluid of Example 12 formulated with sodium dodecyl sulfate (SDS) at various concentrations.

Fig. 2 is a plot of the elastic (G') and viscous moduli (G") as a function of increasing oscillatory stress amplitude (Pa) for the yield stress fluid formulation of Example 13. The plot shows the crossover point of G' and G" corresponding to the yield stress value of the formulation.

Fig. 3 is a plot of the elastic (G') and viscous moduli (G") as a function of increasing oscillatory stress amplitude for a yield stress fluid formulated from the crosslinked, nonionic, amphiphilic polymer of Example 9.

## DESCRIPTION OF EXEMPLARY EMBODIMENTS

Exemplary embodiments in accordance with the present invention will be described. Various modifications, adaptations or variations of the exemplary embodiments described herein may become apparent to those skilled in the art as such are disclosed.

While overlapping weight ranges for the various components and ingredients that can be contained in the compositions of the invention have been expressed for selected embodiments and aspects of the invention, it should be readily apparent that the specific amount of each component in the disclosed compositions will be selected from its disclosed range such that the amount of each component is adjusted such that the sum of all components in the composition will total 100 weight percent. The amounts employed will vary with the purpose and character of the desired product and can be readily determined by one skilled in the art.

Amphiphilic Polymer

The nonionic, amphiphilic polymers useful in the practice of the invention are polymerized from monomer components that contain free radical polymerizable unsaturation. In one embodiment, the nonionic, amphiphilic polymers useful in the practice of the invention are polymerized from a monomer composition comprising at least one nonionic, hydrophilic unsaturated monomer, and at least one unsaturated hydrophobic monomer. According to the invention, the nonionic, amphiphilic emulsion polymers useful in the practice of the invention are crosslinked. The crosslinked polymers are prepared from a monomer composition comprising at least one nonionic, hydrophilic unsaturated monomer, at least one unsaturated hydrophobic monomer, and at least one polyunsaturated crosslinking monomer.

In one embodiment, the copolymers can be prepared from a monomer composition typically having a hydrophilic monomer to hydrophobic monomer ratio of from 5:95 wt.% to 95:5 wt.% in one aspect, from 15:85 wt.% to 85:15 wt.% in another aspect, and from 30:70 wt.% to 70:30 wt.% in a further aspect, based on the total weight of the hydrophilic and hydrophobic monomers present. The hydrophilic monomer component can be selected from a single hydrophilic monomer or a mixture of hydrophilic monomers, and the hydrophobic monomer component can be selected from a single hydrophobic monomer or a mixture of hydrophobic monomers.

Hydrophilic onomer

The hydrophilic monomers suitable for the preparation of the crosslinked, nonionic, amphiphilic polymer compositions of the invention are selected from hydroxy($C_1$-$C_4$)alkyl (meth)acrytates.

The hydroxy($C_1$-$C_4$)alkyl (meth)acrylates can be structurally represented by the following formula:

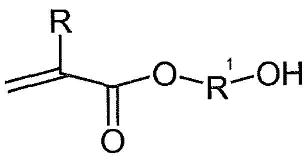 (I)

wherein R is hydrogen or methyl and $R^1$ is an divalent alkylene moiety containing 1 to 4 carbon atoms, wherein the alkylene moiety optionally can be substituted by one or more methyl groups. Representative monomers include 2-hydroxyethyl(meth)acrylate, 3-hydroxypropyl(meth)acrylate, 4-hydroxybutyl(meth)acrylate, and mixtures thereof.

Hydrophobic Monomer

Hydrophobic monomers used for the preparation of the crosslinked, nonionic, amphiphilic polymer compositions of the invention are selected from one or more of alkyl esters of (meth)acrylic acid having an alkyl group containing 1 to 12 carbon atoms such as methylmethacrylate, ethylacrylate, butylacrylate, or mixtures thereof; vinyl esters of aliphatic carboxylic acids containing 1 to 10 carbon atoms; and an associative monomer having a hydrophobic end group containing 8 to 30 carbon atoms, and mixtures thereof.

Semi-Hydrophobic Monomer

Optionally, at least one semi-hydrophobic monomer can be used in the preparation of the amphiphilic polymers of the invention. A semi-hydrophobic monomer is similar in structure to an associative monomer, but has a substantially non-hydrophobic end group selected from hydroxyl or a moiety containing 1 to 4 carbon atoms.
In one aspect of the invention, the alkyl esters of (meth)acrylic acid having an alkyl group containing 1 to 30 carbon atoms can be represented by the following formula

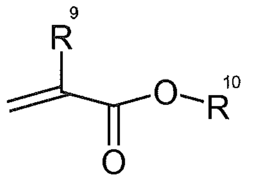
(IV)

wherein $R^9$ is hydrogen or methyl and $R^{10}$ is $C_1$ to $C_{30}$ alkyl. Representative monomers under formula (IV) include but are not limited to methyl (meth)acrylate, ethyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, behenyl (meth)acrylate, and mixtures thereof.
Vinyl esters of aliphatic carboxylic acids containing 1 to 10 carbon atoms can be represented by the following formula:

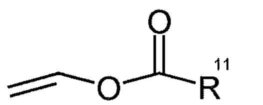
(V)

wherein $R^{11}$ is a $C_1$ to $C_{10}$ aliphatic group which can be an alkyl or alkenyl. Representative monomers under formula (V) include but are not limited to vinyl acetate, vinyl propionate, vinyl butyrate, vinyl isobutyrate, vinyl valerate, vinyl hexanoate, vinyl 2-methylhexanoate, vinyl 2-ethylhexanoate, vinyl iso-octanoate, vinyl nonanoate, vinyl neodecanoate, vinyl decanoate, vinyl versatate, and mixtures thereof.
The associative monomer of the invention has an ethylenically unsaturated end group portion (i) for addition polymerization with the other monomers of the invention; a polyoxyalkylene mid-section portion (ii) for imparting selective hydrophilic and/or hydrophobic properties to the product polymer, and a hydrophobic end group portion (iii) for providing selective hydrophobic properties to the polymer.
The portion (i) supplying the ethylenically unsaturated end group can be a residue derived from an $\alpha,\beta$-ethylenically unsaturated monocarboxylic acid. Alternatively, portion (i) of the associative monomer can be a residue derived from an allyl ether or vinyl ether; a nonionic vinyl-substituted urethane monomer, such as disclosed in U.S. Reissue Patent No. 33,156 or U.S. Patent No. 5,294,692; or a vinyl-substituted urea reaction product, such as disclosed in U.S. Patent No. 5,011,978.
The mid-section portion (ii) is a polyoxyalkylene segment of 2 to 150 in one aspect, from 10 to 120 in another aspect, and from 15 to 60 in a further aspect of repeating $C_2$-$C_4$ alkylene oxide units. The mid-section portion (ii) includes polyoxyethylene, polyoxypropylene, and polyoxybutylene segments, and combinations thereof comprising from 2 to 150 in one aspect, from 5 to 120 in another aspect, and from 10 to 60 in a further aspect of ethylene, propylene and/or butylene oxide units, arranged in random or block sequences of ethylene oxide, propylene oxide and/or butylene oxide units.
The hydrophobic end group portion (iii) of the associative monomer is a hydrocarbon moiety belonging to one

of the following hydrocarbon classes: a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an araalkyl substituted phenyl, and aryl-substituted $C_2$-$C_{30}$ alkyl groups.

Non-limiting examples of suitable hydrophobic end group portions (iii) of the associative monomers are linear or branched alkyl groups having 8 to 30 carbon atoms, such as capryl ($C_8$), iso-octyl (branched $C_8$), decyl ($C_{10}$), lauryl ($C_{12}$), myristyl ($C_{14}$), cetyl ($C_{16}$), cetearyl ($C_{16}$-$C_{18}$), stearyl ($C_{18}$), isostearyl (branched $C_{18}$), arachidyl ($C_{20}$), behenyl ($C_{22}$), lignoceryl ($C_{24}$), cerotyl ($C_{26}$), montanyl ($C_{28}$), melissyl ($C_{30}$), and the like.

Examples of linear and branched alkyl groups having about 8 to about 30 carbon atoms that are derived from a natural source include, without being limited thereto, alkyl groups derived from hydrogenated peanut oil, soybean oil and canola oil (all predominately $C_{18}$), hydrogenated tallow oil ($C_{16}$-$C_{18}$), and the like; and hydrogenated $C_{10}$-$C_{30}$ terpenols, such as hydrogenated geraniol (branched $C_{10}$), hydrogenated farnesol (branched $C_{15}$), hydrogenated phytol (branched $C_{20}$), and the like.

Non-limiting examples of suitable $C_2$-$C_{30}$ alkyl-substituted phenyl groups include octylphenyl, nonylphenyl, decylphenyl, dodecylphenyl, hexadecylphenyl, octadecylphenyl, isooctylphenyl, sec-butylphenyl, and the like.

Exemplary aryl-substituted $C_2$-$C_{40}$ alkyl groups include, without limitation thereto, styryl (e.g., 2-phenylethyl), distyryl (e.g., 2,4-diphenylbutyl), tristyryl (e.g., 2,4,6-triphenylhexyl), 4-phenylbutyl, 2-methyl-2-phenylethyl, tristyrylphenolyl, and the like.

Suitable $C_8$-$C_{30}$ carbocylic alkyl groups include, without being limited thereto, groups derived from sterols from animal sources, such as cholesterol, lanosterol, 7-dehydrocholesterol, and the like; from vegetable sources, such as phytosterol, stigmasterol, campesterol, and the like; and from yeast sources, such as ergosterol, mycosterol, and the like. Other carbocyclic alkyl hydrophobic end groups useful in the present invention include, without being limited thereto, cyclooctyl, cyclododecyl, adamantyl, decahydronaphthyl, and groups derived from natural carbocyclic materials, such as pinene, hydrogenated retinol, camphor, isobornyl alcohol, and the like.

Useful associative monomers can be prepared by any method known in the art. See, for example, U.S. Patents No. 4,421,902 to Chang et al.; No. 4,384,096 to Sonnabend; No. 4,514,552 to Shay et al.; No. 4,600,761 to Ruffner et al.; No. 4,616,074 to Ruffner; No. 5,294,692 to Barron et al.; No. 5,292,843 to Jenkins et al.; No. 5,770,760 to Robinson; and No. 5,412,142 to Wilkerson, III et al.

In one aspect, exemplary associative monomers include those represented by formulas (VII) and (VIIA) as follows:

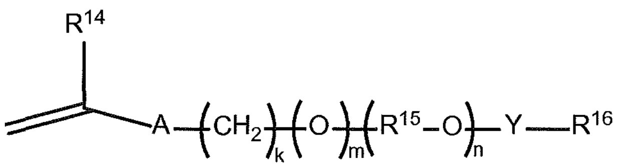

VII

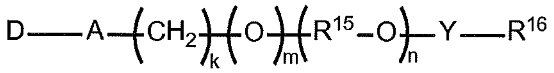

VIIA

wherein $R^{14}$ is hydrogen or methyl; A is $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; $(R^{15}\text{-}O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 10 to 120 in another aspect, and from 15 to 60 in a further aspect; Y is $-R^{15}O-$, $-R^{15}NH-$, $-C(O)-$, $-C(O)NH-$, $-R^{15}NHC(O)NH-$, or $-C(O)NHC(O)-$; $R^{16}$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl; wherein the $R^{16}$ alkyl group, aryl group, phenyl group optionally comprises one or more substituents selected from the group consisting of a hydroxyl group, an alkoxyl group, benzyl group phenylethyl group, and a halogen group.

In one aspect, the hydrophobically modified associative monomer is an alkoxylated (meth)acrylate having a hydrophobic group containing 8 to 30 carbon atoms represented by the following formula:

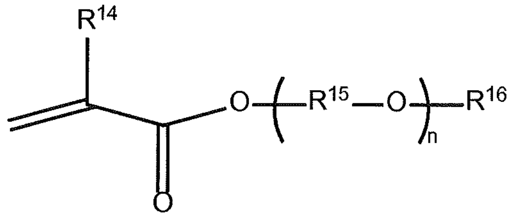

VIIB

wherein $R^{14}$ is hydrogen or methyl; $R^{15}$ is a divalent alkylene moiety independently selected from $C_2H_4$, $C_3H_6$, and $C_4H_8$, and n represents an integer ranging from about 2 to 150 in one aspect, from 5 to 120 in another aspect, and from 10 to 60 in a further aspect, ($R^{15}$-O) can be arranged in a random or a block configuration; $R^{16}$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, and an aryl-substituted $C_2$-$C_{30}$ alkyl.

Representative monomers under formula (VII) include lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, where the polyethoxylated portion of the monomer contains 2 to 150 ethylene oxide units in one aspect, from 5 to 120 in another aspect, and from 10 to 60 in a further aspect; octyloxy polyethyleneglycol (8) polypropyleneglycol (6) (meth)acrylate, phenoxy polyethylene glycol (6) polypropylene glycol (6) (meth)acrylate, and nonylphenoxy polyethylene glycol polypropylene glycol (meth)acrylate.

The semi-hydrophobic monomers of the invention are structurally similar to the associative monomer described above, but have a substantially non-hydrophobic end group portion. The semi-hydrophobic monomer has an ethylenically unsaturated end group portion (i) for addition polymerization with the other monomers of the invention; a polyoxyalkylene mid-section portion (ii) for imparting selective hydrophilic and/or hydrophobic properties to the product polymer and a semi- hydrophobic end group portion (iii). The unsaturated end group portion (i) supplying the vinyl or other ethylenically unsaturated end group for addition polymerization is preferably derived from an α,β-ethylenically unsaturated mono carboxylic acid. Alternatively, the end group portion (i) can be derived from an allyl ether residue, a vinyl ether residue or a residue of a nonionic urethane monomer.

The polyoxyalkylene mid-section (ii) specifically comprises a polyoxyalkylene segment, which is substantially similar to the polyoxyalkylene portion of the associative monomers described above. In one aspect, the polyoxyalkylene portions (ii) include polyoxyethylene, polyoxypropylene, and/or polyoxybutylene units comprising from 2 to 150 in one aspect, from 5 to 120 in another aspect, and from 10 to 60 in a further aspect of ethylene oxide, propylene oxide, and/or butylene oxide units, arranged in random or blocky sequences.

In one aspect, the semi-hydrophobic monomer can be represented by the following formulas:

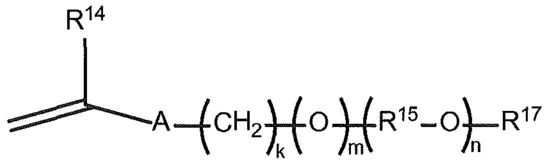

VIII

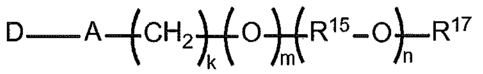

IX

wherein $R^{14}$ is hydrogen or methyl; A is $-CH_2C(O)O-$, $-C(O)O-$, $-O-$, $-CH_2O-$, $-NHC(O)NH-$, $-C(O)NH-$, $-Ar-(CE_2)_z$ $NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; $(R^{15}\text{-}O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 5 to 120 in another aspect, and from 10 to 60 in a further aspect; $R^{17}$ is selected from hydrogen and a linear or branched $C_1$-$C_4$ alkyl group (e.g., methyl, ethyl, propyl, iso-propyl, butyl, iso-butyl, and tert-butyl); and D represents a vinyl or an allyl moiety.

In one aspect, the semi-hydrophobic monomer under formula VIII can be represented by the following formulas:

$CH_2=C(R^{14})C(O)O-(C_2H_4O)_a(C_3H_6O)_b-H$ VIIIA

$CH_2=C(R^{14})C(O)O-(C_2H_4O)_a(C_3H_6O)_b-CH_3$ VIIIB

wherein $R^{14}$ is hydrogen or methyl, and "a" is an integer ranging from 0 or 2 to 120 in one aspect, from 5 to 45 in another aspect, and from 10 to 0.25 in a further aspect, and "b" is an integer ranging from 0 or 2 to 120 in one aspect, from 5 to 45 in another aspect, and from 10 to 25 in a further aspect, subject to the proviso that "a" and "b" cannot be 0 at the same time.

Examples of semi-hydrophobic monomers under formula VIIIA include polyethyleneglycol methacrylate available under the product names Blemmer® PE-90 ($R^{14}$ = methyl, a = 2, b = 0), PE-200 ($R^{14}$ = methyl, a = 4.5, b = 0), and PE-350 ($R^{14}$ = methyl a = 8, b = 0,); polypropylene glycol methacrylate available under the product names Blemmer® PP-1000 ($R^{14}$ = methyl, b = 4-6, a = 0), PP-500 ($R^{14}$ = methyl, a = 0, b = 9), PP-800 ($R^{14}$ = methyl, a = 0, b 13); polyethyleneglycol polypropylene glycol methacrylate available under the product names Blemmer® 50PEP-300 ($R^{14}$ = methyl, a = 3.5, b = 2.5), 70PEP-350B ($R^{14}$ = methyl, a = 5, b = 2); polyethyleneglycol acrylate available under the product names Blemmer® AE-90 ($R^{14}$ = hydrogen, a = 2, b = 0), AE-200 ($R^{14}$ = hydrogen, a = 2, b = 4.5), AE-400 ($R^{14}$ = hydrogen, a = 10, b = 0); polypropyleneglycol acrylate available under the product names Blemmer® AP-150 ($R^{14}$ = hydrogen, a = 0, b = 3), AP-400($R^{14}$ = hydrogen, a = 0, b = 6), AP-550 ($R^{14}$ = hydrogen, a = 0, b = 9). Blemmer® is a trademark of NOF Corporation, Tokyo, Japan.

Examples of semi-hydrophobic monomers under formula VIIIB include methoxypolyethyleneglycol methacrylate available under the product names Visiomer® MPEG 750 MA W ($R^{14}$ = methyl, a = 17, b = 0), MPEG 1005 MA W ($R^{14}$ = methyl, a = 22, b = 0), MPEG 2005 MA W ($R^{14}$ = methyl, a = 45, b = 0), and MPEG 5005 MA W ($R^{14}$ = methyl, a = 113, b = 0) from Evonik Röhm GmbH, Darmstadt, Germany); Bisomer® MPEG 350 MA ($R^{14}$ = methyl, a = 8, b = 0), and MPEG 550 MA ($R^{14}$ = methyl, a = 12, b = 0) from GEO Specialty Chemicals, Ambler PA; Blemmer® PME-100 ($R^{14}$ = methyl, a = 2, b = 0), PME-200 ($R^{14}$ = methyl, a = 4, b = 0), PME-400 ($R^{14}$ = methyl, a = 9, b = 0), PME-1000 ($R^{14}$ = methyl, a = 23, b = 0), PME-4000 ($R^{14}$ = methyl, a = 90, b = 0).

In one aspect, the semi-hydrophobic monomer set forth in formula IX can be represented by the following formulas:

$CH_2=CH-O-(CH_2)_d-O-(C_3H_6O)_e-(C_2H_{4O})_f-H$ IXA

$CH_2=CH-CH_2-O-(C_3H_6O)_9\ (C_2H_4O)_h-H$ IXB

wherein d is an integer of 2, 3, or 4; e is an integer in the range of from 1 to 10 in one aspect, from 2 to 8 in another aspect, and from 3 to 7 in a further aspect; f is an integer in the range of from 5 to 50 in one aspect, from 8 to 40 in another aspect, and from 10 to 30 in a further aspect; g is an integer in the range of from 1 to 10 in one aspect, from 2 to 8 in another aspect, and from 3 to 7 in a further aspect; and h is an integer in the range of from 5 to 50 in one aspect, and from 8 to 40 in another aspect; e, f, g, and h can be 0 subject to the proviso that e and f cannot be 0 at the same time, and g and h cannot be 0 at the same time.

Monomers under formulas IXA and IXB are commercially available under the trade names Emulsogen® R109, R208, R307, RAL109, RAL208, and RAL307 sold by Clariant Corporation; BX-AA-E5P5 sold by Bimax, Inc.; and combinations thereof. EMULSOGEN7 R109 is a randomly ethoxylated/propoxylated 1,4-butanediol vinyl ether having the empirical formula $CH_2=CH-O(CH_2)_4O(C_3H_6O)_4(C_2H_4O)_{10}H$; Emulsogen® R208 is a randomly ethoxylated/propoxylated 1,4-butanediol vinyl ether having the empirical formula $CH_2=CH-O(CH_2)_4O(C_3H_6O)_4(C_2H_4O)_{20}H$; Emulsogen® R307 is a randomly ethoxylated/propoxylated 1,4-butanediol vinyl ether having the empirical formula $CH_2=CH-O(CH_2)_4O(C_3H_6O)_4(C_2H_4O)_{30}H$; Emulsogen® RAL109 is a randomly ethoxylated/propoxylated allyl ether having the empirical formula $CH_2=CHCH_2O(C_3H_6O)_4(C_2H_4O)_{10}H$; Emulsogen® RAL208 is a randomly ethoxylated/propoxylated allyl ether having the empirical formula $CH_2=CHCH_2O(C_3H_6O)_4(C_2H_4O)_{20}H$; Emulsogen® RAL307 is a randomly ethoxylated/propoxylated allyl ether having the empirical formula $CH_2=CHCH_2O(C_3H_6O)_4(C_2H_4O)_{30}H$; and BX-AA-E5P5 is a randomly ethoxylated/propoxylated allyl ether having the empirical formula $CH_2=CHCH_2O(C_3H_6O)_5(C_2H_4O)_5H$.

In the associative and semi-hydrophobic monomers of the invention, the polyoxyalkylene mid-section portion contained in these monomers can be utilized to tailor the hydrophilicity and/or hydrophobicity of the polymers in which they are included. For example, mid-section portions rich in ethylene oxide moieties are more hydrophilic while mid-section portions rich in propylene oxide moieties are more hydrophobic. By adjusting the relative amounts of ethylene oxide to propylene oxide moieties present in these monomers the hydrophilic and hydrophobic properties of the polymers in which these monomers are included can be tailored as desired.

The amount of associative and/or semi-hydrophobic monomer utilized in the preparation of the polymers of the present invention can vary widely and depends, among other things, on the final rheological and aesthetic properties desired in the polymer. When utilized, the monomer reaction mixture contains one or more monomers selected from the

associative and/or semi-hydrophobic monomers disclosed above in amounts ranging from 0.01 to 15 wt.% in one aspect, from 0.1 wt.% to 10 wt.% in another aspect, from 0.5 to 8 wt.% in still another aspect and from 1, 2 or 3 to 5 wt.% in a further aspect, based on the weight of the total monomers.

Ionizable Monomer

In one aspect of the invention, the nonionic, amphiphilic polymer compositions of the invention can be polymerized from a monomer composition including 0 to 5 wt.% of an ionizable and/or ionized monomer, based on the weight of the total monomers, so long as the irritation mitigation properties and the yield stress value of the surfactant compositions in which the polymers of the invention are included are not deleteriously affected.

In another aspect, the amphiphilic polymer compositions of the invention can be polymerized from a monomer composition comprising less than 3 wt.% in one aspect, less than 1 wt.% in a further aspect, less than 0.5 wt.% in a still further aspect, less than 0.1 wt.% in an additional aspect, and less than 0.05 wt.% in a further aspect, of an ionizable and/or an ionized moiety, based on the weight of the total monomers.

Ionizable monomers include monomers having a base neutralizable moiety and monomers having an acid neutralizable moiety. Base neutralizable monomers include olefinically unsaturated monocarboxylic and dicarboxylic acids and their salts containing 3 to 5 carbon atoms and anhydrides thereof. Examples include (meth)acrylic acid, itaconic acid, maleic acid, maleic anhydride, and combinations thereof. Other acidic monomers include styrenesulfonic acid, acrylamidomethylpropanesulfonic acid (AMPS® monomer), vinylsulfonic acid, vinylphosphonic acid, allylsulfonic acid, methallylsulfonic acid; and salts thereof.

Acid neutralizable monomers include olefinically unsaturated monomers which contain a basic nitrogen atom capable of forming a salt or a quaternized moiety upon the addition of an acid. For example, these monomers include vinylpyridine, vinylpiperidine, vinylimidazole, vinylmethylimidazole, dimethylaminomethyl (meth)acrylate, dimethylaminoethyl (meth)acrylate, diethylaminomethyl (meth)acrylate and methacrylate, dimethylaminoneopentyl (meth)acrylate, dimethylaminopropyl (meth)acrylate, and diethylaminoethyl (meth)acrylate.

Crosslinking Monomer

The crosslinked, nonionic, amphiphilic polymers useful in the practice of the invention are polymerized from a monomer composition comprising a first monomer comprising at least one nonionic, hydrophilic unsaturated monomer, at least one nonionic, unsaturated hydrophobic monomer, and mixtures thereof, and a third monomer comprising at least one polyunsaturated crosslinking monomer. The crosslinking monomer(s) is utilized to polymerize covalent crosslinks into the polymer backbone. In one aspect, the crosslinking monomer is a polyunsaturated compound containing at least 2 unsaturated moieties. In another aspect, the crosslinking monomer contains at least 3 unsaturated moieties. Exemplary polyunsaturated compounds include di(meth)acrylate compounds such as ethylene glycol di(meth)acrylate, polyethylene glycol di(meth)acrylate, triethylene glycol di(meth)acrylate, 1,3-butylene glycol di(meth)acrylate, 1,6-butylene glycol di(meth)acrylate, 1,6-hexanediol di(meth)acrylate, neopentyl glycol di(meth)acrylate, 1,9-nonanediol di(meth)acrylate, 2,2'-bis(4-(acryloxy-propyloxyphenyl)propane, and 2,2'-bis(4-(acryloxydiethoxy-phenyl)propane; tri(meth)acrylate compounds such as, trimethylolpropane tri(meth)acrylate, trimethylolethane tri(meth)acrylate, and tetramethylolmethane tri(meth)acrylate; tetra(meth)acrylate compounds such as ditrimethylolpropane tetra(meth)acrylate, tetramethylolmethane tetra(meth)acrylate, and pentaerythritol tetra(meth)acrylate; hexa(meth)acrylate compounds such as dipentaerythritol hexa(meth)acrylate; allyl compounds such as allyl (meth)acrylate, diallylphthalate, diallyl itaconate, diallyl fumarate, and diallyl maleate; polyallyl ethers of sucrose having from 2 to 8 allyl groups per molecule, polyallyl ethers of pentaerythritol such as pentaerythritol diallyl ether, pentaerythritol triallyl ether, and pentaerythritol tetraallyl ether, and combinations thereof; polyallyl ethers of trimethylolpropane such as trimethylolpropane diallyl ether, trimethylolpropane triallyl ether, and combinations thereof. Other suitable polyunsaturated compounds include divinyl glycol, divinyl benzene, and methylenebisacrylamide.

In another aspect, suitable polyunsaturated monomers can be synthesized via an esterification reaction of a polyol made from ethylene oxide or propylene oxide or combinations thereof with unsaturated anhydride such as maleic anhydride, citraconic anhydride, itaconic anhydride, or an addition reaction with unsaturated isocyanate such as 3-isopropenyl-$\alpha$-$\alpha$-dimethylbenzene isocyanate.

Mixtures of two or more of the foregoing polyunsaturated compounds can also be utilized to crosslink the nonionic, amphiphilic polymers of the invention. In one aspect, the mixture of unsaturated crosslinking monomer contains an average of 2 unsaturated moieties. In another aspect, the mixture of crosslinking monomers contains an average of 2.5 unsaturated moieties. In still another aspect, the mixture of crosslinking monomers contains an average of about 3 unsaturated moieties. In a further aspect, the mixture of crosslinking monomers contains an average of about 3.5 unsaturated moieties.

In one embodiment of the invention, the crosslinking monomer component can be used in an amount ranging

from 0.01 to 0.3 wt.% in one aspect, from 0.05 to 0. 3 wt.% in another aspect, and from 0.1 to 0.3wt.% in a further aspect, based on the dry weight of the nonionic, amphiphilic polymer of the invention.

In another embodiment of the invention, the crosslinking monomer component contains an average of 3 unsaturated moieties and can be used in an amount ranging from 0.01 to 0.3 wt.% in one aspect, from 0.02 to 0.25 wt.% in another aspect, from 0.05 to 0.2 wt.% in a further aspect, and from 0.075 to 0.175 wt.% in a still further aspect, and from 0.1 to 0.15 wt.% in another aspect, based upon the total weight of the, nonionic, amphiphilic polymer of the invention.

In one aspect, the crosslinking monomer is selected from trimethylolpropane tri(meth)acrylate, trimethylolethane tri(meth)acrylate, tetramethylolmethane tri(meth)acrylate, pentaerythritol triallylether and polyallyl ethers of sucrose having 3 allyl groups per molecule.

Amphiphilic Polymer Synthesis

The linear and crosslinked, nonionic, amphiphilic, irritation mitigant polymers of the present invention can be made using conventional free-radical emulsion polymerization techniques. The polymerization processes are carried out in the absence of oxygen under an inert atmosphere such as nitrogen. The polymerization can be carried out in a suitable solvent system such as water. Minor amounts of a hydrocarbon solvent, organic solvent, as well as mixtures thereof can be employed. The polymerization reactions are initiated by any means which results in the generation of a suitable free-radical. Thermally derived radicals, in which the radical species is generated from thermal, homolytic dissociation of peroxides, hydroperoxides, persulfates, percarbonates, peroxyesters, hydrogen peroxide and azo compounds can be utilized. The initiators can be water soluble or water insoluble depending on the solvent system employed for the polymerization reaction.

The initiator compounds can be utilized in an amount of up to 30 wt.% in one aspect, 0.01 to 10 wt.% in another aspect, and 0.2 to 3 wt.% in a further aspect, based on the total weight of the dry polymer.

Exemplary free radical water soluble initiators include, but are not limited to, inorganic persulfate compounds, such as ammonium persulfate, potassium persulfate, and sodium persulfate; peroxides such as hydrogen peroxide, benzoyl peroxide, acetyl peroxide, and lauryl peroxide; organic hydroperoxides, such as cumene hydroperoxide and t-butyl hydroperoxide; organic peracids, such as peracetic acid, and water soluble azo compounds, such as 2,2'-azobis(tert-alkyl) compounds having a water solubilizing substituent on the alkyl group. Exemplary free radical oil soluble compounds include, but are not limited to 2,2'-azobisisobutyronitrile, and the like. The peroxides and peracids can optionally be activated with reducing agents, such as sodium bisulfite, sodium formaldehyde, or ascorbic acid, transition metals, hydrazine, and the like.

In one aspect, azo polymerization catalysts include the Vazo® free-radical polymerization initiators, available from DuPont, such as Vazo® 44 (2,2'-azobis(2-(4,5-dihydroimidazolyl)propane), Vazo® 56 (2,2'-azobis(2-methylpropionamidine) dihydrochloride), Vazo® 67 (2,2-azobis(2-methylbutyronitrile)), and Vazo® 68 (4,4'-azobis(4-cyanovaleric acid)).

In emulsion polymerization processes, it can be advantageous to stabilize the monomer/polymer droplets or particles by means of surface active auxiliaries. Typically, these are emulsifiers or protective colloids. Emulsifiers used can be anionic, nonionic, cationic or amphoteric. Examples of anionic emulsifiers are alkylbenzenesulfonic acids, sulfonated fatty acids, sulfosuccinates, fatty alcohol sulfates, alkylphenol sulfates and fatty alcohol ether sulfates. Examples of usable nonionic emulsifiers are alkylphenol ethoxylates, primary alcohol ethoxylates, fatty acid ethoxylates, alkanolamide ethoxylates, fatty amine ethoxylates, EO/PO block copolymers and alkylpolyglucosides. Examples of cationic and amphoteric emulsifiers used are quaternized amine alkoxylates, alkylbetaines, alkylamidobetaines and sulfobetaines.

Optionally, the use of known redox initiator systems as polymerization initiators can be employed. Such redox initiator systems include an oxidant (intiator) and a reductant. Suitable oxidants include, for example, hydrogen peroxide, sodium peroxide, potassium peroxide, t-butyl hydroperoxide, t-amyl hydroperoxide, cumene hydroperoxide, sodium perborate, perphosphoric acid and salts thereof, potassium permanganate, and ammonium or alkali metal salts of peroxydisulfuric acid, typically at a level of 0.01 % to 3.0% by weight, based on dry polymer weight, are used. Suitable reductants include, for example, alkali metal and ammonium salts of sulfur-containing acids, such as sodium sulfite, bisulfite, thiosulfate, hydrosulfite, sulfide, hydrosulfide or dithionite, formadinesulfinic acid, hydroxymethanesulfonic acid, acetone bisulfite, amines such as ethanolamine, glycolic acid, glyoxylic acid hydrate, ascorbic acid, isoascorbic acid, lactic acid, glyceric acid, malic acid, 2-hydroxy-2-sulfinatoacetic acid, tartaric acid and salts of the preceding acids typically at a level of 0.01 % to 3.0% by weight, based on dry polymer weight, is used. In one aspect, combinations of peroxodisulfates with alkali metal or ammonium bisulfites can be used, for example, ammonium peroxodisulfate and ammonium bisulfite. In another aspect, combinations of hydrogen peroxide containing compounds (t-butyl hydroperoxide) as the oxidant with ascorbic or erythorbic acid as the reductant can be utilized. The ratio of peroxide-containing compound to reductant is within the range from 30:1 to 0.05:1.

Examples of typical protective colloids are cellulose derivatives, polyethylene glycol, polypropylene glycol, copolymers of ethylene glycol and propylene glycol, polyvinyl acetate, poly(vinyl alcohol), partially hydrolyzed poly(vinyl

alcohol), polyvinyl ether, starch and starch derivatives, dextran, polyvinylpyrrolidone, polyvinylpyridine, polyethyleneimine, polyvinylimidazole, polyvinylsuccinimide, polyvinyl-2-methylsuccinimide, polyvinyl-1,3-oxazolid-2-one, polyvinyl-2-methylimidazoline and maleic acid or anhydride copolymers. The emulsifiers or protective colloids are customarily used in concentrations from 0.05 to 20 wt.%, based on the weight of the total monomers.

The polymerization reaction can be carried out at temperatures ranging from 20 to 200°C in one aspect, from 50 to 150°C in another aspect, and from 60 to 100°C in a further aspect.

The polymerization can be carried out the presence of chain transfer agents. Suitable chain transfer agents include, but are not limited to, thio- and disulfide containing compounds, such as $C_1$-$C_{18}$ alkyl mercaptans, such as tert-butyl mercaptan, n-octyl mercaptan, n-dodecyl mercaptan, tert-dodecyl mercaptan hexadecyl mercaptan, octadecyl mercaptan; mercaptoalcohols, such as 2-mercaptoethanol, 2-mercaptopropanol; mercaptocarboxylic acids, such as mercaptoacetic acid and 3-mercaptopropionic acid; mercaptocarboxylic acid esters, such as butyl thioglycolate, isooctyl thioglycolate, dodecyl thioglycolate, isooctyl 3-mercaptopropionate, and butyl 3-mercaptopropionate; thioesters; $C_1$-$C_{18}$ alkyl disulfides; aryldisulfides; polyfunctional thiols such as trimethylolpropane-tris-(3-mercaptopropionate), pentaerythritol-tetra-(3-mercaptopropionate), pentaerythritol-tetra-(thioglycolate), pentaerythritol-tetra-(thiolactate), dipentaerythritol-hexa-(thioglycolate), and the like; phosphites and hypophosphites; $C_1$-$C_4$ aldehydes, such as formaldehyde, acetaldehyde, propionaldehyde; haloalkyl compounds, such as carbon tetrachloride, bromotrichloromethane, and the like; hydroxylammonium salts such as hydroxylammonium sulfate; formic acid; sodium bisulfite; isopropanol; and catalytic chain transfer agents such as, for example, cobalt complexes (e.g., cobalt (II) chelates).

The chain transfer agents are generally used in amounts ranging from 0.1 to 10 wt.%, based on the total weight of the monomers present in the polymerization medium.

Emulsion Process

The crosslinked, nonionic, amphiphilic polymer is polymerized via an emulsion process. The emulsion process can be conducted in in a single reactor or in multiple reactors as is well-known in the art. The monomers can be added as a batch mixture or each monomer can be metered into the reactor in a staged process. A typical mixture in emulsion polymerization comprises water, monomer(s), an initiator (usually water-soluble) and an emulsifier. The monomers may be emulsion polymerized in a single-stage, two-stage or multi-stage polymerization process according to well-known methods in the emulsion polymerization art. In a two-stage polymerization process, the first stage monomers are added and polymerized first in the aqueous medium, followed by addition and polymerization of the second stage monomers. The aqueous medium optionally can contain an organic solvent. If utilized the organic solvent is less than about 5 wt.% of the aqueous medium. Suitable examples of water-miscible organic solvents include, without limitation, esters, alkylene glycol ethers, alkylene glycol ether esters, lower molecular weight aliphatic alcohols, and the like.

To facilitate emulsification of the monomer mixture, the emulsion polymerization is carried out in the presence of at least one surfactant. In one embodiment, the emulsion polymerization is carried out in the presence of surfactant (active weight basis) ranging in the amount of 0.2% to 5% by weight in one aspect, from 0.5% to 3% in another aspect, and from 1% to 2% by weight in a further aspect, based on a total monomer weight basis. The emulsion polymerization reaction mixture also includes one or more free radical initiators which are present in an amount ranging from 0.01% to 3% by weight based on total monomer weight. The polymerization can be performed in an aqueous or aqueous alcohol medium. Surfactants for facilitating the emulsion polymerization include anionic, nonionic, amphoteric, and cationic surfactants, as well as mixtures thereof. Most commonly, anionic and nonionic surfactants can be utilized as well as mixtures thereof.

Suitable anionic surfactants for facilitating emulsion polymerizations are well known in the art and include, but are not limited to ($C_6$-$C_{18}$) alkyl sulfates, ($C_6$-$C_{18}$) alkyl ether sulfates (e.g., sodium lauryl sulfate and sodium laureth sulfate), amino and alkali metal salts of dodecylbenzenesulfonic acid, such as sodium dodecyl benzene sulfonate and dimethylethanolamine dodecylbenzenesulfonate, sodium ($C_6$-$C_{16}$) alkyl phenoxy benzene sulfonate, disodium ($C_6$-$C_{16}$) alkyl phenoxy benzene sulfonate, disodium ($C_6$-$C_{16}$) di-alkyl phenoxy benzene sulfonate, disodium laureth-3 sulfosuccinate, sodium dioctyl sulfosuccinate, sodium di-sec-butyl naphthalene sulfonate, disodium dodecyl diphenyl ether sulfonate, disodium n-octadecyl sulfosuccinate, phosphate esters of branched alcohol ethoxylates, and the like.

Nonionic surfactants suitable for facilitating emulsion polymerizations are well known in the polymer art, and include, without limitation, linear or branched $C_8$-$C_{30}$ fatty alcohol ethoxylates, such as capryl alcohol ethoxylate, lauryl alcohol ethoxylate, myristyl alcohol ethoxylate, cetyl alcohol ethoxylate, stearyl alcohol ethoxylate, cetearyl alcohol ethoxylate, sterol ethoxylate, oleyl alcohol ethoxylate, and, behenyl alcohol ethoxylate; alkylphenol alkoxylates, such as octylphenol ethoxylates; and polyoxyethylene polyoxypropylene block copolymers, and the like. Additional fatty alcohol ethoxylates suitable as non-ionic surfactants are described below. Other useful nonionic surfactants include $C_8$-$C_{22}$ fatty acid esters of polyoxyethylene glycol, ethoxylated mono- and diglycerides, sorbitan esters and ethoxylated sorbitan esters, $C_8$-$C_{22}$ fatty acid glycol esters, block copolymers of ethylene oxide and propylene oxide, and combinations thereof. The number of ethylene oxide units in each of the foregoing ethoxylates can range from 2 and above in one

aspect, and from 2 to 150 in another aspect.

Optionally, other emulsion polymerization additives and processing aids which are well known in the emulsion polymerization art, such as auxiliary emulsifiers, protective colloids, solvents, buffering agents, chelating agents, inorganic electrolytes, polymeric stabilizers, biocides, and pH adjusting agents can be included in the polymerization system.

In one embodiment of the invention, the protective colloid or auxiliary emulsifier is selected from poly(vinyl alcohol) that has a degree of hydrolysis ranging from 80 to 95 % in one aspect, and from 85 to 90 % in another aspect.

In a typical two stage emulsion polymerization, a mixture of the monomers is added to a first reactor under inert atmosphere to a solution of emulsifying surfactant (e.g., anionic surfactant) in water. Optional processing aids can be added as desired (e.g., protective colloids, auxiliary emulsifier(s)). The contents of the reactor are agitated to prepare a monomer emulsion. To a second reactor equipped with an agitator, an inert gas inlet, and feed pumps are added under inert atmosphere a desired amount of water and additional anionic surfactant and optional processing aids. The contents of the second reactor are heated with mixing agitation. After the contents of the second reactor reaches a temperature in the range of 55 to 98°C, a free radical initiator is injected into the so formed aqueous surfactant solution in the second reactor, and the monomer emulsion from the first reactor is gradually metered into the second reactor over a period typically ranging from about one half to about four hours. The reaction temperature is controlled in the range of about 45 to about 95°C. After completion of the monomer addition, an additional quantity of free radical initiator can optionally be added to the second reactor, and the resulting reaction mixture is typically held at a temperature of 45 to 95°C for a time period sufficient to complete the polymerization reaction to obtain the polymer emulsion.

In one embodiment, the crosslinked, nonionic, amphiphilic polymers of the invention are selected from an emulsion polymer polymerized from a monomer mixture comprising at least 30 wt.% of at least one $C_1$-$C_4$ hydroxyalkyl (meth)acrylate (e.g., hydroxyethyl methacrylate), 15 to 70 wt.% of at least one $C_1$-$C_{12}$ alkyl acrylate, 5 to 40 wt.% of at least one vinyl ester of a $C_1$-$C_{10}$ carboxylic acid (based on the weight of the total monomers), and 0.01 to 1 wt.% at least one crosslinker (based on the dry weight of the polymer).

In another aspect, the crosslinked, nonionic, amphiphilic polymers of the invention are selected from an emulsion polymer polymerized from a monomer mixture comprising at least 30 wt.% hydroxyethyl methacrylate, 15 to 35 wt.% ethyl acrylate, 5 to 25 wt.% butyl acrylate, 10 to 25 wt.% of a vinyl ester of a $C_1$-$C_5$ carboxylic acid selected from vinyl, acetate, vinyl propionate, vinyl butyrate, vinyl isobutyrate, and vinyl valerate (said weight percent is based on the weight of the total monomers), and from 0.01 to 0.3 wt.% of a crosslinking monomer having an average of at least 3 crosslinkable unsaturated groups (based on the dry weight of the polymer).

In another embodiment, the crosslinked, nonionic, amphiphilic polymers of the invention are selected from an emulsion polymer polymerized from a monomer mixture comprising from 30 to 60 wt.% of at least one $C_1$-$C_4$ hydroxyalkyl (meth)acrylate (e.g., hydroxyethyl methacrylate), 15 to 70 wt.% of at least one $C_1$-$C_{12}$ alkyl acrylate (at least one $C_1$-$C_5$ alkyl acrylate in another aspect), from 0.1 to 10 wt. of at least one associative and/or semi-hydrophobic monomer (based on the weight of the total monomers), and from 0.01 to 1 wt.% at least one crosslinker (based on the dry weight of the polymer).

In another embodiment, the crosslinked, nonionic, amphiphilic polymers of the invention are selected from an emulsion polymer polymerized from a monomer mixture comprising from 35 to 50 wt.% of at least one $C_1$-$C_4$ hydroxyalkyl (meth)acrylate (e.g., hydroxyethyl methacrylate), 15 to 60 wt.% of at least one $C_1$-$C_{12}$ alkyl acrylate (at least one $C_1$-$C_5$ alkyl acrylate in another aspect), from 0.1 to 10 wt.% of at least one associative and/or semi-hydrophobic monomer (based on the weight of the total monomers), and from 0.01 to 1 wt.% at least one crosslinker (based on the dry weight of the polymer).

In another embodiment, the crosslinked, nonionic, amphiphilic polymers of the invention are selected from an emulsion polymer polymerized from a monomer mixture comprising from 40 to 45 wt.% of at least one $C_1$-$C_4$ hydroxyalkyl (meth)acrylate (e.g., hydroxyethyl methacrylate), 15 to 60 wt.% of at least two different $C_1$-$C_5$ alkyl acrylate monomers, from 1 to 5 wt.% of at least one associative and/or semi-hydrophobic monomer (based on the weight of the total monomers), and from 0.01 to 1 wt.% at least one crosslinker (based on the dry weight of the polymer).

In another embodiment, the crosslinked, nonionic, amphiphilic polymers of the invention are selected from an emulsion polymer polymerized from a monomer mixture comprising from 40 to 45 wt.% of hydroxyethyl acrylate, 30 to 50 wt. % of ethyl acrylate, 10 to 20 wt.% of butyl acrylate and from 1 to 5 wt.% of at least one associative and/or semi-hydrophobic monomer (based on the weight of the total monomers), and from 0.01 to 1 wt.% at least one crosslinker (based on the weight of the dry polymer).

Detersive Compositions

Surprisingly, the present crosslinked, nonionic, amphiphilic, irritation mitigant polymers can be activated by a surfactant to provide a stable yield stress cleansing composition with desirable rheological and aesthetic properties and the ability to suspend particulate and insoluble materials in an aqueous medium for indefinite periods of time independent of pH. The yield stress value, elastic modulus and optical clarity are substantially independent of pH in the compositions

in which the present polymers are included. The nonionic, amphiphilic, irritation mitigant polymers of the invention are useful in the pH range of from 2 to 14 in one aspect, from 3 to 11 in another aspect, and from 4 to 9 in a further aspect. Unlike the pH responsive crosslinked polymers (acid or base sensitive) that require neutralization with an acid or a base to impart a desired rheological profile, the crosslinked, nonionic, amphiphilic polymers of the rheological profiles of the invention are substantially independent of pH. By substantially independent of pH is meant that the yield stress fluid within which the polymer of the invention is included imparts a desired rheological profile (e.g., a yield stress of at least 0.1 Pa in one aspect, at least at least 0.5 Pa in another aspect, at least 1 Pa in still another aspect, and at least 2 Pa in a further aspect) across a wide pH range (e.g., from 2 to 14) wherein the standard deviation in yield stress values across the pH range is less than 1 Pa in one aspect, less than 0.5 Pa in another aspect, and less than 0.25 Pa in a further aspect of the invention.

In one exemplary aspect of the invention, the cleansing compositions comprise: i) at least one nonionic, amphiphilic, irritation mitigant polymer of the invention; ii) at least one surfactant selected from at least one anionic surfactant, at least one amphoteric surfactant, at least one nonionic surfactant, and combinations thereof; and iii) water.

In another exemplary aspect of the invention, the cleansing compositions comprise: i) at least one crosslinked, nonionic, amphiphilic irritation mitigant polymer of the invention; ii) at least one anionic surfactant; and iii) water.

In another exemplary aspect of the invention, the cleansing compositions comprise: i) at least one crosslinked, nonionic, amphiphilic, irritation mitigant polymer of the invention; ii) at least one anionic surfactant and at least one amphoteric surfactant; and iii) water.

In another exemplary aspect of the invention, the cleansing compositions comprise: i) at least one crosslinked, nonionic, amphiphilic irritation mitigant polymer of the invention; ii) at least one anionic surfactant, iii) an optional nonionic surfactant; and iv) water.

In another exemplary aspect of the invention, the cleansing compositions comprise: i) at least one crosslinked, nonionic, amphiphilic irritation mitigant polymer of the invention; ii) at least one anionic surfactant, iii) an amphoteric surfactant; iv) an optional nonionic surfactant; and v) water.

In another exemplary aspect of the invention, the cleansing compositions comprise: i) at least one crosslinked, nonionic, amphiphilic irritation mitigant polymer of the invention; ii) at least one anionic ethoxylated surfactant; iii) an optional nonionic surfactant; and iv) water. In one aspect, the average degree of ethoxylation in the anionic ethoxylated surfactant can range from about 1 to about 3. In another aspect, the average degree of ethoxylation is about 2.

In another exemplary aspect of the invention, the cleansing compositions comprise: i) at least one crosslinked, nonionic, amphiphilic irritation mitigant polymer of the invention; ii) at least one anionic ethoxylated surfactant; iii) at least one amphoteric surfactant, iv) an optional nonionic surfactant; and iv) water. In one aspect, the average degree of ethoxylation in the anionic ethoxylated surfactant can range from 1 to 3. In another aspect, the average degree of ethoxylation is 2.

In another exemplary aspect of the invention, the cleansing compositions comprise: i) at least one crosslinked, nonionic, amphiphilic irritation mitigant polymer of the invention; ii) at least one anionic non-ethoxylated surfactant; iii) at least one anionic ethoxylated surfactant; iv) an optional nonionic surfactant; and v) water. In one aspect, the average degree of ethoxylation in the anionic ethoxylated surfactant can range from 1 to 3. In another aspect, the average degree of ethoxylation is 2.

In another exemplary aspect of the invention, the cleansing compositions comprise: i) at least one crosslinked, nonionic, amphiphilic irritation mitigant polymer of the invention; ii) at least one anionic non-ethoxylated surfactant; iii) at least one anionic ethoxylated surfactant; iv) at least one amphoteric surfactant; v) an optional nonionic surfactant; and vi) water. In one aspect, the average degree of ethoxylation in the anionic ethoxylated surfactant can range from 1 to 3. In another aspect, the average degree of ethoxylation is 2.

Any amount of the nonionic, amphiphilic polymeric material can be utilized so long as the amount is effective to induce a reduction in irritation to the skin and/or eyes when included in a cleansing composition comprising at least one surfactant selected from anionic surfactants, amphoteric surfactants, nonionic surfactants, and combinations thereof. In one aspect, the amount of irritation mitigant polymer that can be incorporated into the surfactant containing cleansing compositions of the invention ranges from 0.5 to 5 wt.% polymer solids (100 % active polymer) based on the weight of the total composition. In another aspect, the amount of polymer utilized in the formulation ranges from 0.75 wt.% to 3.5 wt.%. In still another aspect, the amount of amphiphilic polymer employed in the cleansing composition ranges from 1 to 3 wt.%. In a further aspect, the amount of polymer employed in the cleansing composition ranges from 1.5 wt.% to 2.75 wt.%. In a still further aspect, the amount of polymer utilized in the cleansing composition ranges from 2 to 2.5 wt.%.

In one aspect, the at least one nonionic, amphiphilic polymer utilized in formulating the mild cleansing compositions of the invention is a linear polymer. In one aspect, the number average molecular weight ($M_n$) of the linear copolymeric mitigants of the present invention as measured by gel permeation chromatography (GPC) calibrated with a poly(methyl methacrylate) (PMMA) standard is 500,000 daltons or less. In another aspect the molecular weight is 100,000 daltons or less. In still another aspect, the molecular weight ranges between 5,000 and 80,000 daltons, in a further aspect between 10,000 and 50,000 daltons, and in a still further aspect between 15,000 and 40,000 daltons

In another aspect, the at least one nonionic, amphiphilic polymer utilized in formulating the mild cleansing compositions of the invention is crosslinked. The crosslinked nonionic, amphiphilic polymers of the invention are random copolymers and have weight average molecular weights ranging from above 500,000 to at least a billion Daltons or more in one aspect, and from 600,000 to 4.5 billion Daltons in another aspect, and from 1,000,000 to 3,000,000 Daltons in a further aspect, and from 1,500,000 to 2,000,000 Daltons in a still further aspect (see TDS-222, October 15, 2007, Lubrizol Advanced Materials, Inc).

Detersive Surfactants

The surfactants utilized to formulate the mild cleansing compositions of the invention are from at least one detersive surfactant selected from anionic surfactants, amphoteric surfactants, nonionic surfactants, and mixtures thereof.
Non-limiting examples of anionic surfactants are disclosed in McCutcheon's Detergents and Emulsifiers, North American Edition, 1998, published by Allured Publishing Corporation; and McCutcheon's, Functional Materials, North American Edition (1992). The anionic surfactant can be any of the anionic surfactants known or previously used in the art of aqueous surfactant compositions. Suitable anionic surfactants include but are not limited to alkyl sulfates, alkyl ether sulfates, alkyl sulphonates, alkaryl sulfonates, α-olefin-sulphonates, alkylamide sulphonates, alkarylpolyether sulphates, alkylamidoether sulphates, alkyl monoglyceryl ether sulfates, alkyl monoglyceride sulfates, alkyl monoglyceride sulfonates, alkyl succinates, alkyl sulfosuccinates, alkyl sulfosuccinamates, alkyl ether sulphosuccinates, alkyl amidosulfosuccinates; alkyl sulphoacetates, alkyl phosphates, alkyl ether phosphates, alkyl ether carboxylates, alkyl amidoethercarboxylates, N-alkylamino acids, N-acyl amino acids, alkyl peptides, N-acyl taurates, alkyl isethionates, carboxylate salts wherein the acyl group is derived from fatty acids; and the alkali metal, alkaline earth metal, ammonium, amine, and triethanolamine salts thereof.
In one aspect, the cation moiety of the forgoing salts is selected from sodium, potassium, magnesium, ammonium, mono-, di- and triethanolamine salts, and mono-, di-, and tri-isopropylamine salts. The alkyl and acyl groups of the foregoing surfactants contain from 6 to 24 carbon atoms in one aspect, from 8 to 22 carbon atoms in another aspect and from 12 to 18 carbon atoms in a further aspect and can be saturated or unsaturated. The aryl groups in the surfactants are selected from phenyl or benzyl. The ether containing surfactants set forth above can contain from 1 to 10 ethylene oxide and/or propylene oxide units per surfactant molecule in one aspect, and from 1 to 3 ethylene oxide units per surfactant molecule in another aspect.
Examples of suitable anionic surfactants include but are not limited to the sodium, potassium, lithium, magnesium, and ammonium salts of laureth sulfate, trideceth sulfate, myreth sulfate, $C_{12}$-$C_{13}$ pareth sulfate, $C_{12}$-$C_{14}$ pareth sulfate, and $C_{12}$-$C_{15}$ pareth sulfate, ethoxylated with 1, 2, 3, 4 or 5 moles of ethylene oxide; sodium, potassium, lithium, magnesium, ammonium, and triethanolamine lauryl sulfate, coco sulfate, tridecyl sulfate, myrstyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium $C_{12}$-$C_{14}$ olefin sulfonate, sodium laureth-6 carboxylate, sodium methyl cocoyl taurate, sodium cocoyl glycinate, sodium myristyl sarcocinate, sodium dodecylbenzene sulfonate, sodium cocoyl sarcosinate, sodium cocoyl glutamate, potassium myristoyl glutamate, triethanolamine monolauryl phosphate, and fatty acid soaps, including the sodium, potassium, ammonium, and triethanolamine salts of a saturated and unsaturated fatty acids containing from 8 to 22 carbon atoms.
The term "amphoteric surfactant" as used herein, is also intended to encompass zwitterionic surfactants, which are well known to formulators skilled in the art as a subset of amphoteric surfactants. Nonlimiting examples of amphoteric surfactants are disclosed McCutcheon's Detergents and Emulsifiers, North American Edition, supra, and McCutcheon's, Functional Materials, North American Edition, supra; both of which are incorporated by reference herein in their entirety. Suitable examples include but are not limited to amino acids (e.g., N-alkyl amino acids and N-acyl amino acids), betaines, sultaines, and alkyl amphocarboxylates.
Amino acid based surfactants suitable in the practice of the present invention include surfactants represented by the formula:

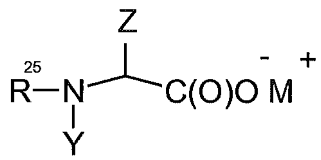

wherein $R^{25}$ represents a saturated or unsaturated hydrocarbon group having 10 to 22 carbon atoms or an acyl group containing a saturated or unsaturated hydrocarbon group having 9 to 22 carbon atoms, Y is hydrogen or methyl, Z is selected from hydrogen, $-CH_3$, $-CH(CH_3)_2$, $-CH_2CH(CH_3)_2$, $-CH(CH_3)CH_2CH_3$, $-CH_2C_6H_5$, $-CH_2C_6H_4OH$, $-CH_2OH$, $-CH(OH)CH_3$, $-(CH_2)_4NH_2$, $-(CH_2)_3NHC(NH)NH_2$, $-CH_2C(O)O^-M^+$, $-(CH_2)_2C(O)O^-M^+$. M is a salt forming cation. In one

aspect, $R^{25}$ represents a radical selected from a linear or branched $C_{10}$ to $C_{22}$ alkyl group, a linear or branched $C_{10}$ to $C_{22}$ alkenyl group, an acyl group represented by $R^{26}C(O)$-, wherein $R^{26}$ is selected from a linear or branched $C_9$ to $C_{22}$ alkyl group, a linear or branched $C_9$ to $C_{22}$ alkenyl group. In one aspect, $M^+$ is a cation selected from sodium, potassium, ammonium, and triethanolamine (TEA).

The amino acid surfactants can be derived from the alkylation and acylation of $\alpha$-amino acids such as, for example, alanine, arginine, aspartic acid, glutamic acid, glycine, isoleucine, leucine, lysine, phenylalanine, serine, tyrosine, and valine. Representative N-acyl amino acid surfactants are, but not limited to the mono- and di- carboxylate salts (e.g., sodium, potassium, ammonium and TEA) of N-acylated glutamic acid, for example, sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, and potassium myristoyl glutamate; the carboxylate salts (e.g., sodium, potassium, ammonium and TEA) of N-acylated alanine, for example, sodium cocoyl alaninate, and TEA lauroyl alaninate; the carboxylate salts (e.g., sodium, potassium, ammonium and TEA) of N-acylated glycine, for example, sodium cocoyl glycinate, and potassium cocoyl glycinate; the carboxylate salts (e.g., sodium, potassium, ammonium and TEA) of N-acylated sarcosine, for example, sodium lauroyl sarcosinate, sodium cocoyl sarcosinate, sodium myristoyl sarcosinate, sodium oleoyl sarcosinate, and ammonium lauroyl sarcosinate; and mixtures of the foregoing surfactants.

The betaines and sultaines useful in the present invention are selected from alkyl betaines, alkylamino betaines, and alkylamido betaines, as well as the corresponding sulfobetaines (sultaines) represented by the formulas:

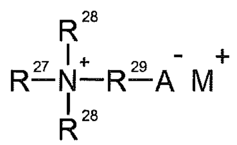

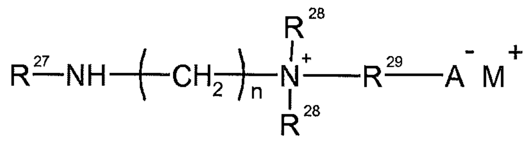

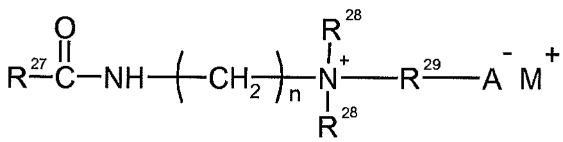

wherein $R^{27}$ is a $C_7$-$C_{22}$ alkyl or alkenyl group, each $R^{28}$ independently is a $C_1$-$C_4$ alkyl group, $R^{29}$ is a $C_1$-$C_5$ alkylene group or a hydroxy substituted $C_1$-$C_5$ alkylene group, n is an integer from 2 to 6, A is a carboxylate or sulfonate group, and M is a salt forming cation. In one aspect, $R^{27}$ is a $C_{11}$-$C_{18}$ alkyl group or a $C_{11}$-$C_{18}$ alkenyl group. In one aspect, $R^{28}$ is methyl. In one aspect, $R^{29}$ is methylene, ethylene or hydroxy propylene. In one aspect, n is 3. In a further aspect, M is selected from sodium, potassium, magnesium, ammonium, and mono-, di- and triethanolamine cations.

Examples of suitable betaines include, but are not limited to, lauryl betaine, coco betaine, oleyl betaine, cocohexadecyl dimethylbetaine, lauryl amidopropyl betaine, cocoamidopropyl betaine (CAPB), and cocamidopropyl hydroxysultaine.

The alkylamphocarboxylates such as the alkylamphoacetates and alkylamphopropionates (mono- and disubstituted carboxylates) can be represented by the formula:

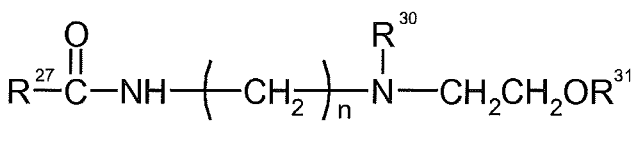

wherein $R^{27}$ is a $C_7$-$C_{22}$ alkyl or alkenyl group, $R^{30}$ is $-CH_2C(O)O^- M^+$, $- CH_2CH_2C(O)O^-M^+$, or $-CH_2CH(OH)CH_2SO_3^- M^+$, $R^{31}$ is hydrogen or $-CH_2C(O)O^-M^+$, and M is a cation selected from sodium, potassium, magnesium, ammonium, and mono-, di- and triethanolamine.

Exemplary alkylamphocarboxylates include, but are not limited to, sodium cocoamphoacetate, sodium lauroamphoacetate, sodium capryloamphoacetate, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, and disodium capryloamphodipropionate.

Non-limiting examples of nonionic surfactants are disclosed in McCutcheon's Detergents and Emulsifiers, North American Edition, 1998, supra; and McCutcheon's, Functional Materials, North American, supra. Additional Examples of nonionic surfactants are described in U.S. Patent No. 4,285,841, to Barrat et al., and U.S. Patent. No. 4,284,532, to Leikhim et al. Nonionic surfactants typically have a hydrophobic portion, such as a long chain alkyl group or an alkylated aryl group, and a hydrophilic portion containing various degrees of ethoxylation and/or propoxylation (e.g., 1 to 50) ethoxy and/or propoxy moieties. Examples of some classes of nonionic surfactants that can be used include, but are not limited to, ethoxylated alkylphenols, ethoxylated and propoxylated fatty alcohols, polyethylene glycol ethers of methyl glucose, polyethylene glycol ethers of sorbitol, ethylene oxide-propylene oxide block copolymers, ethoxylated esters of fatty acids, condensation products of ethylene oxide with long chain amines or amides, condensation products of ethylene oxide with alcohols, and mixtures thereof.

Suitable nonionic surfactants include, for example, alkyl polysaccharides, alcohol ethoxylates, block copolymers, castor oil ethoxylates, ceto/oleyl alcohol ethoxylates, cetearyl alcohol ethoxylates, decyl alcohol ethoxylates, dinonyl phenol ethoxylates, dodecyl phenol ethoxylates, endcapped ethoxylates, ether amine derivatives, ethoxylated alkanolamides, ethylene glycol esters, fatty acid alkanolamides, fatty alcohol alkoxylates, lauryl alcohol ethoxylates, mono-branched alcohol ethoxylates, nonyl phenol ethoxylates, octyl phenol ethoxylates, oleyl amine ethoxylates, random copolymer alkoxylates, sorbitan ester ethoxylates, stearic acid ethoxylates, stearyl amine ethoxylates, tallow oil fatty acid ethoxylates, tallow amine ethoxylates, tridecanol ethoxylates, acetylenic diols, polyoxyethylene sorbitols, and mixtures thereof. Various specific examples of suitable nonionic surfactants include, but are not limited to, methyl gluceth-10, PEG-20 methyl glucose distearate, PEG-20 methyl glucose sesquistearate, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, or ethoxylated fatty ($C_6$-$C_{22}$) alcohol, including 3 to 20 ethylene oxide moieties, polyoxyethylene-20 isohexadecyl ether, polyoxyethylene-23 glycerol laurate, polyoxyethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxyethylene-6 tridecyl ether, laureth-2, laureth-3, laureth-4, PEG-3 castor oil, PEG 600 dioleate, PEG 400 dioleate, poloxamers such as poloxamer 188, polysorbate 21, polysorbate 40, polysorbate 60, polysorbate 61, polysorbate 65, polysorbate 80, polysorbate 81, polysorbate 85, sorbitan caprylate, sorbitan cocoate, sorbitan diisostearate, sorbitan dioleate, sorbitan distearate, sorbitan fatty acid ester, sorbitan isostearate, sorbitan laurate, sorbitan oleate, sorbitan palmitate, sorbitan sesquiisostearate, sorbitan sesquioleate, sorbitan sesquistearate, sorbitan stearate, sorbitan triisostearate, sorbitan trioleate, sorbitan tristearate, sorbitan undecylenate, or mixtures thereof.

Alkyl glycoside nonionic surfactants can also be employed and are generally prepared by reacting a monosaccharide, or a compound hydrolyzable to a monosaccharide, with an alcohol such as a fatty alcohol in an acid medium. For example, U.S. Patent Nos. 5,527,892 and 5,770,543 describe alkyl glycosides and/or methods for their preparation. Suitable examples are commercially available under the names of Glucopon™ 220, 225, 425, 600 and 625, PLANTACARE®, and PLANTAPON®, all of which are available from Cognis Corporation of Ambler, Pennsylvania.

In another aspect, nonionic surfactants include, but are not limited to, alkoxylated methyl glucosides such as, for example, methyl gluceth-10, methyl gluceth-20, PPG-10 methyl glucose ether, and PPG-20 methyl glucose ether, available from Lubrizol Advanced Materials, Inc., under the trade names, Glucam® E10, Glucam® E20, Glucam® P10, and Glucam® P20, respectively; and hydrophobically modified alkoxylated methyl glucosides, such as PEG 120 methyl glucose dioleate, PEG-120 methyl glucose trioleate, and PEG-20 methyl glucose sesquistearate, available from Lubrizol Advanced Materials, Inc., under the trade names, Glucamate® DOE-120, Glucamate™ LT, and Glucamate™ SSE-20, respectively, are also suitable. Other exemplary hydrophobically modified alkoxylated methyl glucosides are disclosed in U.S. Patent Nos. 6,573,375 and 6,727,357.

Other useful nonionic surfactants include water soluble silicones such as PEG-10 Dimethicone, PEG-12 Dimethicone, PEG-14 Dimethicone, PEG-17 Dimethicone, PPG-12 Dimethicone, PPG-17 Dimethicone and derivatized/functionalized forms thereof such as Bis-PEG/PPG-20/20 Dimethicone Bis-PEG/PPG-16/16 PEG/PPG-16/16 Dimethicone, PEG/PPG-14/4 Dimethicone, PEG/PPG-20/20 Dimethicone, PEG/PPG-20/23 Dimethicone, and Perfluorononylethyl Carboxydecyl PEG-10 Dimethicone.

The amount of the at least one surfactant (active weight basis) utilized in formulating the cleansing compositions of the invention ranges from 1 to 30 wt.% based on the weight of the total composition. In another aspect, the amount of the at least one surfactant utilized in the formulation of the cleansing composition ranges from 3 to 25 wt.%. In still another aspect, the amount of the at least one surfactant employed in the cleansing composition ranges from 5 to 22 wt.%. In a further aspect, the amount of the at least one surfactant utilized ranges from 6 to 20 wt.%. In still a further aspect, the amount of at least one surfactant is 10, 12, 14, 16, and 18 wt.% based on the total weight yield of the cleansing composition.

In one embodiment of the invention, the weight ratio (based on active material) of anionic surfactant (non-ethoxylated and/or ethoxylated) to amphoteric surfactant can range from 10:1 to 2:1 in one aspect, and can be 9:1, 8:1, 7:1 6:1, 5:1, 4.5:1, 4:1, or 3:1 in another aspect. When employing an ethoxylated anionic surfactant in combination with a non-ethoxylated anionic surfactant and an amphoteric surfactant, the weight ratio (based on active material) of ethoxylated anionic surfactant to non-ethoxylated anionic surfactant to amphoteric surfactant can range from 3.5:3.5:1 in one aspect to 1:1:1 in another aspect.

The yield stress value of the cleansing composition containing the linear, nonionic, amphiphilic polymers of the invention is 0 Pa.

In one embodiment, the yield stress value of the cleansing composition containing the crosslinked nonionic, amphiphilic polymers of the invention is at least 0.1 Pa in one aspect, 0.5 Pa in one aspect, at least 1 Pa in another aspect and at least 1.5 Pa in a further aspect. In another embodiment, the yield stress of the cleansing composition ranges from 0.1 to 20 Pa in one aspect, from 0.5 Pa to 10 Pa in another aspect, from 1 to 3 Pa in a further aspect, and from 1.5 to 3.5 in a still further aspect.

Optionally, the cleansing compositions of the invention can contain an electrolyte. Suitable electrolytes are known compounds and include salts of multivalent anions, such as potassium pyrophosphate, potassium tripolyphosphate, and sodium or potassium citrate, salts of multivalent cations, including alkaline earth metal salts such as calcium chloride and calcium bromide, as well as zinc halides, barium chloride and calcium nitrate, salts of monovalent cations with monovalent anions, including alkali metal or ammonium halides, such as potassium chloride, sodium chloride, potassium iodide, sodium bromide, and ammonium bromide, alkali metal or ammonium nitrates, and blends thereof. The amount of the electrolyte used will generally depend on the amount of the amphiphilic polymer incorporated, but may be used at concentration levels of from 0.1 to 4 wt.% in one aspect and from 0.2 to 2 wt.% in another aspect, based on the weight of the total composition.

The cleansing composition must be easily pourable with a shear thinning index of less than 0.5 at shear rates between 0.1 and 1 reciprocal second. The cleansing compositions of the invention can be utilized in combination with an auxiliary rheology modifier (thickener) to enhance the yield value of a thickened liquid. In one aspect, a rheology modifier can be combined with a nonionic rheology modifier to attain a desired yield stress value when a linear irritation mitigation polymer is utilized. Any rheology modifier is suitable including, but are not limited to, natural gums (e.g., polygalactomannan gums selected from fenugreek, cassia, locust bean, tara and guar), modified cellulose (e.g., ethylhexylethylcellulose (EHEC), hydroxybutylmethylcellulose (HBMC), hydroxyethylmethylcellulose (HEMC), hydroxypropylmethylcellulose (HPMC), methyl cellulose (MC), hydroxyethylcellulose (HEC), hydroxypropylcellulose (HPC) and cetyl hydroxyethylcellulose); and mixtures thereofmethylcellulose, polyethylene glycols (e.g., PEG 4000, PEG 6000, PEG 8000, PEG 10000, PEG 20000), polyvinyl alcohol, polyacrylamides (homopolymers and copolymers), and hydrophobically modified ethoxylated urethanes (HEUR). The rheology modifier can be utilized in an amount ranging from 0.5 to 25 wt.% in one aspect, from 1 to 15 wt.% in another aspect, and from 2 to 10 wt.% in a further aspect, and from 2.5 to 5 wt. %based on the weight of the total weight of the composition.

The linear and crosslinked, nonionic, amphiphilic polymers of the invention can be used in any cleansing or detersive application to mitigate irritation induced by harsh surfactants to the skin and/or eyes. The linear polymers of the invention can be utilized in detersive formulations where irritation mitigation is desirable but an increase in yield value or thickening is not. The crosslinked, nonionic, amphiphilic polymers of the invention can be used in any cleansing or detersive application to mitigate irritation induced by harsh surfactants to the skin and/or the eyes and requires a concomitant enhancement of yield stress properties.

In one embodiment, crosslinked, nonionic, amphiphilic polymers of the invention can be utilized to mitigate irritation to the skin and/or the eyes as well as to stably suspend particulate materials and insoluble droplets within a surfactant containing cleansing and cleaning composition formulated for the personal care and home care industries.

In the personal care industry, the crosslinked, nonionic, amphiphilic polymers of the invention can be utilized to improve mildness and the yield stress properties of cleansing compositions for the hair and skin, and can be utilized for the stable suspension of insoluble silicones, opacifiers and pearlescent agents (e.g., mica, coated mica, ethylene glycol monostearate (EGMS), ethylene glycol distearate (EGDS), polyethylene glycol monostearate (PGMS) or polyethyleneglycol distearate (PGDS)), pigments, exfoliants, antidandruff agents, clay, swellable clay, laponite, gas bubbles, liposomes, microsponges, cosmetic beads, cosmetic microcapsules, and flakes. These compositions may be in the form of a body wash, shower gel, bubble bath, two-in-one shampoo, conditioner, facial scrub, moisture rinse, make-up removal product, and the like.

Exemplary bead components include, but are not limited to, agar beads, alginate beads, jojoba beads, gelatin beads, Styrofoam™ beads, polyacrylate, polymethylmethacrylate (PMMA), polyethylene beads, Unispheres™ and Unipearls™ cosmetic beads (Induchem USA, Inc., New York, NY), Lipocapsule™, Liposphere™, and Lipopearl™ microcapsules (Lipo Technologies Inc., Vandalia, OH), and Confetti II™ dermal delivery flakes (United-Guardian, Inc., Hauppauge, NY). Beads can be utilized as aesthetic materials or can be used to encapsulate benefit agents to protect them from the deteriorating effects of the environment or for optimal delivery, release and performance in the final product.

In one aspect, the cosmetic beads range in size from 0.5 to 1.5 mm. In another aspect, the difference in specific gravity of the bead and water is between +/- 0.01 and 0.5 in one aspect and from +/-0.2 to 0.3 g/ml in another aspect.

In one aspect, the microcapsules range in size from 0.5 to 300 μm. In another aspect, the difference in specific gravity between the microcapsules and water is from +/- 0.01 to 0.5. Non-limiting examples of microcapsule beads are disclosed in U.S. Patent No. 7,786,027,

In one aspect of the invention, the amount of particulate component and/or insoluble droplets can range from 0.1 % to 10% by weight based on the total weight of the composition.

The stable compositions maintain a smooth, acceptable rheology with good shear thinning properties without significant increases or decreases in viscosity, with no phase separation, e.g., settling or creaming out (rising to the surface), or loss of clarity over extended periods of time, such as for at least one month at 45°C.

In the home care industry, the crosslinked, nonionic, amphiphilic polymers of the invention can be utilized to improve mildness and the yield stress properties of detersive compositions for hard surfaces (e.g., floors, countertops, walls, wood surfaces, appliances, tile, and the like), fabric care, dish care, for improvement of cling (toilet bowl, sink and vertical surface cleaners) and for the stable suspension of abrasive materials in abrasive cleaners.

While overlapping weight ranges for the various components and ingredients that can be contained in the yield stress fluids of the invention have been expressed for selected embodiments and aspects of the invention, it should be readily apparent that the specific amount of each component in the compositions will be selected from its disclosed range such that the amount of each component is adjusted so that the sum of all components in the composition will total 100 weight percent. The amounts employed will vary with the purpose and character of the desired product and can be readily determined by one skilled in the formulation art and from the literature.

This invention is illustrated by the following examples that are merely for the purpose of illustration. Unless specifically indicated otherwise, parts and percentages are given by weight.

The following abbreviations and trade names are utilized in the examples.

Abbreviations and Trade Names

| | |
|---|---|
| AA | Acrylic Acid |
| AMD | Acrylamide |
| AMPS® Monomer | 2-Acrylamido-2-Methylpropanesulfonic Acid, Lubrizol Advanced Materials, Inc. |
| AN | Acrylonitrile |
| APE | Allyl Pentaerythritol |
| n-BA | n-Butyl Acrylate |
| BDGMA | Butyl Diglycol Methacrylate |
| BEM | Sipomer® Ethoxylated (25) Behenyl Methacrylate, Rhodia |
| | |
| i-BMA | iso-Butyl Methacrylate |
| s-BMA | sec-Butyl Methacrylate |
| ChembetaineTM CAD | Cocamidopropyl Betaine (amphoteric surfactant), Lubrizol Advanced Materials, Inc. (35% active) |
| CSEM | Visiomer® C18 PEG 1105 MA W Polyethyleneglycol (25) Cetearyl Methacrylate, Evonik Röhm GmbH |
| CYCLO | Cyclohexane |
| Celvol® 502 PVA | Polyvinyl Alcohol (hydrolysis % = 87-89%), Celanese Corpoartion |
| EA | Ethyl Acrylate |
| EMA | Ethyl Methacrylate |
| HBMA | 4-Hydroxybutyl Methacrylate |
| 2-HEA | 2-Hydroxyethyl Acrylate |
| HEMA | 2-Hydroxyethyl Methacrylate |
| HPA | Hydroxypropyl Acrylate |

(continued)

| | |
|---|---|
| HPMA | 3-Hydroxypropyl Methacrylate |
| LEM | Blemmer® PLE-200 Lauroxy Polyethyleneglycol Methacrylate, NOF Corporation |
| LMA | Lauryl Methacrylate |
| MA | Methyl Acrylate |
| MAA | Methacrylic Acid |
| MA EO/PO-300 | Blemmer® 50PEP-300 Polyethyleneglycol (3.5) Polypropyleneglycol (2.5) Methacrylate, NOF Corporation |
| MA EO/PO-800 | ,Blemmer® 55PET-800 Polyethyleneglycol (10) Polypropyleneglycol (5) Methacrylate, NOF Corporation |
| MAMD | Methacrylamide |
| MMA | Methyl Methacrylate |
| MPEG 350 | Bisomer® 350 MA Methoxy Polyethyleneglycol (8) Methacrylate, GEO Specialty Chemicals |
| MPEG 400 | Blemmer® PME-400 Methoxy Polyethyleneglycol (9) Methacrylate, NOF Corporation |
| MPEG S10 W | Bisomer® S10 W Methoxy Polyethyleneglycol (23) Methacrylate, GEO Specialty Chemicals |
| NPEA-1300 | Blemmer® ANE-1300, Nonylphenoxy Polyethyleneglycol (30) Acrylate, NOF Corporation |
| OEO/POMA | Blemmer® 50POEP-800B Octoxy Polyethyleneglycol (8) Polypropyleneglycol (6) Methacrylate, NOF Corporation (hydrophobe = 2-ethylhexyl) |
| PEA | Blemmer® AAE-300 Phenoxy Polyethyleneglycol (5.5) acrylate, NOF Corporation |
| PEO/POMA | Blemmer® 43PAPE-600B Phenoxy Polyethyleneglycol (6) Polypropyleneglycol (6) Methacrylate, NOF Corporation |
| SEM-400 | Blemmer® PSE-400 Stearoxy Polyethyleneglycol (9) Methacrylate, NOF Corporation |
| SEM-1300 | Blemmer® PSE-1300 Stearoxy Polyethyleneglycol (30) Methacrylate, NOF Corporation |
| SMA | Stearyl Methacrylate |
| STYSEM-25 | Sipomer®, ω-Tristyrylphenyl Polyoxyethylene (25) Methacrylate) |
| Sulfochem™ ALS-K | Ammonium Lauryl Sulfate (anionic surfactant preserved with Kathon® CG preservative from Rohm and Haas Company), Lubrizol Advanced Materials, Inc. (30% active) |
| Sulfochem™ ES-2 | Sodium Laureth Sulfate - 2 moles of ethoxylation (anionic surfactant), Lubrizol Advanced Materials, Inc. (26% active) |
| Sulfochem™ SLS | Sodium Lauryl Sulfate (anionic surfactant), Lubrizol Advanced Materials, Inc. (30% active) |
| Sulfochem™ TLS | TEA-Lauryl Sulfate (anionic surfactant) Lubrizol Advanced Materials, Inc. (40% active) |
| TBHP | tert-butyl t-butyl hydroperoxide |
| VA | Vinyl Acetate |
| VA-10 | Vinyl Decanoate |
| VP | N-Vinylpyrrolidone |
| i-PAMD | iso-Propylacrviamide |
| MAMD | Methacrylamide |

Example 1

An emulsion polymer polymerized from a monomer mixture comprising 50 wt.% EA, 10 wt.% n-BA, 10 wt.%

MMA, 30 wt.% HEMA, and crosslinked with APE (0.08 wt.% based on the weight of the dry polymer) is synthesized as follows.

A monomer premix is made by mixing 140 grams of water, 16.67 grams of Sulfochem™ SLS surfactant (hereafter SLS), 250 grams of EA, 50 grams of n-BA, 50 grams of MMA, 0.57 grams of 70% APE, and 150 grams of HEMA. Initiator A is made by mixing 2.86 grams of 70% TBHP in 40 grams of water. Reductant A is prepared by dissolving 0.13 grams of erythorbic acid in 5 grams of water. Reductant B is prepared by dissolving 2.0 grams of erythorbic acid in 100 grams of water. A 3 liter reactor vessel is charged with 800 grams of water and 1.58 grams of SLS surfactant, and then is heated to 60°C under a nitrogen blanket and proper agitation. Initiator A is then added to the reaction vessel and followed by adding reductant A. After about 1 minute, the monomer premix is metered to the reaction vessel for over a period of 150 minutes. About 3 minutes after the start of monomer premix proportioning, reductant B is metered to the reaction vessel for over a period of 180 minutes. After completion of reductant B feed, the temperature of the reaction vessel is maintained at 60°C for 60 minutes. The reaction vessel is then cooled to 55°C. A solution of 1.79 grams of 70% TBHP and 0.58 grams of SLS in 25 grams of water is added to the reaction vessel. After 5 minutes, a solution of 1.05 grams of erythorbic acid and 0.1 grams of SLS in 25 grams of water is added to the reaction vessel. The reaction vessel is maintained at 55°C. After 30 minutes, a solution of 1.79 grams of 70% TBHP and 0.3 grams of SLS in 25 grams of water is added to the reaction vessel. After 5 minutes, a solution of 1.0 grams of erythorbic acid and 0.17 grams of SLS in 25 grams of water is added to the reaction vessel. The reaction vessel is maintained at 55°C for about 30 minutes. Then, the reaction vessel is cooled to room temperature and its contents are filtered through 100 μm cloth. The pH of the resulting emulsion is adjusted to 5 to 6 with ammonium hydroxide. The polymer emulsion has 30 wt.% polymer solids, a viscosity 15 MPa·s (cps) and a particle size of 209 nm.

Example 2

An emulsion polymer polymerized from a monomer mixture comprising 35 wt.% EA, 20 wt.% n-BA, 45 wt.% HEMA, and crosslinked with APE (0.08 wt.% based on the weight of the dry polymer) is prepared as follows.

A monomer premix is made by mixing 140 grams of water, 5 grams of SLS, 175 grams of EA, 100 grams of n-BA, 0.57 grams of 70% APE, and 225 grams of HEMA. Initiator A is made by mixing 2.86 grams of 70% TBHP in 40 grams of water. Reductant A is prepared by dissolving 0.13 grams of erythorbic acid in 5 grams of water. Reductant B is prepared by dissolving 2.0 grams of erythorbic acid in 100 grams of water. A 3 liter reactor vessel is charged with 800 grams of water, 13.3 grams of SLS, and 25 grams of poly(vinyl alcohol) (having an average molecular weight 13,000-23,000 Daltons and 87-89% hydrolyzed from Sigma-Aldrich Co.). The reactor vessel is heated to 60°C under a nitrogen blanket and proper agitation. Initiator A is then added to the reaction vessel and followed by the addition of reductant A. After about 1 minute, the monomer premix is metered into the reaction vessel over a period of 150 minutes. About 3 minutes after the start of monomer premix metering, reductant B is metered into the reaction vessel over a period of 180 minutes. After completion of reductant B feed, th e temperature of the reaction vessel is maintained at 60°C for 60 minutes. The reaction vessel is then cooled to 55°C. A solution of 1.79 grams of 70% TBHP and 0.58 grams of 30% SLS in 25 grams of water is added to the reaction vessel. After 5 minutes, a solution of 1.05 grams of erythorbic acid and 0.1 grams of SLS in 25 grams of water is added to the reaction vessel. The reaction vessel is maintained at 55°C. After 30 minutes, a solution of 1.79 grams of 70% TBHP and 0.3 grams of SLS in 25 grams of water is added to the reaction vessel. After 5 minutes, a solution of 1.0 grams of erythorbic acid solution and 0.17 grams of SLS in 25 grams of water is added to the reaction vessel. The reaction vessel was maintained at 55°C for about 30 minutes. Then, the reaction vessel is cooled to room temperature and its contents are filtered through 100 μm cloth. The pH of the resulting emulsion is adjusted to between 5 and 6 with ammonium hydroxide. The polymer emulsion has 29.74 wt.% polymer solids, a viscosity of 21 MPa·s (cps) and a particle size of 109 nm.

Example 3

An emulsion polymer polymerized from a monomer mixture comprising 45 wt.% EA, 15 wt.% n-BA, 45 wt.% HEMA, and crosslinked with APE (0.08 wt.% based on the weight of the dry polymer) is prepared by a method similar to Example 2 except that 200 grams of EA and 75 grams of n-BA are used. The polymer emulsion has 29.43 wt.% polymer solids, a viscosity of 26 MPa·s (cps), and a particle size of 101 nm.

Example 4 (comparative)

An emulsion polymer polymerized from a monomer mixture comprising 50 wt.% EA, 20 wt.% MMA, 30 wt.% HEMA, and crosslinked with APE (0.35 wt.% based on the weight of the dry polymer) is prepared as follows.

A monomer premix is made by mixing 140 grams of water, 16.67 grams of SLS, 250 grams of EA, 75 grams of MMA, 1.75 grams of APE, and 150 grams of HEMA. Initiator A is made by mixing 2.86 grams of 70% TBHP in 40

grams of water. Reductant A is prepared by dissolving 0.13 grams of erythorbic acid in 5 grams of water. Reductant B is prepared by dissolving 2.0 grams of erythorbic acid in 100 grams of water. A 3 liter reactor vessel is charged with 800 grams of water and 1.58 grams of SLS, and then is heated to 60°C under a nitrogen blanket and proper agitation. Initiator A is then added to the reaction vessel and followed by adding reductant A. After about 1 minute, the monomer premix is metered to the reaction vessel over a period of 144 minutes. About 3 minutes after the start of monomer premix metering, reductant B is proportioned to the reaction vessel over a period of 180 minutes. After completion of monomer premix feed, 25 grams of MMA is metered into the reaction vessel over a period of 6 minutes. After completion of the reductant B feed, the temperature of the reaction vessel is maintained at 60°C for 60 minutes. The reaction vessel is then cooled to 55°C. A solution of 1.79 grams of 70% TBHP and 0.58 grams of SLS in 25 grams of water is added to the reaction vessel. After 5 minutes, a solution of 1.05 grams of erythorbic acid and 0.1 grams of 30% SLS in 25 grams of water is added to the reaction vessel. The reaction vessel is maintained at 55°C. After 30 minutes, a solution of 1.79 grams of 70% TBHP and 0.3 grams of 30% SLS in 25 grams of water is added to the reaction vessel. After 5 minutes, a solution of 1.0 grams of erythorbic acid solution and 0.17 grams of SLS in 25 grams of water is added to the reaction vessel. The reaction vessel is maintained at 55°C for about 30 minutes. Then, the reaction vessel is cooled to room temperature and filtered through 100 μm cloth. The pH of the resulting emulsion is adjusted to between 5 and 6 with ammonium hydroxide. The polymer emulsion has 28.65 wt.% polymer solids, a viscosity 6 MPa·s (cps) and a particle size of 94 nm. This polymer contains a relatively high level of a crosslinker (APE).

Example 5 (comparative)

An emulsion polymer polymerized from a monomer mixture comprising 50 wt.% EA, 20 wt.% MMA, 30 wt.% HEMA, and crosslinked with APE (0.53 wt.% based on the weight of the dry polymer) is prepared by a method similar to Example 4 except that 2.65 grams of APE is used. The polymer emulsion has 26.31 wt.% polymer solids, a viscosity of 5 MPa·s (cps), and a particle size of 94 nm. This polymer contains a relatively high level of cross-linker (APE).

Example 6

An emulsion polymer polymerized from a monomer mixture comprising 35 wt.% EA, 20 wt.% n-BA, 45 wt.% HEMA, and no crosslinker is prepared by a method similar to Example 2 except that no APE is used. The polymer emulsion has 29.55 wt.% polymer solids, a viscosity of 26 MPa·s (cps), and a particle size of 93 nm.

Example 7 (comparative)

An emulsion polymer polymerized from a monomer mixture comprising 70 wt.% EA, 20 wt.% n-BA, 10 wt.% HEMA, and crosslinked with APE (0.08 wt.% based on the weight of the dry polymer) is synthesized by a method similar to Example 2. The polymer emulsion has 29.73 wt.% polymer solids, a viscosity of 26 MPa·s (cps), and a particle size of 93 nm.

Example 8

An emulsion polymer polymerized from a monomer mixture comprising 40 wt.% EA, 15 wt.% n-BA, 10 wt.% HEA, 35 wt.% HEMA, and crosslinked with APE (0.06 wt.% based on the weight of the dry polymer) is prepared as follows.

A monomer premix is made by mixing 140 grams of water, 5 grams of SLS, 200 grams of EA, 75 grams of n-BA, 50 grams of 2-hydroxyl ethyl acrylate (HEA), and 175 grams of HEMA. Initiator A is made by mixing 2.86 grams of 70% TBHP in 40 grams of water. Reductant A is prepared by dissolving 0.13 grams of erythorbic acid in 5 grams of water. Reductant B is prepared by dissolving 2.0 grams of erythorbic acid in 100 grams of water. A 3 liter reactor vessel is charged with 800 grams of water, 13.3 grams of 30% SLS, and 25 grams of poly(vinyl alcohol) (having an average molecular weight 13,000-23,000 Daltons and 87-89% hydrolyzed). The reactor vessel is heated to 60° under a nitrogen blanket and proper agitation. Initiator A is then added to the reaction vessel and followed by the addition of reductant A. After about 1 minute, the monomer premix is metered to the reaction vessel over a period of 150 minutes. About 3 minutes after the start of monomer premix metering, reductant B is metered to the reaction vessel over a period of 180 minutes. About 60 minutes after the start of monomer premix metering, 0.43 grams of 70% APE is added to the monomer premix. After completion of reductant B feed, the temperature of the reaction vessel is maintained at 60°C for 60 minutes. The reaction vessel is then cooled to 55°C. A solution of 1.79 grams of 70% TBHP and 0.58 grams of SLS in 25 grams of water is added to the reaction vessel. After 5 minutes, a solution of 1.05 grams of erythorbic acid and 0.1 grams of SLS in 25 grams of water is added to the reaction vessel. The reaction vessel is maintained at 55°C. After 30 minutes, a solution of 1.79 grams of 70% TBHP and 0.3 grams of SLS in 25 grams of water is added to the reaction vessel. After 5 minutes, a solution of 1.0 grams of erythorbic acid solution and 0.17 grams of SLS in 25 grams of water is added to

the reaction vessel. The reaction vessel is maintained at 55°C for about 30 minutes. Then, the reaction vessel is cooled to room temperature and the contents are filtered through 100-μm cloth. The pH of the resulting emulsion is adjusted to between 5 and 6 with ammonium hydroxide. The polymer emulsion had 30.44% polymer solids, a viscosity of 17 MPa·s (cps), and a particle size of 99 nm.

Example 9

An emulsion polymer polymerized from a monomer mixture comprising 20 wt.% EA, 15 wt.% n-BA, 20 wt.% VA, 45 wt.% HEMA, and crosslinked with APE (0.06 wt.% based on the weight of the dry polymer) is synthesized in a manner similar to that of Example 8. The monomer mixture contains 20 grams of VA, 20 grams of EA, 75 grams of n-BA, and 225 grams of HEMA. The poly(vinyl alcohol) in the reactor is switched to one with an average molecular weight about 9,000-1,0000 Daltons and 80% hydrolyzed. The polymer emulsion has 30.1 wt.% polymer solids, a viscosity of 14 MPa·s (cps), and a particle size of 135 nm.

Example 10

An emulsion polymer polymerized from a monomer mixture comprising 20 wt.% EA, 15 wt.% n-BA, 20 wt.% VA, 45 wt.% HEMA, and crosslinked with APE (0.06 wt.% based on the weight of the dry polymer) is synthesized in a manner similar to that of Example 9 except APE is added into the monomer premix at about 90 minutes after the start of monomer premix metering. The resulting polymer emulsion has 29.94 wt.% polymer solids, and a viscosity of 16 MPa·s (cps), and a particle size of 130 nm.

Examples 11 to 17

The swelling of individual polymer particles in the emulsions of Examples 1 to 7 by the anionic surfactant, sodium dodecyl sulfate (SDS), is determined by preparing test samples containing 0.01 wt.% of the polymer (total polymer solids), 20 mM sodium chloride at surfactant concentrations ranging from 0 to 6 mM in water. In cases where there is swelling, the particle size, measured by dynamic light scattering (DLS), remained constant up to a critical surfactant concentration but increased monotonically above this concentration to a plateau value at the highest surfactant concentrations. Referring to Figure 1 a swelling or expansion ratio is obtained for the polymer of Example 12 by dividing the plateau value (250 nm) by the size of the particle below the critical concentration threshold (93.5 nm) (polymer expansion ratio: 250 nm/93.5 nm = 2.7).

Samples containing 3 wt.% polymer solids and 5 wt.% SLS in water are prepared using each of the polymers prepared in Examples 1 to 7. The yield stress, viscosity and shear thinning index of these samples were determined by oscillatory and steady shear measurements on a controlled stress rheometer (TA Instruments AR1000N rheometer, New Castle, DE) with cone and plate geometry (40mm cone with a cone angle of 2 degrees and 56μm gap) at 25°C. The oscillatory measurements are performed at a fixed frequency ranging from 1 Hz to 0.001 Hz. The elastic and viscous moduli (G' and G" respectively) are obtained as a function of increasing stress amplitude. In cases where the swollen polymer particles created a jammed network, G' is larger than G" at low stress amplitudes but decreases at higher amplitudes crossing G" because of rupture of the network. The stress corresponding to the crossover of G' and G" is noted as the yield stress. Figure 2 illustrates the G' (solid fill) and G" (no fill) crossover point (yield stress value) for the yield stress fluid of Example 13. A plot of viscosity versus shear rate is obtained from the steady shear measurements. The viscosity at a shear rate of $3s^{-1}$ is noted. The shear thinning index is obtained from a power law fit ($\eta = K\gamma^{n-1}$) in the shear rate range 0.1 to $1s^{-1}$ where $\eta$ is viscosity, y is shear rate, n is the shear thinning index and K is a constant. The optical clarity (expressed as percent transmittance or %T) of the samples is measured using a Brinkmann PC 910 colorimeter with a 420nm filter. The results of these measurements are shown in Table 1, along with the polymer expansion ratio.

TABLE 1

| Example No. | Polymer No. | Yield Stress (Pa) | Viscosity (Pa·s) | Shear Thinning Index | % T | Polymer Expansion Ratio | Suspension Stability (wks.) |
|---|---|---|---|---|---|---|---|
| 11 | 1 | 2.7 | 1.1 | 0.26 | 28.5 | 2.9 | 16+ |
| 12 | 2 | 3.0 | 1.2 | 0.29 | 41.5 | 2.7 | 16+ |
| 13 | 3 | 1.6 | 1.0 | 0.3 | 52 | 3.0 | 16+ |
| 14 (comparative) | 4 | NONE | 0.024 | 1.0 | 4.5 | 2.2 | Fail |
| 15 (comparative) | 5 | NONE | 0.023 | 1.0 | 4.4 | 2.2 | Fail |
| 16 | 6 | NONE | 0.06 | 1.0 | 93 | – | Fail |
| 17 (comparative) | 7 | NONE | 0.002 | 1.0 | 4.9 | 1.2 | Fail |

It is clear that the compositions of Examples 11 to 13 (prepared with crosslinked amphiphilic polymers having expansion ratios greater than 2.5) have a high yield stress (greater than 0.5 Pa), excellent shear thinning and good optical clarity. The comparative formulations of Examples 14 and 15 are formulated with polymers having a relatively high a level of crosslinker and they are not able to swell adequately in the surfactant medium. These compositions do not display a yield stress or shear thinning and have extremely low viscosities and optical clarity.

Example 16 is formulated with a polymer that contains no crosslinking (linear). In this case there is high optical clarity but no yield stress or shear thinning attributes. The linear polymers of the invention can be utilized to mitigate irritation in composition where an increase in yield stress is not desired.

Comparative Example 17 is formulated with a polymer having the right level of crosslinker but too low a level of hydrophilic monomer. This polymer also does not exhibit adequate swelling in the surfactant medium and displays no yield stress or shear thinning attributes coupled with poor optical clarity and low viscosities.

The ability of a polymer system to suspend active and/or aesthetically pleasing insoluble oily, gaseous and particulate materials is important from the standpoint of product efficacy and appeal. The long-term suspension of 1.2 mm sized beads with specific gravity of approximately 1.4 (Unisphere™ REL 552 from Induchem AG, Switzerland) is examined in Examples 12 to 17. A six dram vial (approximately 70 mm high x 25 mm in diameter) is filled to the 50 mm point with each formulation. The beads are weighed into each sample (0.6 wt.% based on the weight of the total formulation) and stirred gently with a wooden spatula until they are uniformly dispersed throughout each sample. The vials are placed on a lab bench at ambient room temperature to age for a 16 week period. The bead suspension property of each sample is monitored on a daily basis. The suspension results are visually observed over the 16 week test period. The beads remain suspended (do not rise or settle) in the formulations of the invention. The formulations of Comparative Examples 14 to 17 fail in that the beads settle to the bottom of the vials in 2 weeks.

Example 18

This example illustrates the effect of alternative anionic surfactants containing different salts on the rheology and optical clarity of yield stress fluids. Aqueous compositions containing 3 wt.% (total polymer solids) of the polymer from Example 2 and 5 wt.% surfactant (active material) listed in the table below are prepared and the yield stress, viscosity, shear thinning index and optical clarity are measured as in Examples 11 to 17. The results are shown in Table 2.

TABLE 2

| Salt | Surfactant | Yield Stress (Pa) | Viscosity (Pa·s) | Shear Thinning Index | %T |
|---|---|---|---|---|---|
| Triethylammonium | Sulfochem™ TLS | 3.3 | 1.5 | 0.18 | 10 |
| Ammonium | Sulfochem™ ALS-K | 5.0 | 2.2 | 0.15 | 18 |

It is clear that yield stress fluids displaying high yield stresses, excellent shear thinning and acceptable optical clarity are obtained with various anionic surfactants.

Example 19

This example illustrates a combination of anionic ethoxylated surfactant and amphoteric surfactant on the rheology and optical clarity of yield stress fluids containing the polymers of the invention. Aqueous compositions containing 3 wt.% polymer solids and 14 wt.% of a surfactant blend (12 wt.% (active) anionic surfactant, Sulfochem™ ES-2 and 2 wt.% (active) amphoteric surfactant, Chembetaine™ CAD, are prepared by mixing the polymer and the surfactant combination. The yield stress, viscosity, shear thinning index and optical clarity are measured as in Examples 11 to 17. The results are shown in Table 3.

TABLE 3

| Polymer No. | Yield Stress (Pa) | Viscosity (Pa·s) | Shear Thinning Index | %T |
|---|---|---|---|---|
| Ex. 8 | 4.1 | 2.2 | 0.33 | 59 |
| Ex. 9 | 6.8 | 2.3 | 0.24 | 32 |
| Ex. 10 | 3.8 | 1.5 | 0.32 | 74 |

Yield stress fluids displaying high yield stresses, excellent shear thinning and acceptable optical clarity are obtained by using polymers of the invention in combination with a mixture of anionic and amphoteric surfactant.

Figure 3 is a plot showing oscillatory rheology measurements on the yield stress fluid formulated above from the polymer of Example 9. The vertical line drawn through the crossover point of G' (no fill) and G" (solid fill) on the plot indicates the boundary between a jammed network of micro-gels at low stresses and a fluid above a threshold (yield) stress. The plot of G" versus stress displays a maximum that is characteristic of a soft glassy material (SGM).

Example 20

The long-term suspension of 1.2 mm sized beads with a specific gravity of approximately 1.4 (Unisphere™ REL 552 from Induchem AG, Switzerland) is examined for the yield stress fluids exemplified in Table 4 (which include the polymers of Examples 8, 9, and 10) according to the method of Examples 11 to 17. The beads remain suspended in the yield stress fluid formulations set forth in this example for over 4 months at room temperature (approximately 23°C).

Example 21 (comparative)

This example illustrates the behavior of nonionic hydrophobically modified associative thickeners in combination with an anionic surfactant in water.

A hydrophobic ethoxylated urethane (HEUR) polymer (Aculyn® 44 from Dow Chemical) and a hydrophobically modified hydroxyethylcellulose (HMHEC) polymer (Natrosol® Plus 330 PA from Ashland Chemical) are combined with SDS surfactant to prepare compositions containing 3 wt.% polymer (total polymer solids) and 5 wt.% surfactant (active material) in water. The rheology of the compositions is determined using the procedure described in Example 1. In both cases, it is found that the samples did not exhibit a yield stress value.

Example 22

This example compares the effect of pH on the yield stress of fluid compositions containing a mixture of surfactant and polymer of the invention versus compositions containing a pH responsive polymer formulated in the same surfactant system. The comparative polymer is Acrylates Crosspolymer-4 (INCI) (marketed as Carbopol® Aqua SF-2, Lubrizol Advanced Materials, Inc.), a crosslinked, anionic acrylic emulsion polymer of (meth)acrylic acid or one or more of their $C_1$ to $C_4$ alkyl esters.

Several examples containing 2.5 wt.% (total polymer solids) of the polymer of Example 10 and 14 wt.% of a surfactant blend (12 wt.% (active material) anionic ethoxylated surfactant, Sulfochem™ ES-2, and 2 wt.% (active material) amphoteric surfactant Chembetaine™ CAD) and 10 mM sodium chloride in water are prepared. Identical samples are formulated with the comparative Acrylates Crosspolymer-4. The pH of these samples is adjusted to values ranging from 3 to 12 using dilute aqueous solutions of sodium hydroxide (18% wt./wt.) or citric acid (50% wt./wt.). The yield stress at a frequency of 1 Hz is measured using the methods of Examples 11 to 17. The results for the compositions formulated with the polymer of Example 10 are shown in Table 4, and the results for compositions formulated with the pH responsive comparative polymer are shown in Table 5.

TABLE 4 (Invention)

| pH | Yield Stress (Pa) |
|---|---|
| 4 | 2.96 |
| 4.6 | 2.71 |
| 5.7 | 2.58 |
| 6.7 | 2.45 |
| 7.8 | 2.54 |
| 8.5 | 2.52 |
| 9.5 | 2.52 |
| 10.3 | 2.19 |
| 11.5 | 2.55 |

The yield stress values listed in Table 4 have a mean value of 2.56 Pa and standard deviation of 0.19 Pa whereas the yield stress values listed in Table 5 have a mean value of 1.58 Pa and a standard deviation of 2.07 Pa. It is clear that the polymer of the invention provides significantly more uniform yield stress over a broad range in pH compared to the control polymer.

TABLE 5 (Comparative)

| pH | Yield Stress (Pa) |
|---|---|
| 3.8 | 4.7 |
| 4.7 | 4.6 |
| 5.3 | 3.3 |
| 7.2 | 0 |
| 8.5 | 0 |
| 9.4 | 0 |
| 10.7 | 0 |
| 11.1 | 0 |

The long term suspension of 1.4mm sized beads with a specific gravity of approximately 1.3 (Unisphere™ REL 551 from Induchem AG, Switzerland) is examined according to the method of Examples 11 to 17. It is found that the beads remain suspended in all samples exemplified in Table 4 for over 4 months at room temperature (approximately 23°C) but the beads failed to remain suspended in the last five samples listed in Table 5.

Example 23

This example illustrates the effect of compositions of the invention on alignment of mica and pearlescence.

Samples containing 3 wt.% polymer and 5 wt.% of sodium dodecyl sulfate (SDS) in water are prepared using the polymers of Example 1 and Example 2. Mica platelets coated with iron oxide (Colorona Copper Cosmetic Pigment, product # 017378 from EM Industries, Inc.) are added to these samples at a concentration of 0.7 mg per ml. A drop of the sample containing mica is placed on a microscope slide, covered with a cover slip and allowed to equilibrate for 5 minutes. The slide is then placed on the stage of a microscope (Olympus BX51TRF) equipped with a polarizer, analyzer and a color camera. After focusing in bright field, the polarizer and analyzer are crossed and an image is captured with the color camera. The image is then decomposed into its three component color channels: red, green and blue. Using image analysis software (Image J software, National Institutes of Health), the total number of platelets darker than the background in the blue channel and the total number of platelets brighter than the background in the red channel are counted. Platelets that are not aligned under shear appear bright in the red channel when viewed with crossed polarizers. The fraction of platelets not aligned under shear is calculated as the total number of platelets counted in the red channel

divided by the total number of platelets counted in the blue channel. The fraction of aligned platelets is calculated as 1 minus the fraction of platelets not aligned. Samples containing polymers of Example 1 and Example 2 show 88.8% and 87.4% alignment of mica platelets with standard deviations of 5.2 and 5.3, respectively. Alignment greater than 80% provides the extremely pleasing visual appearance of pearlescence.

Example 24

An emulsion polymer is polymerized from a monomer mixture comprising 45wt% HEMA, 35wt% EA, 15wt% n-BA, 5wt% BEM, and crosslinked with APE (0.08wt% based on the weight of the dry polymer) is prepared as follows.

A monomer premix is made by mixing 140 grams of water, 3.75 grams of 40% alpha olefin sulfonate (AOS) aqueous solution, 175 grams of EA, 71 grams of n-BA, 33.33 grams of BEM and 225 grams of HEMA. Initiator A was made by mixing 2.86 grams of 70% TBHP in 40 grams of water. Reductant A is prepared by dissolving 0.13 grams of erythorbic acid in 5 grams of water. Reductant B is prepared by dissolving 2.0 grams of erythorbic acid in 100 grams of water. A 3-liter reactor vessel is charged with 800 grams of water, 10 grams of 40% AOS and 25 grams of Celvol® 502 PVA and then is heated to 65°C under a nitrogen blanket and proper agitation. Initiator A is then added to the reaction vessel and followed by adding reductant A. After about 1 minute, the monomer premix is metered into the reaction vessel over a period of 150 minutes; simultaneously, reductant B is metered into the reaction vessel over a period of 180 minutes. After the addition of monomer premix, a solution of 0.40 grams of 70% APE and 3.6 grams n-BA is added into the monomer premixer. After the completion of monomer premix feed, 33 grams of water is added to flush the residual monomers from the premixer. After the completion of reductant B feed, the temperature of the reaction vessel is maintained at 65°C for 65 minutes. The reaction vessel is then cooled to 60°C. A solution of 1.79 grams of 70% TBHP and 0.13 grams of 40% AOS in 25 grams of water is added to the reaction vessel. After 5 minutes, a solution of 1.05 grams of erythorbic acid in 25 grams of water is added to the reaction vessel. After 30 minutes, a solution of 1.79 grams of 70% TBHP and 0.13 grams of 40% AOS in 25 grams of water is added to the reaction vessel. After 5 minutes, a solution of 1.05 grams of erythorbic acid in 25 grams of water is added to the reaction vessel. The reaction vessel is maintained at 60°C for about 30 minutes. Then, the contents of the reaction vessel is cooled to room temperature and filtered through 100 μm cloth. The pH of the resulting emulsion is adjusted to 3.5-4.5 with 28% ammonium hydroxide.

Example 25

An emulsion polymer polymerized from a monomer mixture comprising 45% HEMA 35wt% EA, 15wt% n-BA, 5wt% MPEG 350, and crosslinked with APE (0.08% based on the weight of the dry polymer) is prepared as follows.

A monomer premix is made by mixing 140 grams of water, 5 grams of 30% sodium lauryl sulfate (SLS) aqueous solution, 175 grams of EA, 71 grams of n-BA, 25 grams of Bisomer® MPEG 350 MA, and 225 grams of HEMA. Initiator A is made by mixing 2.86 grams of 70% TBHP in 40 grams of water. Reductant A is prepared by dissolving 0.13 grams of erythorbic acid in 5 grams of water. Reductant B is prepared by dissolving 2.0 grams of erythorbic acid in 100 grams of water. A 3-liter reactor vessel is charged with 800 grams of water, 13.33 grams of 30% SLS and 25 grams of Celvol® 502 PVA, and the contents are heated to 65°C under a nitrogen blanket and proper agitation. Initiator A is added to the reaction vessel and followed by adding reductant A. After about 1 minute, the monomer premix is metered into the reaction vessel over a period of 150 minutes; simultaneously, reductant B is metered into the reaction vessel over a period of 180 minutes. After the addition of monomer premix, a solution of 0.40 grams of 70% APE and 3.6 grams n-BA is added into the monomer premixer. After the completion of monomer premix feed, 33 grams of water is added to flush the residual monomers in the premixer. After the completion of reductant B feed, the temperature of the reaction vessel is maintained at 65°C for 65 minutes. The reaction vessel is then cooled to 60°C. A solution of 1.79 grams of 70% TBHP and 0.17 grams of 30% SLS in 25 grams of water is added to the reaction vessel. After 5 minutes, a solution of 1.05 grams of erythorbic acid in 25 grams of water is added to the reaction vessel. After 30 minutes, a solution of 1.79 grams of 70% TBHP and 0.17 grams of 30% SLS in 25 grams of water is added to the reaction vessel. After 5 minutes, a solution of 1.05 grams of erythorbic acid in 25 grams of water is added to the reaction vessel. The reaction vessel is maintained at 60°C for about 30 minutes. Then, the reaction vessel is cooled to room temperature and filtered through 100 μm cloth. The pH of the resulting emulsion is adjusted to 3.5-4.5 with 28% ammonium hydroxide. The resulting polymer latex had a solids level of 30%, a viscosity of 16 MPa·s (cps), and particle size of 125 nm.

Example 26

Samples containing 2.5% (total polymer solids) of the polymer of Example 33 and 17 wt.% of a surfactant blend (14 wt.% (active material) anionic surfactant Sulfochem™ ES-2, and 3 wt.% (active material) amphoteric surfactant Chembetaine™ CAD) and 0.1 wt.% sodium chloride in water are prepared. The pH of these samples is adjusted to values ranging from 3 to 12 using dilute aqueous solutions of sodium hydroxide (18% wt./wt.) or citric acid (50% wt./wt.).

Yield stress and optical clarity for each sample is measured and recorded in Table 12. The yield stress at a frequency of 1 Hz is measured on a controlled stress rheometer (TA instruments AR2000EX rheometer, New Castle, DE) with cone and plate geometry (60mm cone with a cone angle of 2 degrees and 56 μm gap) at 25°C using the method described in Examples 15 to 21. The optical clarity (expressed as percent transmittance or %T) of each sample is measured using a Brinkmann PC 910 colorimeter with a 420 nm filter. The results are shown in Table 6.

Table 6

| pH | Yield Stress (Pa) | Optical Clarity (%T) |
|---|---|---|
| 3.9 | 7.4 | 72.1 |
| 4.9 | 7 | 75.5 |
| 5.8 | 6.7 | 76.1 |
| 6.4 | 6.7 | 77.9 |
| 7.2 | 6.5 | 78.4 |
| 8.7 | 5.7 | 77.1 |
| 9.6 | 5.5 | 78.5 |
| 10.3 | 5.7 | 78.7 |
| 11.4 | 5.6 | 77.9 |

The yield stress values have a mean value of 6.3 with a standard deviation of 0.7. The ratio of the standard deviation to the mean is 0.11 in the pH range 3 to 12. The optical clarity values in have a mean value of 76.9 and a standard deviation of 2.1. The ratio of the standard deviation to the mean is 0.03 in the pH range 3 to 12.

Example 27

Samples containing 2.5% (total polymer solids) of the polymer of Example 34 are prepared and evaluated for yield stress and optical clarity properties as described in Example 35. The results are given in Table 7.

Table 7

| pH | Yield Stress (Pa) | Optical Clarity (%T) |
|---|---|---|
| 3.7 | 10.1 | 42.1 |
| 4.4 | 8.9 | 38.4 |
| 5.9 | 9.6 | 37.9 |
| 6.3 | 7.4 | 35.4 |
| 7.1 | 8.3 | 37.2 |
| 8.6 | 8.4 | 37.3 |
| 9.7 | 8.5 | 35.3 |
| 10.2 | 8.6 | 36.9 |
| 11.7 | 9.4 | 36.5 |

The yield stress values have a mean value of 8.8 with a standard deviation of 0.8. The ratio of the standard deviation to the mean is 0.09 in the pH range 3 to 12. The optical clarity values have a mean value of 37.4 and a standard deviation of 2.0. The ratio of the standard deviation to the mean is 0.05 in the pH range 3 to 12.

Examples 28 to 45

Emulsion polymers of the invention are prepared from the monomer components and amounts (wt.% based on the total monomer weight) set forth in Table 14 in accordance with the procedures and conditions of Example 24. A crosslinking monomer (APE) is used at 0.1 wt.% (based on the total weight of the dry polymer) in all examples.

Table 8

| Ex. No. | HEMA | EA | n-BA | BEM | AMPS® Monomer | AA | MAA | AMD | MAMD | STYEM | CSEM | BDGMA | MPEG S10 W | MPEG 350 |
|---|---|---|---|---|---|---|---|---|---|---|---|---|---|---|
| 28 | 45 | 35 | 15 | 5 | | | | | | | | | | |
| 29 | 30 | 50 | 15 | 5 | | | | | | | | | | |
| 30 | 45 | 30 | 15 | 10 | | | | | | | | | | |
| 31 | 50 | 30 | 15 | 5 | | | | | | | | | | |
| 32 | 45 | 38 | 15 | 2 | | | | | | | | | | |
| 33 | 43 | 35 | 15 | 5 | 2 | | | | | | | | | |
| 34 | 43 | 35 | 15 | 5 | | 2 | | | | | | | | |
| 35 | 43 | 35 | 15 | 5 | | | 2 | | | | | | | |
| 36 | 43 | 35 | 15 | 5 | | | | 2 | | | | | | |
| 37 | 43 | 35 | 15 | 5 | | | | | 2 | | | | | |
| 38 | 45 | 35 | 15 | | | | | | | 5 | | | | |
| 39 | 45 | 35 | 15 | 1 | | | | | | 4 | | | | |
| 40 | 45 | 30 | 20 | | | | | | | | 5 | | | |
| 41 | 45 | 35 | 15 | | | | | | | | | 5 | | |
| 42 | 45 | 35 | 15 | | | | | | | | | | 5 | |
| 43 | 35 | 35 | 20 | 2 | | | | | | | | | 8 | |
| 44 | 37 | 35 | 20 | 3 | | | | | | | | | | 5 |
| 45 | 35 | 35 | 15 | 5 | | | | | | | | | | 10 |

Examples 46 to 55

Emulsion polymers of the invention are prepared from the monomer components and amounts (wt.% based on the total monomer weight) set forth in Table 9 in accordance with the procedures and conditions of Example 24. A crosslinking monomer (APE) is used at 0.9 wt.% (based on the total weight of the dry polymer) in all examples.

Table 9

| Ex. No. | HEMA | EA | n-BA | BEM | MA EO/PO-300 | MA EO/PO-800 | MPEG PME-400 | NPEA-1300 | OEO/POMA | LEM | SEM-400 | SEM-1300 | PEO/POMA | PEA |
|---|---|---|---|---|---|---|---|---|---|---|---|---|---|---|
| 46 | 45 | 35 | 15 | | 5 | | | | | | | | | |
| 47 | 45 | 35 | 15 | | | 5 | | | | | | | | |
| 48 | 42 | 35 | 15 | 3 | | | 5 | | | | | | | |
| 49 | 45 | 35 | 15 | | | | | 5 | | | | | | |
| 50 | 44 | 35 | 15 | 1 | | | | | 5 | | | | | |
| 51 | 45 | 35 | 15 | | | | | | | 5 | | | | |
| 52 | 45 | 35 | 15 | | | | | | | | 5 | | | |
| 53 | 45 | 35 | 15 | | | | | | | | | 5 | | |
| 54 | 45 | 35 | 15 | | | | | | | | | | 5 | |
| 55 | 45 | 35 | 15 | | | | | | | | | | | 5 |

Example 56

An emulsion polymer polymerized from a monomer mixture comprising 15 wt.% EA, 20 wt.% n-BA, 20 wt.% VA, 45 wt.% HEMA, and crosslinked with APE (0.086 wt.% based on the weight of the dry polymer) is prepared as follows.

A monomer mixture is prepared by mixing 140 grams of water, 5 grams of Sulfochem™ SLS surfactant (30% active), 75 grams of EA, 100 grams of n-BA, 100 grams of VA, 0.43 grams of APE, and 225 grams of HEMA. Initiator A is made by mixing 1.79 grams of 70% TBHP and 40 grams of water. Reductant A is prepared by dissolving 0.15 grams of erythorbic acid in 5 grams of water. Reductant B is prepared by dissolving 1.25 grams of erythorbic acid in 100 grams of water. A 3 liter reactor vessel is charged with 800 grams of water, 13.33 grams of SLS surfactant (30% active), 25 grams of PVOH, and then is heated to 60°C under a nitrogen blanket and proper agitation. Initiator A is then added to the reaction vessel followed by adding reductant A. Immediately after, reductant B is metered into the reaction vessel over a period of 180 minutes and the monomer mixture is metered into the reaction vessel over a period of 150 minutes. After completion of metering reductant B, the temperature of the reaction vessel is maintained at 60°C for 60 minutes. The reaction vessel is then cooled to 55°C. A solution of 0.86 grams of 70% TBHP, 0.17 grams of 30% SLS surfactant, and 25 grams of water is added to the reaction vessel. After 5 minutes, 0.5 grams of erythorbic acid dissolved in 25 grams of water is added to the reaction vessel. The reaction vessel is maintained at 55°C. After 30 minutes, a solution of 0.86 grams of 70% TBHP, 0.17 grams of 30% SLS, and 25 grams of water is added to the reaction vessel. After 5 minutes, 0.5 grams of erythorbic acid dissolved in 25 grams of water is added to the reaction vessel. The reaction vessel is maintained at 55°C for 30 minutes. Then, the contents of the reaction vessel are cooled to room temperature and filtered through 100 μm cloth. The pH of the resulting emulsion (approximately 3) is adjusted to between 5 and 5.5 with ammonium hydroxide (28%).

Example 57

Mitigation of skin irritancy induced by sodium dodecyl sulfate (SDS) surfactant dosed with the polymer of Example 56 is assessed in the Epiderm™ Human Tissue Model (EPI-200) bioassay conducted by MatTek Corporation, Ashland, MA. The MatTek bioassay utilizes human derived epidermal keratinocytes (NHEK) which have been cultured to form a multilayered, highly differentiated model of the human epidermis. The model contains uniform and highly reproducible functional skin tissue layers (basal, spinous, granular, and comified layers) corresponding to those found *in vivo,* and exhibits *in vivo-like* morphological and growth characteristics and is mitotically and metabolically active.

The Epiderm™ skin cultures treated with the SDS/polymer test formulation are evaluated by a MTT (3-[4,5-dimethylthiazol-2-yl]-2,5-diphenyltetrazolium bromide) tissue viability assay (MTT Effective Time-50 (ET-50) Protocol, MatTek Corporation) which assesses the potential irritancy mitigation (over a pre-determined exposure period) of test materials by assaying for MTT reduction in tissue cultures treated with SDS surfactant dosed with the test polymer relative to neat SDS surfactant control samples containing no polymer. The MTT tissue viability assay measures the NAD(P)H dependent microsomal enzyme reduction of MTT (and to a lesser extent, the succinate dehydrogenase reduction of MTT) after exposure to a treated skin culture test sample for various exposure times. The duration of exposure resulting in a 50% decrease in MTT conversion in test formulation treated EpiDerm™ cultures, relative to control cultures, is determined (ET-50).

A SDS surfactant/polymer test formulation is formulated from the components set forth in Table 10 and evaluated for irritancy potential using the MTT viability assay. Test samples containing neat SDS surfactant (no polymer) are formulated for comparative purposes.

Table 10

| Test Formulation | Polymer (wt.% active) | Surfactant (wt.% active) | D.I. Water |
|---|---|---|---|
| Example 57 | 2.5 | 3.2 | q.s. to 100 |
| Blank Surfactant | -- | 3.2 | q.s. to 100 |

Skin tissue culture inserts (Cat. No.EPI-200) are pre-incubated in the wells of 6-well plates containing 0.9 ml of MatTek assay medium (Cat. No. EPI-100-ASY) for 1 hr. at 37°C in a humidified atmosphere of 5% $CO_2$ in air. The 6-well plates containing the tissue culture inserts are removed from the incubator and the assay media is aspirated from the wells and replaced by an additional aliquot of 0.9 ml of pre-warmed (37°C) assay media. For each exposure time wells are then dosed (2 repetitions) with 100 μl of the test formulations set forth in Table 10 (3 tissue culture wells are dosed with 100 μl ultra-pure water as a negative control for each of the test candidates). In addition, 100 μl of a positive control formulation (1% Triton X-100 in ultrapure water) is dosed into tissue culture wells (3 repetitions for each exposure time), as well as 3 repetitions of ultrapure water as a negative control. The exposure times of the test formulations, negative control and positive control are set forth below.

MTT assay plates are prepped by preparing a 24-well plate by adding 300 μl of MTT reagent solution to an appropriate number of wells to accommodate the test. The MTT reagent is utilized at a concentration of 1 mg/ml of MTT diluted in Dulbecco's Modified Eagle Medium (DMEM). The MTT/DMEM solution is centrifuged and decanted to remove any precipitate before use.

After exposure times of 60 (1 hr.), 120 (2 hrs.) and 210 (3.5 hrs.) minutes to the test solutions, the tissue cultures are removed from the incubator and thoroughly rinsed (2 times) with Dulbecco's phosphate buffered saline (DPBS) to remove the test material. The negative control is exposed for 4 hours and the positive control is exposed for time periods of 4, 6, 8, and 10 hours. Any remaining rinse media is decanted from the top of the tissue cultures. Each tissue culture insert is transferred to an appropriately labeled well of the 24-well plate containing the MTT reagent solution and placed back in the incubator. After 30 minutes reaction with the MTT reagent, the tissue cultures are removed from the incubator. Each tissue culture insert is removed from its well and the bottom is gently blotted with a lab tissue. The inserts are immersed into a labeled 24-well extraction plate containing 2 ml of isopropanol extractant solution. The 24-well extraction plate is covered with aluminum foil (to protect the samples from light) and placed into a sealable plastic bag to minimize evaporation of the extractant solution. The sealed 24-well extraction plate is set on an orbital shaker and gently shaken for 2 hours at ambient room temperature (approximately 20°C).

Upon the conclusion of the extraction period, the extractant liquid within each tissue culture insert is decanted back into the well from which it was taken and thoroughly mixed with the extractant solution contained in the well. 200 μl of the mixed extractant solution in each well is transferred into a 96-well microtiter plate for spectrophotometric analysis. A 200 μl sample of neat extractant solution (isopropanol) is utilized as a blank. The absorbance (optical density) at 570 nm ($OD_{570}$) of the extracted samples in each well is measured with a spectrophotometer (EMax® Microplate Reader, Molecular Devices, LLC, Sunnyvale, CA) equipped with a 96-well plate reader and no reference filter. Background noise in all samples is subtracted to improve the quality of the acquired data. The mean $OD_{570}$ values of the polymer test wells, blank surfactant test wells, negative control wells and positive control for each exposure time are calculated. The percent viability of each sample is calculated utilizing the formula:

$$\%\ \text{viability} = \text{(ave.) } OD_{570} \text{ of Test Sample/(ave.) } OD_{570} \text{ of Negative Control Sample X 100}$$

Viabilities are determined using the MTT assay and the exposure time which reduces tissue viability to 50% (ET-50). ET-50 values for each tested formulation are determined mathematically using a spreadsheet which interpolates between exposure times that brackets 50% viability. The results for each formulation are presented in the spreadsheets set forth in the tables below.

Table 11 (Polymer + Surfactant)

| Exposure Time (hr) | Test Well | OD (570 nm) | Ave. (OD) | Viability (%) |
|---|---|---|---|---|
| 1 | Rep 1 | 1.361 | 1.403 | 137.8[1] |
| | Rep 2 | 1.445 | | |
| 2 | Rep 1 | 0.53 | 0.670 | 65.8 |
| | Rep 2 | 0.81 | | |
| 3.5 | Rep 1 | 0.205 | 0.209 | 20.5 |
| | Rep 2 | 0.212 | | |
| $H_2O$ | Rep 1 | 1.017 | 1.018 | 100.0 |
| | Rep 2 | 1.001 | | |
| | Rep 3 | 1.036 | | |
| ET-50 (hr) | 2.43 | | | |
| Temporary hormesis effect | | | | |

Table 12 (Neat Surfactant)

| Exposure Time (hr) | Test Well | OD (570 nm) | Ave. (OD) | Viability (%) |
|---|---|---|---|---|
| 1 | Rep 1 | 0.647 | 0.733 | 72.0 |
| | Rep 2 | 0.818 | | |
| 2 | Rep 1 | 0.322 | 0.285 | 28.0 |
| | Rep 2 | 0.248 | | |
| 3.5 | Rep 1 | 0.179 | 0.200 | 19.6 |
| | Rep 2 | 0.221 | | |
| $H_2O$ | Rep 1 | 1.017 | 1.018 | 100 |
| | Rep 2 | 1.001 | | |
| | Rep 3 | 1.036 | | |
| ET-50 (hr) | 1.41 | | | |

Table 13 (1% Triton X-100)

| Exposure Time (hr) | Test Well | OD (570 nm) | Ave. (OD) | Viability (%) |
|---|---|---|---|---|
| 4 | Rep 1 | 1.206 | 1.250 | 74.3 |
| | Rep 2 | 1.244 | | |
| | Rep 3 | 1.301 | | |
| 6 | Rep 1 | 0.68 | 0.830 | 49.3 |
| | Rep 2 | 0.983 | | |
| | Rep 3 | 0.827 | | |

(continued)

| Exposure Time (hr) | Test Well | OD (570 nm) | Ave. (OD) | Viability (%) |
|---|---|---|---|---|
| 8 | Rep 1 | 0.211 | 0.233 | 13.9 |
| | Rep 2 | 0.266 | | |
| | Rep 3 | 0.223 | | |
| 10 | Rep 1 | 0.21 | 0.218 | 13.0 |
| | Rep 2 | 0.229 | | |
| | Rep 3 | 0.215 | | |
| $H_2O$ | Rep 1 | 1.596 | 1.683 | 100.0 |
| | Rep 2 | 1.697 | | |
| | Rep 3 | 1.756 | | |
| ET-50 (hr) | 5.93 | | | |

Based on longer ET-50 values, anionic surfactant formulations containing the polymers of the invention are less irritating (ET-50 = 2.43 hrs.) than the same concentrations of neat (without polymer) anionic surfactant (ET-50 = 1.41 hrs.). The ET-50 value of 5.93 hrs. for the positive control (1 % Triton X-100) fell within two standard deviations of the historical mean (4.77 to 8.72 hrs.), thereby meeting the acceptance value.

## Claims

1. A crosslinked nonionic amphiphilic emulsion polymer prepared from a free radically polymerizable monomer composition comprising at least 30 wt.% of at least one $C_1$-$C_4$ hydroxyalkyl (meth)acrylate, 15 to 70 wt. of at least one $C_1$-$C_{12}$ alkyl (meth)acrylate, 5 to 40 wt.% of at least one vinyl ester of a $C_1$-$C_{10}$ aliphatic carboxylic acid (based on the weight of the total monomers), and 0.01 to 0.3 wt.% of at least one crosslinker (based on the dry weight of the polymer) for use in mitigating ocular and/or skin irritation of detersive compositions comprising at least one anionic surfactant.

2. A crosslinked nonionic amphiphilic emulsion polymer prepared from a free radically polymerizable monomer composition comprising at least 30 wt.% of at least one $C_1$-$C_4$ hydroxyalkyl (meth)acrylate, 15 to 70 wt. of at least one $C_1$-$C_{12}$ alkyl (meth)acrylate, 1 to 10 wt.% of at least one monomer selected from an associative monomer, a semi-hydrophobic monomer, or mixtures thereof (based on the weight of the total monomers), and 0.01 to 0.3 wt.% of at least one crosslinker (based on the dry weight of the polymer) for use in mitigating ocular and/or skin irritation of detersive compositions comprising at least one anionic surfactant, wherein said associative monomer is represented by formulas VII and/or VIIA:

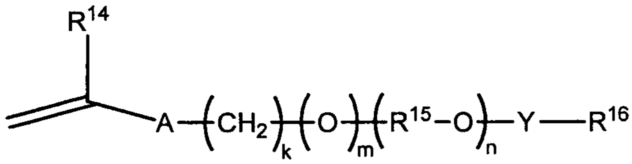

VII

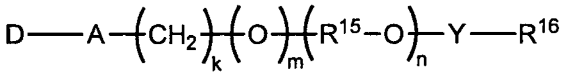

VIIA

wherein $R^{14}$ is hydrogen or methyl; A is $-CH_2C(O)O-$, -C(O)O-, -O-, $-CH_2O-$, -NHC(O)NH-, -C(O)NH-, $-Ar-(CE_2)_z-NHC(O)O-$, $-Ar-(CE_2)_z-NHC(O)NH-$, or $-CH_2CH_2NHC(O)-$; Ar is a divalent arylene; E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; $(R^{15}-O)n$ is a polyoxyalkylene moiety, which can be a homopolymer, a random copolymer, or a block copolymer of $C_2$-$C_4$ oxyalkylene units, $R^{15}$ is a divalent

alkylene moiety selected from $C_2H_4$, $C_3H_6$, or $C_4H_8$, and combinations thereof; and n is an integer in the range of 2 to 150 in one aspect, from 10 to 120 in another aspect, and from 15 to 60 in a further aspect; Y is $-R^{15}O-$, $-R^{15}NH-$, -C(O)-, -C(O)NH-, $-R^{15}NHC(O)NH-$, or -C(O)NHC(O)-; $R^{16}$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an araalkyl substituted phenyl, and an aryl-substituted $C_2$-$C_{30}$ alkyl; wherein the $R^{16}$ alkyl group, aryl group, phenyl group optionally comprises one or more substituents selected from the group consisting of a hydroxyl group, an alkoxyl group, benzyl group, styryl group, and a halogen group; and said semi-hydrophobic monomer is selected from at least one monomer represented by formulas VIII and IX:

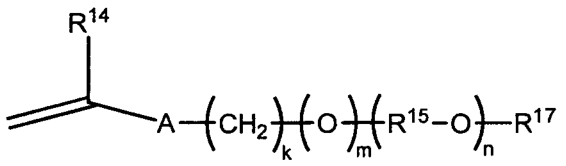

VIII

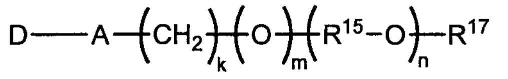

IX

wherein $R^{14}$ is hydrogen or methyl; A is $-CH_2C(O)O-$, -C(O)O-, -O-, $-CH_2O-$,-NHC(O)NH-, -C(O)NH-, $-Ar-(CE_2)_z-NHC(O)O-$, $-Ar-(CE_2)_z-NHC(O)NH-$, or $-CH_2CH_2NHC(O)-$; Ar is a divalent arylene; E is H or methyl; z is 0 or 1; k is an integer ranging from 0 to 30, and m is 0 or 1, with the proviso that when k is 0, m is 0, and when k is in the range of 1 to 30, m is 1; $(R^{15}-O)_n$ is a polyoxyalkylene moiety, which can be a homopolymer, a random copolymer, or a block copolymer of $C_2$-$C_4$ oxyalkylene units, $R^{15}$ is a divalent alkylene moiety selected from $C_2H_4$, $C_3H_6$, or $C_4H_8$, and combinations thereof; and n is an integer in the range of 2 to 150 in one aspect, from 5 to 120 in another aspect, and from 10 to 60 in a further aspect; $R^{17}$ is selected from hydrogen and a linear or branched $C_1$-$C_4$ alkyl group; and D represents a vinyl or an allyl moiety.

**3.** The polymer for the use according to claim 1 wherein said $C_1$-$C_4$ hydroxyalkyl (meth)acrylate is hydroxyethyl methacrylate, said $C_1$-$C_{12}$ alkyl acrylate is selected from methyl methacrylate, ethyl acrylate, butyl acrylate, or mixtures thereof, said vinyl ester of a $C_1$-$C_{10}$ carboxylic acid is selected from vinyl formate, vinyl acetate, vinyl propionate, vinyl butyrate, vinyl isobutyrate, vinyl valerate, vinyl hexanoate, vinyl 2-methylhexanoate, vinyl 2-ethylhexanoate, vinyl iso-octanoate, vinyl nonanoate, vinyl neodecanoate, vinyl decanoate, vinyl versatate, vinyl laurate, vinyl palmitate, vinyl stearate, or mixtures thereof; or mixtures thereof.

**4.** The polymer for the use according to claim 1 wherein said emulsion polymer is polymerized from a monomer mixture comprising hydroxyethyl methacrylate, and a monomer selected from methyl methacrylate, ethyl acrylate, butyl acrylate, vinyl acetate, vinyl neodecanoate, vinyl decanoate, or mixtures thereof, or
wherein said emulsion polymer is polymerized from a monomer mixture comprising hydroxyethyl methacrylate, ethyl acrylate, butyl acrylate and vinyl acetate, or
wherein said emulsion polymer is polymerized from a monomer mixture comprising hydroxyethyl methacrylate, ethyl acrylate, and butyl acrylate and a monomer selected from an associative and/or semi-hydrophobic monomer.

**5.** The polymer for the use according to claim 2 wherein said associative monomer is represented by formula VIIB:

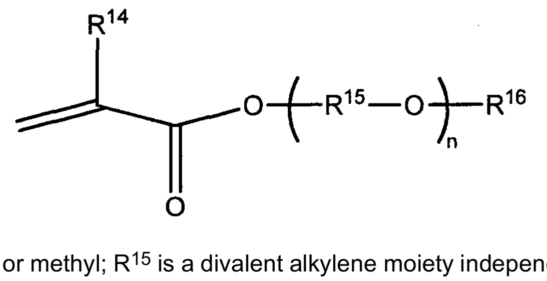

VIIB

wherein $R^{14}$ is hydrogen or methyl; $R^{15}$ is a divalent alkylene moiety independently selected from $C_2H_4$, $C_3H_6$, and $C_4H_8$, and n represents an integer ranging from 10 to 60, $(R^{15}-O)$ can be arranged in a random or a block configuration; $R^{16}$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an araalkyl substituted phenyl, and an aryl-substituted $C_2$-$C_{30}$

alkyl, wherein the $R^{16}$ alkyl group, aryl group, phenyl group optionally comprises one or more substituents selected from the group consisting of a hydroxyl group, an alkoxyl group, benzyl group styryl group, and a halogen group.

**6.** The polymer for the use according to claim 2 wherein said semi-hydrophobic monomer comprises (i) an ethylenically unsaturated end group portion; (ii) a polyoxyalkylene mid-section portion, and (iii) an end group portion selected from hydrogen or a alkyl group containing 1 to 4 carbon atoms.

**7.** The polymer for the use according to any of claims 1 to 4 wherein said crosslinker is selected from a monomer having an average of 3 crosslinkable unsaturated functional groups.

**8.** The polymer for the use according to any of claims 2, 4 to 6 wherein said emulsion polymer is polymerized from a monomer mixture comprising from 40 to 45 wt.% of hydroxyethyl acrylate, 30 to 50 wt. % of ethyl acrylate, 10 to 20 wt.% of butyl acrylate and from 1 to 5 wt.% of at least one associative and/or semi-hydrophobic monomer (based on the weight of the total monomers), and at least one crosslinker.

**9.** A crosslinked nonionic amphiphilic emulsion polymer prepared from a free radically polymerizable monomer mixture comprising:

i) 40 to 50 wt.% of at least one hydroxy($C_1$-$C_5$)alkyl (meth)acrylate monomer (based on the total monomer wt.);
ii) 15 to 70 wt.% of at least two different monomers selected from a ($C_1$-$C_5$)alkyl (meth)acrylate monomer (based on the total monomer wt.);
iii) 0.5 to 5 wt.% of an associative and/or a semi-hydrophobic monomer; and
iv) 0.01 to 0.3 wt.% of at least one crosslinker (based on the dry weight of the polymer) for use in mitigating ocular and/or skin irritation of a detersive composition comprising

a) water;
b) 1 to 5 wt.% of said crosslinked nonionic amphiphilic emulsion polymer; and
c) 6 to 20 wt.% of a surfactant mixture containing an anionic surfactant and an amphoteric surfactant.

**10.** The polymer for the use according to claim 9 wherein said monomer i) is hydroxyethyl methacrylate.

**11.** The polymer for the use according to any of claims 9 or 10 wherein said monomers ii) are ethyl acrylate and n-butyl acrylate.

**12.** The polymer for the use according to any of claims 9 to 11 wherein said associative monomer is selected from behenyl polyethoxylated methacrylate.

**13.** The polymer for the use according to any of claims 9 to 12 wherein said semi hydrophobic monomer is selected from methoxy polyethyleneglycol methacrylate.

**14.** The polymer for the use according to any of claims 9 to 13 wherein said anionic surfactant is selected from the sodium or ammonium salts of dodecyl sulfate, lauryl sulfate, laureth sulfate, or mixtures thereof.

**15.** The polymer for the use according to any of claims 9 to 14 wherein said amphoteric surfactant is cocamidopropyl betaine.

**Patentansprüche**

**1.** Vernetztes nichtionisches amphiphiles Emulsionspolymer, das aus einer radikalisch polymerisierbaren Monomerzusammensetzung hergestellt ist, die wenigstens 30 Gew.-% wenigstens eines $C_1$-$C_4$-Hydroxyalkyl(meth)-acrylats, 15 bis 70 Gew.-% wenigstens eines $C_1$-$C_{12}$-Alkyl(meth)acrylats, 5 bis 40 Gew.-% wenigstens eines Vinylesters einer aliphatischen $C_1$-$C_{10}$-Carbonsäure (bezogen auf das Gewicht der Gesamtmonomere) und 0,01 bis 0,3 Gew.-% wenigstens eines Vernetzers (bezogen auf das Trockengewicht des Polymers) umfasst, zur Verwendung bei der Linderung von Augen- und/oder Hautreizungen durch waschaktive Zusammensetzungen, die wenigstens ein anionisches Tensid umfassen.

**2.** Vernetztes nichtionisches amphiphiles Emulsionspolymer, das aus einer radikalisch polymerisierbaren Monomerzusammensetzung hergestellt ist, die wenigstens 30 Gew.-% wenigstens eines $C_1$-$C_4$-Hydroxyalkyl(meth)-acrylats, 15 bis 70 Gew.-% wenigstens eines $C_1$-$C_{12}$-Alkyl(meth)acrylats, 1 bis 10 Gew.-% wenigstens eines Monomers, das aus einem assoziativen Monomer, einem halbhydrophoben Monomer oder Gemischen davon ausgewählt ist (bezogen auf das Gewicht der Gesamtmonomere), und 0,01 bis 0,3 Gew.-% wenigstens eines Vernetzers (bezogen auf das Trockengewicht des Polymers) umfasst, zur Verwendung bei der Linderung von Augen- und/oder Hautreizungen durch waschaktive Zusammensetzungen, die wenigstens ein anionisches Tensid umfassen, wobei das assoziative Monomer durch die Formeln VII und/oder VIIA dargestellt wird:

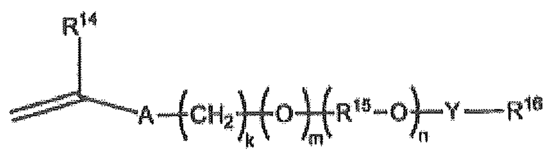

VII

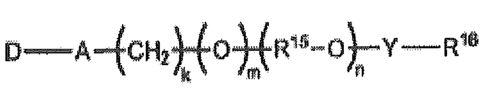

VIIA

wobei $R^{14}$ Wasserstoff oder Methyl ist, A = $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist, Ar ein zweiwertiges Arylen ist, E = H oder Methyl ist, z = 0 oder 1 ist, k eine ganze Zahl im Bereich von 0 bis 30 ist und m = 0 oder 1 ist, mit der Maßgabe, dass m = 0 ist, wenn k = 0 ist, und dass m = 1 ist, wenn k im Bereich von 1 bis 30 liegt; D für eine Vinyl- oder Allyl-Struktureinheit steht; $(R^{15}-O)_n$ eine Polyoxyalkylen-Struktureinheit ist, die ein Homopolymer, ein statistisches Copolymer oder ein Blockcopolymer aus $C_2$-$C_4$-Oxyalkyleneinheiten sein kann, $R^{15}$ eine zweiwertige Alkylen-Struktureinheit ist, die aus $C_2H_4$, $C_3H_6$ oder $C_4H_8$ und Kombinationen davon ausgewählt ist, und n eine ganze Zahl im Bereich von in einem Aspekt 2 bis 150, in einem anderen Aspekt 10 bis 120 und in einem weiteren Aspekt 15 bis 60 ist; Y = $-R^{15}O-$, $-R^{15}NH-$, $-C(O)-$, $-C(O)NH-$, $-R^{15}NHC(O)NH-$ oder $-C(O)NHC(O)-$ ist; $R^{16}$ ein substituiertes oder unsubstituiertes Alkyl ist, das aus einem linearen $C_8$-$C_{30}$-Alkyl, einem verzweigten $C_8$-$C_{30}$-Alkyl, einem carbocyclischen $C_8$-$C_{30}$-Alkyl, einem $C_2$-$C_{30}$-Alkyl-substituierten Phenyl, einem Aralkyl-substituierten Phenyl und einem arylsubstituierten $C_2$-$C_{30}$-Alkyl ausgewählt ist, wobei die $R^{16}$-Alkylgruppe, die Arylgruppe oder die Phenylgruppe gegebenenfalls einen oder mehrere Substituenten umfasst, die aus der Gruppe ausgewählt sind, die aus einer Hydroxygruppe, einer Alkoxygruppe, Benzylgruppe, Styrylgruppe und einer Halogengruppe besteht; und das halbhydrophobe Monomer (i) aus wenigstens einem Monomer ausgewählt ist, das durch die Formeln VIII und IX repräsentiert wird:

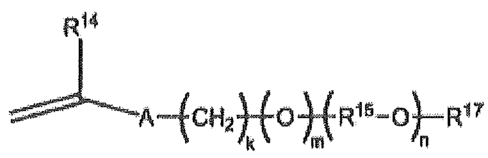

VIII

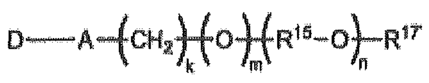

IX

wobei $R^{14}$ Wasserstoff oder Methyl ist, A = $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist, Ar ein zweiwertiges Arylen ist, E = H oder Methyl ist, z = 0 oder 1 ist, k eine ganze Zahl im Bereich von 0 bis 30 ist und m = 0 oder 1 ist, mit der Maßgabe, dass m = 0 ist, wenn k = 0 ist, und dass m = 1 ist, wenn k im Bereich von 1 bis 30 liegt; $(R^{15}-O)_n$ eine Polyoxyalkylen-Struktureinheit ist, die ein Homopolymer, ein statistisches Copolymer oder ein Blockcopolymer aus $C_2$-$C_4$-Oxyalkyleneinheiten sein kann, $R^{15}$ eine zweiwertige Alkylen-Struktureinheit ist, die aus $C_2H_4$, $C_3H_6$ oder $C_4H_8$ und Kombinationen davon ausgewählt ist, und n eine ganze Zahl im Bereich von 2 bis 150 in einem Aspekt, 5 bis 120 in einem anderen Aspekt und 10 bis 60 in einem weiteren Aspekt ist; $R^{17}$ aus Wasserstoff und einer linearen oder verzweigten $C_1$-$C_4$-Alkylgruppe ausgewählt ist und D für eine Vinyl- oder Allyl-Struktureinheit steht.

**3.** Polymer zur Verwendung gemäß Anspruch 1, wobei es sich bei dem $C_1$-$C_4$-Hydroxyalkyl(meth)acrylat um Hydroxyethylmethacrylat handelt, das $C_1$-$C_{12}$-Alkylacrylat aus Methylmethacrylat, Ethylacrylat, Butylacrylat oder Gemischen davon ausgewählt ist, der Vinylester einer $C_1$-$C_{10}$-Carbonsäure aus Vinylformiat, Vinylacetat, Vinylpropionat,

Vinylbutyrat, Vinylisobutyrat, Vinylvalerat, Vinylhexanoat, Vinyl-2-methylhexanoat, Vinyl-2-ethyl-hexanoat, Vinylisooctanoat, Vinylnonanoat, Vinylneodecanoat, Vinyldecanoat, Vinylversatat, Vinyllaurat, Vinylpalmitat, Vinylstearat oder Gemischen davon ausgewählt ist; oder Gemische davon.

**4.** Polymer zur Verwendung gemäß Anspruch 1, wobei das Emulsionspolymer aus einem Monomergemisch polymerisiert ist, das Hydroxyethylmethacrylat und ein Monomer, das aus Methylmethacrylat, Ethylacrylat, Butylacrylat, Vinylacetat, Vinylneodecanoat, Vinyldecanoat oder Gemischen davon ausgewählt ist, umfasst, oder
wobei das Emulsionspolymer aus einem Monomergemisch polymerisiert ist, das Hydroxyethylmethacrylat, Ethylacrylat, Butylacrylat und Vinylacetat umfasst, oder
wobei das Emulsionspolymer aus einem Monomergemisch polymerisiert ist, das Hydroxyethylmethacrylat, Ethylacrylat und Butylacrylat sowie ein Monomer, das aus einem assoziativen Monomer und/oder einem halbhydrophoben Monomer ausgewählt ist, umfasst.

**5.** Polymer zur Verwendung gemäß Anspruch 2, wobei das assoziative Monomer durch die Formel VIIB dargestellt wird:

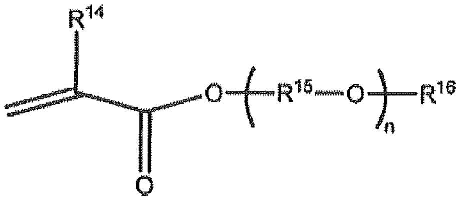
VIIB

wobei $R^{14}$ Wasserstoff oder Methyl ist, $R^{15}$ eine zweiwertige Alkylen-Struktureinheit ist, die unabhängig aus $C_2H_4$, $C_3H_6$ und $C_4H_8$ ausgewählt ist, und n für eine ganze Zahl im Bereich von 10 bis 60 steht, ($R^{15}$-O) in einer statistischen oder Blockkonfiguration angeordnet sein kann, $R^{16}$ ein substituiertes oder unsubstituiertes Alkyl ist, das aus einem linearen $C_8$-$C_{30}$-Alkyl, einem verzweigten $C_8$-$C_{30}$-Alkyl, einem carbocyclischen $C_8$-$C_{30}$-Alkyl, einem $C_2$-$C_{30}$-Alkyl-substituierten Phenyl, einem Aralkyl-substituierten Phenyl und einem arylsubstituierten $C_2$-$C_{30}$-Alkyl ausgewählt ist, wobei die $R^{16}$-Alkylgruppe, die Arylgruppe oder die Phenylgruppe gegebenenfalls einen oder mehrere Substituenten umfasst, die aus der Gruppe ausgewählt sind, die aus einer Hydroxygruppe, einer Alkoxygruppe, Benzylgruppe, Styrylgruppe und einer Halogengruppe besteht.

**6.** Polymer zur Verwendung gemäß Anspruch 2, wobei das halbhydrophobe Monomer (i) einen ethylenisch ungesättigten Endgruppenteil, (ii) einen Polyoxyalkylen-Mittelabschnittsteil und (iii) einen Endgruppenteil, der aus Wasserstoff oder einer Alkylgruppe, die 1 bis 4 Kohlenstoffatome enthält, ausgewählt ist, umfasst.

**7.** Polymer zur Verwendung gemäß einem der Ansprüche 1 bis 4, wobei der Vernetzer aus einem Monomer ausgewählt ist, das im Durchschnitt 3 vernetzbare ungesättigte funktionelle Gruppen aufweist.

**8.** Polymer zur Verwendung gemäß einem der Ansprüche 2, 4 bis 6, wobei das Emulsionspolymer aus einem Monomergemisch polymerisiert ist, das 40 bis 45 Gew.-% Hydroxyethylacrylat, 30 bis 50 Gew.-% Ethylacrylat, 10 bis 20 Gew.-% Butylacrylat und 1 bis 5 Gew.-% wenigstens eines assoziativen und/oder halbhydrophoben Monomers (bezogen auf das Gewicht der Gesamtmonomere) und wenigstens einen Vernetzer umfasst.

**9.** Vernetztes nichtionisches amphiphiles Emulsionspolymer, das aus einem radikalisch polymerisierbaren Monomergemisch hergestellt ist, umfassend:

i) 40 bis 50 Gew.-% wenigstens eines Hydroxy($C_1$-$C_5$)alkyl(meth)-acrylat-Monomers (bezogen auf das Gesamtmonomergewicht);
ii) 15 bis 70 Gew.-% von wenigstens zwei verschiedenen Monomeren, die aus einem ($C_1$-$C_5$)Alkyl(meth)acrylat-Monomer ausgewählt sind (bezogen auf das Gesamtmonomergewicht);
iii) 0,5 bis 5 Gew.-% eines assoziativen und/oder halbhydrophoben Monomers; und
iv) 0,01 bis 0,3 Gew.-% wenigstens eines Vernetzers (bezogen auf das Trockengewicht des Polymers)

zur Verwendung bei der Linderung von Augen- und/oder Hautreizungen durch eine waschaktive Zusammensetzung, umfassend:

a) Wasser;

b) 1 bis 5 Gew.-% des vernetzten nichtionischen amphiphilen Emulsionspolymers; und

c) 6 bis 20 Gew.-% eines Tensidgemischs, das ein anionisches Tensid und ein amphoteres Tensid enthält.

**10.** Polymer zur Verwendung gemäß Anspruch 9, wobei es sich bei dem Monomer i) um Hydroxyethylmethacrylat handelt.

**11.** Polymer zur Verwendung gemäß einem der Ansprüche 9 oder 10, wobei es sich bei den Monomeren ii) um Ethylacrylat und n-Butylacrylat handelt.

**12.** Polymer zur Verwendung gemäß einem der Ansprüche 9 bis 11, wobei das assoziative Monomer aus Behenylpolyethoxyliertem-methacrylat ausgewählt ist.

**13.** Polymer zur Verwendung gemäß einem der Ansprüche 9 bis 12, wobei das halbhydrophobe Monomer aus Methoxypolyethylenglycolmethacrylat ausgewählt ist.

**14.** Polymer zur Verwendung gemäß einem der Ansprüche 9 bis 13, wobei das anionische Tensid aus den Natrium- oder Ammoniumsalzen von Dodecylsulfat, Laurylsulfat, Laurethsulfat oder Gemischen davon ausgewählt ist.

**15.** Polymer zur Verwendung gemäß einem der Ansprüche 9 bis 14, wobei es sich bei dem amphoteren Tensid um Cocamidopropylbetain handelt.

**Revendications**

**1.** Polymère en émulsion amphiphile non ionique réticulé préparé à partir d'une composition de monomères polymérisables par voie radicalaire comprenant au moins 30 % en poids d'au moins un (méth)acrylate d'hydroxyalkyle en $C_1$-$C_4$, de 15 à 70 % en poids d'au moins un (méth)acrylate d'alkyle en $C_1$-$C_{12}$, de 5 à 40 % en poids d'au moins un ester de vinyle d'un acide carboxylique aliphatique en $C_1$-$C_{10}$ (sur la base du poids des monomères totaux), et de 0,01 à 0,3 % en poids d'au moins un agent de réticulation (sur la base du poids sec du polymère) pour son utilisation dans le soulagement d'une irritation oculaire et/ou cutanée induite par des compositions détergentes comprenant au moins un tensio-actif anionique.

**2.** Polymère en émulsion amphiphile non ionique réticulé préparé à partir d'une composition de monomères polymérisables par voie radicalaire comprenant au moins 30 % en poids d'au moins un (méth)acrylate d'hydroxyalkyle en $C_1$-$C_4$, de 15 à 70 % en poids d'au moins un (méth)acrylate d'alkyle en $C_1$-$C_{12}$, de 1 à 10 % en poids d'au moins un monomère sélectionné parmi un monomère associatif, un monomère semi-hydrophobe, ou des mélanges de ceux-ci (sur la base du poids des monomères totaux), et de 0,01 à 0,3 % en poids d'au moins un agent de réticulation (sur la base du poids sec du polymère) pour son utilisation dans le soulagement d'une irritation oculaire et/ou cutanée induite par des compositions détergentes comprenant au moins un tensio-actif anionique, dans lequel ledit monomère associatif est représenté par les formules VII et/ou VIIA :

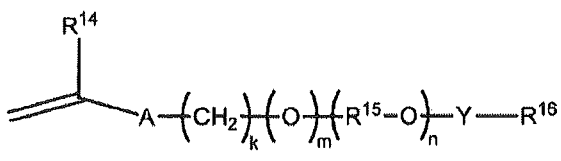

VII

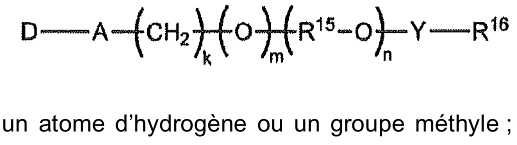

VIIA

dans lequel $R^{14}$ est un atome d'hydrogène ou un groupe méthyle ; A est $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, ou $-CH_2CH_2NHC(O)-$; Ar est un groupe arylène bivalent ; E est H ou un groupe méthyle ; z est 0 ou 1 ; k est un nombre entier dans la plage de 0 à 30, et m est 0 ou 1, à condition que lorsque k est 0, m soit 0, et lorsque k est dans la plage de 1 à 30, m soit 1 ; D représente une fraction vinyle ou allyle ; $(R^{15}-O)n$ est une fraction polyoxyalkylène, qui peut être un homopolymère, un copolymère statistique, ou un copolymère séquencé d'unités oxyalkylène en $C_2$-$C_4$, $R^{15}$ est une fraction alkylène bivalente sélectionnée parmi $C_2H_4$, $C_3H_6$, ou $C_4H_8$, et leurs combinaisons ; et n est un nombre entier dans la plage de 2 à

150 dans un aspect, de 10 à 120 dans un autre aspect, et de 15 à 60 dans encore un aspect ; Y est $-R^{15}-O-$, $-R^{15}NH-$, -C(O)-, -C(O)NH-, $-R^{15}NHC(O)NH-$, ou -C(O)NHC(O)- ; $R^{16}$ est un groupe alkyle substitué ou non substitué sélectionné parmi un groupe alkyle linéaire en $C_8-C_{30}$, un groupe alkyle ramifié en $C_8-C_{30}$, un groupe alkyle carbocyclique en $C_8-C_{30}$, un groupe phényle substitué par un groupe alkyle en $C_2-C_{30}$, un groupe phényle substitué par un groupe araalkyle, et un groupe alkyle en $C_2-C_{30}$ substitué par un groupe aryle ; dans lequel le groupe alkyle, groupe aryle, groupe phényle $R^{16}$ comprend facultativement un ou plusieurs substituants sélectionnés dans le groupe constitué d'un groupe hydroxyle, d'un groupe alcoxyle, d'un groupe benzyle, d'un groupe styryle, et d'un groupe halogène ; et ledit monomère semi-hydrophobe est sélectionné parmi au moins un monomère représenté par les formules VIII et IX :

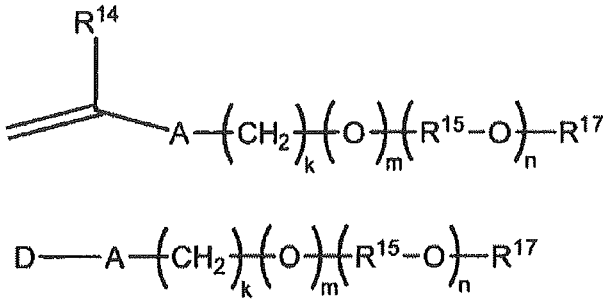

VIII

IX

dans lequel $R^{14}$ est un atome d'hydrogène ou un groupe méthyle ; A est $-CH_2C(O)O-$, -C(O)O-, -O-, $-CH_2O-$ ,-NHC(O)NH-, -C(O)NH-, $-Ar-(CE_2)_z-NHC(O)O-$, $-Ar-(CE_2)_z-NHC(O)NH-$, ou $-CH_2CH_2NHC(O)-$ ; Ar est un groupe arylène bivalent ; E est H ou un groupe méthyle ; z est 0 ou 1 ; k est un nombre entier dans la plage de 0 à 30, et m est 0 ou 1, à condition que lorsque k est 0, m soit 0, et lorsque k est dans la plage de 1 à 30, m soit 1 ; $(R^{15}-O)_n$ est une fraction polyoxyalkylène, qui peut être un homopolymère, un copolymère statistique, ou un copolymère séquencé d'unités oxyalkylène en $C_2-C_4$, $R^{15}$ est une fraction alkylène bivalente sélectionnée parmi $C_2H_4$, $C_3H_6$, ou $C_4H_8$, et leurs combinaisons ; et n est un nombre entier dans la plage de 2 à 150 dans un aspect, de 5 à 120 dans un autre aspect, et de 10 à 60 dans encore un aspect ; $R^{17}$ est sélectionné parmi un atome d'hydrogène et un groupe alkyle en $C_1-C_4$ linéaire ou ramifié ; et D représente une fraction vinyle ou allyle.

**3.** Polymère pour son utilisation selon la revendication 1, dans lequel ledit (méth)acrylate d'hydroxyalkyle en $C_1-C_4$ est le méthacrylate d'hydroxyéthyle, ledit acrylate d'alkyle en $C_1-C_{12}$ est sélectionné parmi le méthacrylate de méthyle, l'acrylate d'éthyle, l'acrylate de butyle, ou les mélanges de ceux-ci, ledit ester de vinyle d'un acide carboxylique en $C_1-C_{10}$ est sélectionné parmi le formiate de vinyle, l'acétate de vinyle, le propionate de vinyle, le butyrate de vinyle, l'isobutyrate de vinyle, le valérate de vinyle, l'hexanoate de vinyle, le 2-méthylhexanoate de vinyle, le 2-éthylhexanoate de vinyle, l'iso-octanoate de vinyle, le nonanoate de vinyle, le néodécanoate de vinyle, le décanoate de vinyle, le versatate de vinyle, le laurate de vinyle, le palmitate de vinyle, le stéarate de vinyle, ou les mélanges de ceux-ci ; ou les mélanges de ceux-ci.

**4.** Polymère pour son utilisation selon la revendication 1, dans lequel ledit polymère en émulsion est polymérisé à partir d'un mélange de monomères comprenant le méthacrylate d'hydroxyéthyle, et d'un monomère sélectionné parmi le méthacrylate de méthyle, l'acrylate d'éthyle, l'acrylate de butyle, l'acétate de vinyle, le néodécanoate de vinyle, le décanoate de vinyle, ou les mélanges de ceux-ci, ou
dans lequel ledit polymère en émulsion est polymérisé à partir d'un mélange de monomères comprenant le méthacrylate d'hydroxyéthyle, l'acrylate d'éthyle, l'acrylate de butyle et l'acétate de vinyle, ou
dans lequel ledit polymère en émulsion est polymérisé à partir d'un mélange de monomères comprenant le méthacrylate d'hydroxyéthyle, l'acrylate d'éthyle, et l'acrylate de butyle et d'un monomère sélectionné parmi un monomère associatif et/ou semi-hydrophobe.

**5.** Polymère pour son utilisation selon la revendication 2, dans lequel ledit monomère associatif est représenté par la formule VIIB :

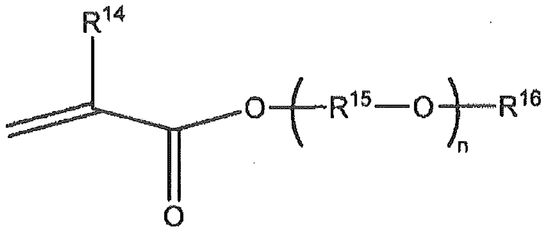

VIIB

dans lequel $R^{14}$ est un atome d'hydrogène ou un groupe méthyle ; $R^{15}$ est une fraction alkylène bivalente indépendamment sélectionnée parmi $C_2H_4$, $C_3H_6$, et $C_4H_8$, et n représente un nombre entier dans la plage de 10 à 60, ($R^{15}$-O) peut être agencé dans une configuration statistique ou séquencée ; $R^{16}$ est un groupe alkyle substitué ou non substitué sélectionné parmi un groupe alkyle linéaire en $C_8$-$C_{30}$, un groupe alkyle ramifié en $C_8$-$C_{30}$, un groupe alkyle carbocyclique en $C_8$-$C_{30}$, un groupe phényle substitué par un groupe alkyle en $C_2$-$C_{30}$, un groupe phényle substitué par un groupe araalkyle, et un groupe alkyle en $C_2$-$C_{30}$ substitué par un groupe aryle, dans lequel le groupe alkyle, groupe aryle, groupe phényle $R^{16}$ comprend facultativement un ou plusieurs substituants sélectionnés dans le groupe constitué d'un groupe hydroxyle, d'un groupe alcoxyle, d'un groupe benzyle, d'un groupe styryle, et d'un groupe halogène.

**6.** Polymère pour son utilisation selon la revendication 2, dans lequel ledit monomère semi-hydrophobe comprend

(i) une partie groupe terminal éthyléniquement insaturé ;
(ii) une partie section intermédiaire polyoxyalkylène, et (iii) une partie groupe terminal sélectionnée parmi un atome d'hydrogène ou un groupe alkyle contenant de 1 à 4 atomes de carbone.

**7.** Polymère pour son utilisation selon l'une quelconque des revendications 1 à 4, dans lequel ledit agent de réticulation est sélectionné parmi un monomère ayant une moyenne de 3 groupes fonctionnels insaturés réticulables.

**8.** Polymère pour son utilisation selon l'une quelconque des revendications 2, 4 à 6, dans lequel ledit polymère en émulsion est polymérisé à partir d'un mélange de monomères comprenant de 40 à 45 % en poids d'acrylate d'hydroxyéthyle, de 30 à 50 % en poids d'acrylate d'éthyle, de 10 à 20 % en poids d'acrylate de butyle et de 1 à 5 % en poids d'au moins un monomère associatif et/ou semi-hydrophobe (sur la base du poids des monomères totaux), et au moins un agent de réticulation.

**9.** Polymère en émulsion amphiphile non ionique réticulé préparé à partir d'un mélange de monomères polymérisables par voie radicalaire comprenant :

i) de 40 à 50 % en poids d'au moins un monomère de (méth)acrylate d'hydroxy(alkyle en $C_1$-$C_5$) (sur la base du poids total des monomères) ;
ii) de 15 à 70 % en poids d'au moins deux monomères différents sélectionnés parmi un monomère de (méth)acrylate d'alkyle en $C_1$-$C_5$ (sur la base du poids total des monomères) ;
iii) de 0,5 à 5 % en poids d'un monomère associatif et/ou semi-hydrophobe ; et
iv) de 0,01 à 0,3 % en poids d'au moins un agent de réticulation (sur la base du poids sec du polymère) pour son utilisation dans le soulagement d'une irritation oculaire et/ou cutanée induite par une composition détergente comprenant

a) de l'eau ;
b) de 1 à 5 % en poids dudit polymère en émulsion amphiphile non ionique réticulé ; et
c) de 6 à 20 % en poids d'un mélange de tensio-actifs contenant un tensio-actif anionique et un tensio-actif amphotère.

**10.** Polymère pour son utilisation selon la revendication 9, dans lequel ledit monomère i) est le méthacrylate d'hydroxyéthyle.

**11.** Polymère pour son utilisation selon l'une quelconque des revendications 9 ou 10, dans lequel lesdits monomères ii) sont l'acrylate d'éthyle et l'acrylate de n-butyle.

**12.** Polymère pour son utilisation selon l'une quelconque des revendications 9 à 11, dans lequel ledit monomère associatif

est sélectionné parmi le méthacrylate polyéthoxylé de béhényle.

13. Polymère pour son utilisation selon l'une quelconque des revendications 9 à 12, dans lequel ledit monomère semi-hydrophobe est sélectionné parmi le méthacrylate de méthoxy polyéthylèneglycol.

14. Polymère pour son utilisation selon l'une quelconque des revendications 9 à 13, dans lequel ledit tensio-actif anionique est sélectionné parmi les sels de sodium ou d'ammonium de sulfate de docécyle, de sulfate de lauryle, de sulfate de laureth, ou les mélanges de ceux-ci.

15. Polymère pour son utilisation selon l'une quelconque des revendications 9 à 14, dans lequel ledit tensio-actif amphotère est la cocamidopropyl bétaïne.

Fig. 1

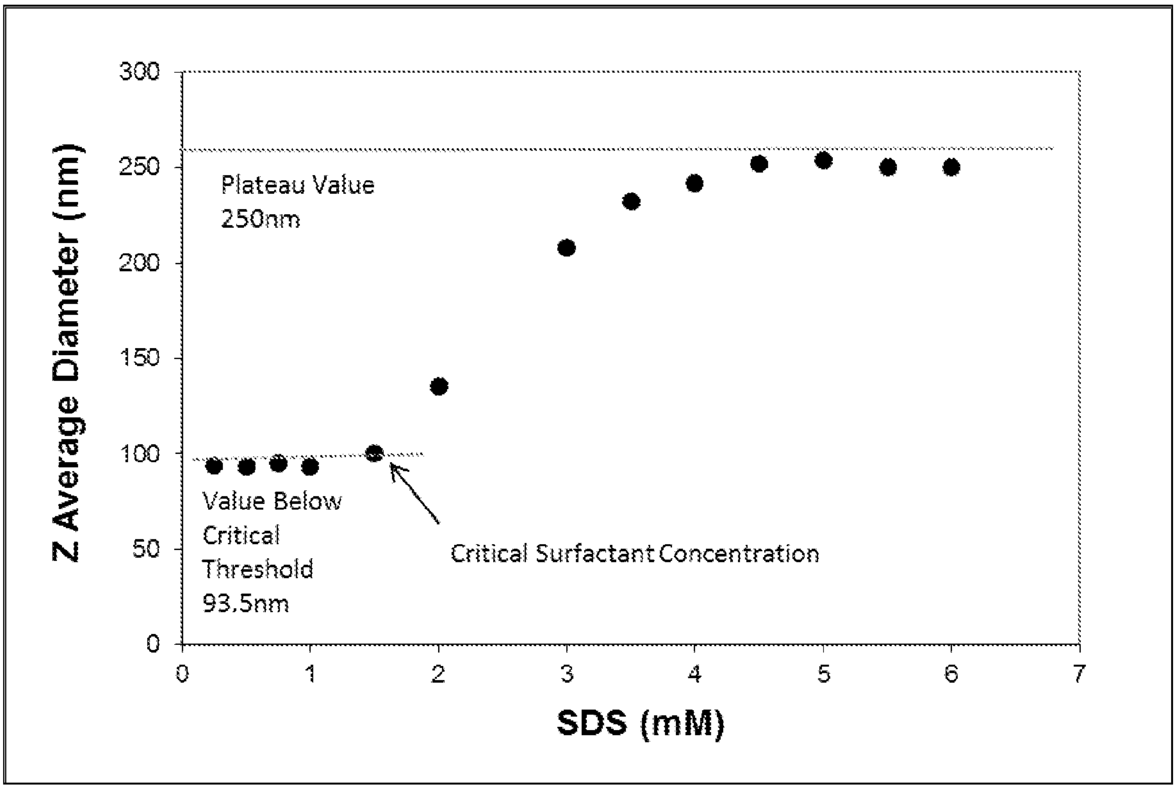
300
250
200
150
100
50
0
Z Average Diameter (nm)
0
1
2
3
4
5
6
7
SDS (mM)
Plateau Value
250nm
Value Below
Critical
Threshold
93.5nm
Critical Surfactant Concentration

Fig. 2

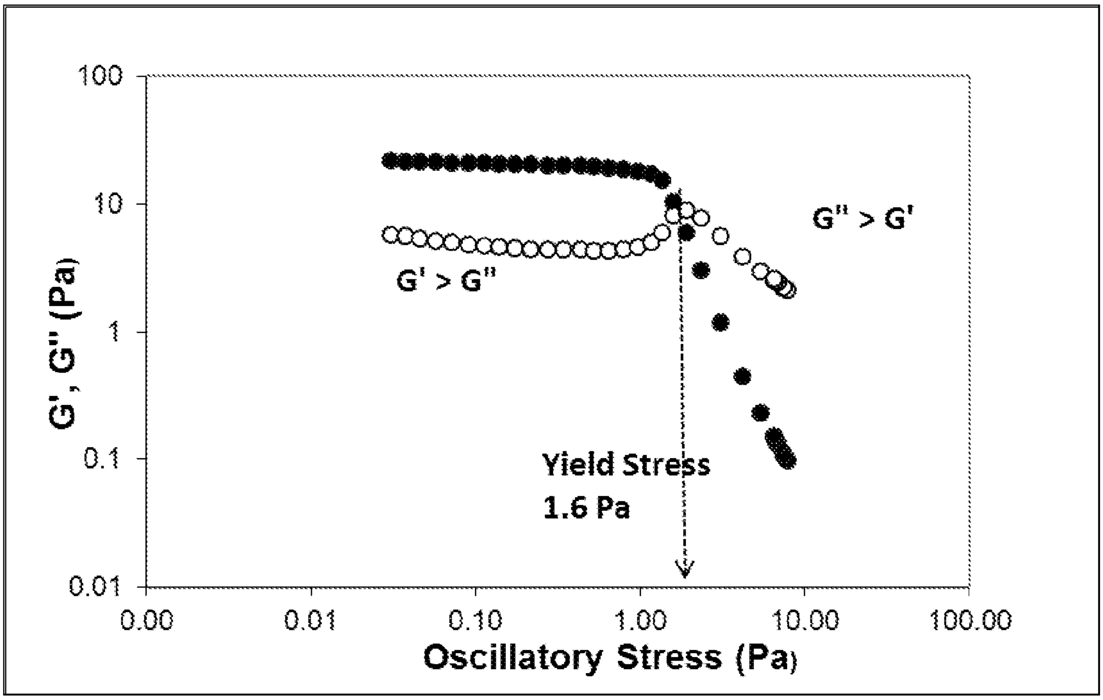
100
10
1
0.1
0.01
G', G'' (Pa)
0.00
0.01
0.10
1.00
10.00
100.00
Oscillatory Stress (Pa)
G' > G''
G'' > G'
Yield Stress
1.6 Pa

Fig. 3

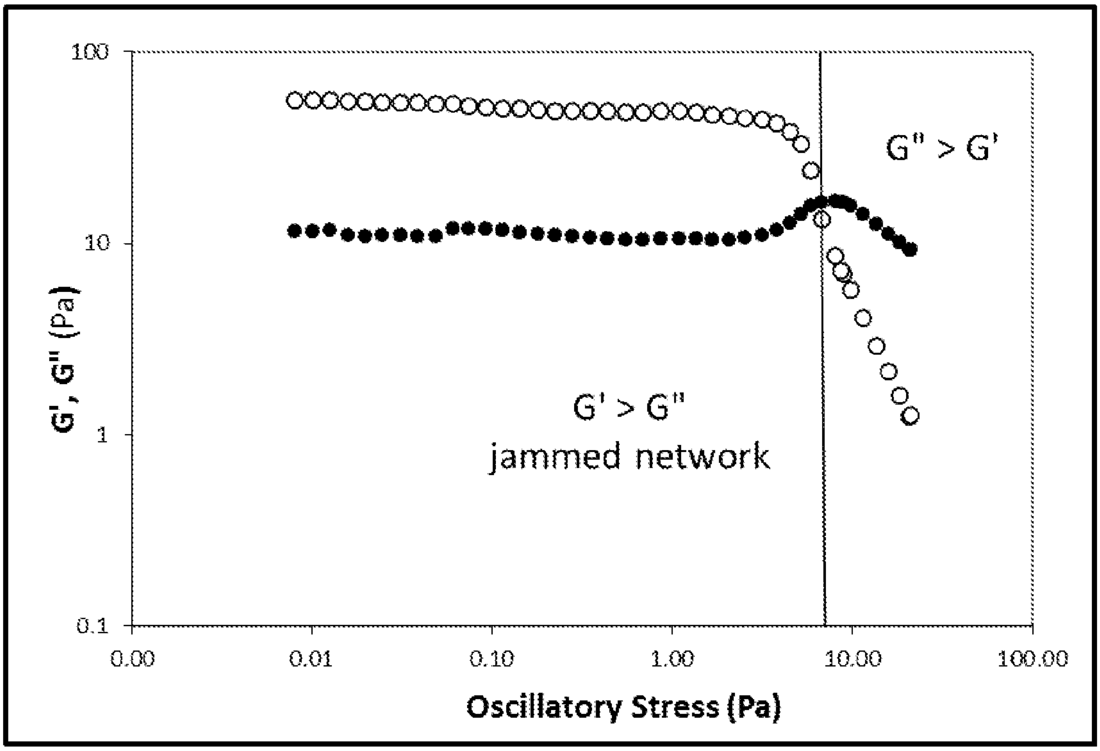
100
10
1
0.1
G', G" (Pa)
0.00
0.01
0.10
1.00
10.00
100.00
Oscillatory Stress (Pa)
G" > G'
G' > G"
jammed network

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4726915 A
- WO 2005023970 A
- US 6433061 B
- US 8293845 B
- WO 2011042379 A
- WO 2008060997 A
- US 20040096474 A
- US 33156 A
- US 5294692 A
- US 5011978 A
- US 4421902 A, Chang
- US 4384096 A, Sonnabend
- US 4514552 A, Shay
- US 4600761 A, Ruffner
- US 4616074 A, Ruffner
- US 5292843 A, Jenkins
- US 5770760 A, Robinson
- US 5412142 A, Wilkerson, III
- US 4285841 A, Barrat
- US 4284532 A, Leikhim
- US 5527892 A
- US 5770543 A
- US 6573375 B
- US 6727357 B
- US 7786027 B


### Non-patent literature cited in the description


- **SCHOENBERG.** Baby Shampoo. *Household & Personal Products Industry,* September 1979, vol. 60
- TDS-222. Lubrizol Advanced Materials, Inc, 15 October 2007
- McCutcheon's Detergents and Emulsifiers. Allured Publishing Corporation, 1998
- **MCCUTCHEON'S.** Functional Materials,. 1992
- McCutcheon's Detergents and Emulsifiers
- McCutcheon's, Functional Materials
- McCutcheon's Detergents and Emulsifiers. 1998
- McCutcheon's, Functional Materials,